# EUROPEAN PATENT SPECIFICATION

(11) **EP 4 346 928 B1**
(45) Date of publication and mention of the grant of the patent: **14.01.2026**
(21) Application number: 22732802.8
(22) Date of filing: 26.05.2022
(51) Int. Cl.: A61L 27/18, A61L 27/56, A61L 27/48, A61L 27/38

(54) **BIODEGRADABLE TISSUE SCAFFOLD WITH SECONDARY MATRIX TO HOST WEAKLY ADHERENT CELLS**
BIOLOGISCH ABBAUBARES GEWEBEGERÜST MIT SEKUNDÄRMATRIX ZUM HOSTEN SCHWACH ADHÄRENTER ZELLEN
ÉCHAFAUDAGE DE TISSU BIODÉGRADABLE AYANT UNE MATRICE SECONDAIRE POUR HÉBERGER DES CELLULES FAIBLEMENT ADHÉRENTES

(30) Priority: 28.05.2021 US 202163194770 P
(43) Date of publication of application: 10.04.2024
(73) Proprietor: The United States of America, as represented by The Secretary, Department of Health and Human Services, Bethesda, MD 20817 (US)
(72) Inventor: SHARMA, Ruchi, Bethesda, Maryland 20892 (US); BHARTI, Kapil, Bethesda, Maryland 20892 (US); MAMINISHKIS, Arvydas, Bethesda, Maryland 20814 (US); ORTOLAN, Davide, Bethesda, Maryland 20892 (US); NGUYEN, Eric, Bethesda, Maryland 20892 (US)
(74) Representative: Symbiosis IP Limited
(86) International application number: PCT/US2022/031107
(87) International publication number: WO 2022/251477

(56) References cited:
- WO-A1-2020/106622

## Description

### CROSS-REFERENCE TO RELATED APPLICATION

This application claims the benefit of U.S. Application No. 63/194,770, filed May 28, 2021.

### STATEMENT OF GOVERNMENT SUPPORT

This invention was made with Government support under project number Z01#: ZIA EY000533-04 by the National Institutes of Health, National Eye Institute. The United States Government has certain rights in the invention.

### FIELD OF THE DISCLOSURE

This relates to the field of ophthalmology, specifically to scaffolds having a layer of poly(lactic-co-glycolic acid) (PLGA), and a layer containing polycaprolactone (PCL) loops attached to the PLGA. Such scaffolds can be seeded with retinal pigment epithelial (RPE) cells and photoreceptor progenitor (PRP) cells, and then implanted into the retina of a subject to treat a retinal degenerative disease, retinal dysfunction, retinal degradation, retinal damage, or loss of retinal pigment epithelium.

### BACKGROUND

The retinal pigment epithelium is a monolayer of cells located in the back of the eye. On the apical side, RPE cells form contact with photoreceptors through apical processes. On the basal side, they face the capillaries of the choroid. The main function of retinal pigment epithelium is to exchange metabolites with photoreceptors and choroid vessels and maintain retinal homeostasis.

In human central retina, there is a cone-enriched region named the macula, which is responsible for sharp and color vision. Evidence suggests that the human RPE monolayer is a phenotypically and functionally heterogeneous population of cells. Phenotypically, macular RPE cells are smaller in size than peripheral RPE cells (Bhatia et al., Molecular Vision, 22, 898-9162016, 2016) and have petal-like apical processes that predominantly support cone photoreceptors. In comparison, peripheral RPE cells have finger-like apical processes, which predominantly support rod photoreceptors (Pfeffer & Fisher, Journal of Ultrastructure Research, 76(2), 158-172. https://doi.org/10.1016/S0022-5320(81)80014-71981; Steinberg, Zeitschrift Fur Zellforschung Und Mikroskopische Anatomie, 143(4), 451-463, doi.org/10.1007/BF003067651973). Functionally, peripheral RPE cells express higher levels of Na/K ATPase than macular RPE cells (Burke et al., Investigative Ophthalmology & Visual Science, 32(7), 2042-2046, 1991). RPE lysosomal enzymes activity is also regionally different: Cathepsin D is more active in macular RPE cells (Boulto et al., The British Journal of Ophthalmology, 78(2), 125-129, 1994; Burke & Twining, The British Journal of Ophthalmology, 78(2), 125-129, 1988; Cabral et al., Investigative Ophthalmology & Visual Science, 31(4), 670-676. 1990), while acid phosphatase, β-glucuronidase, and N-acetyl-β-glucosaminidase are more active in peripheral RPE cells (Cabral et al., *supra,* 1990). At least 5% of genes are differentially expressed between macular and peripheral RPE cells (Radeke et al., Experimental Eye Research, 85(3), 366-380, 2007; van Soest et al., Molecular Vision, 13, 1608-1617, 2007). Metabolically, macular and peripheral human RPE/choroid consume and release different levels of metabolites (Li et al., BioRxiv, 2020.07.10.196295. doi.org/10.1101/2020.07.10.196295, 2020

WO2020/106622 A discloses a tissue replacement implant, comprising polarized retinal pigment epithelial cells on a poly(lactic-co-glycolic acid) (PLGA) scaffold, wherein the PLGA scaffold is 20-30 microns in thickness, has a DL-lactide/glycolide ratio of about 1:1, an average pore size of less than about 1 micron, and a fibre diameter of about 150 to about 650 nm. The implant can be used for treating a subject with a retinal degenerative disease, retinal or retinal pigment epithelium dysfunction, retinal degradation, retinal damage, or loss of retinal pigment epithelium.

A need remains for methods to differentiate the different types of RPE cells, such as macular and peripheral RPE.

### SUMMARY OF THE DISCLOSURE

Provided herein are scaffolds that can be used to seed retinal pigment epithelial (RPE) cells, photoreceptor progenitor (PRP) cells, or both RPE cells and PRP cells, for example RPE cells generated using the disclosed methods. Scaffolds containing RPE cells and PRP cells can be implanted into the eye of a subject to treat a retinal degenerative disease, retinal dysfunction, retinal degradation, retinal damage, or loss of retinal pigment epithelium.

Provided herein are scaffolds that include two layers. The first layer in some examples is about at least 5 microns in height (e.g., thickness) (such as about 5 to about 40 microns, such as 5, 10, 15, 20, 25, 30, 35, or 40 microns) and has an average pore size of at least about 0.2 microns (such as about 0.2 to about 2 microns, such as 0.2, 0.5, 1, or 2 microns) in diameter. The first layer includes poly(lactic-co-glycolic acid) (PLGA) having a DL-lactide/glycolide ratio of at least about 0.25:0.25 (such as 1:1), and a fiber diameter of at least 150 nm (such as about 150 to about 650 nm, such as 150, 200, 300, 400, 450, 500, 600, or 650 nm). The second layer includes polycaprolactone (PCL) loops with a diameter of at least 5 microns (such as about 5 to about 300 microns, such as 5, 10, 25, 30, 50, 100, 200, or 300 microns), wherein the second layer is attached to the first layer. In some examples, the second layer of PCL loops is attached to the first layer using electrospinning.

The disclosed scaffolds can further include a coating containing a recombinant cellular adhesion protein, such as one or more of vitronectin, laminin, and fibronectin.

The disclosed scaffolds can further include retinal pigment epithelial (RPE) cells, photoreceptor progenitor (PRP) cells, or both RPE cells and PRP cells, such as RPE cells made using the methods provided herein (such as macular, central, and/or peripheral RPE cells). Thus, in some examples, the disclosed scaffolds include induced pluripotent stem cells (iPSCs), which are cultured on the scaffold to generate RPE cells. In one example, the RPE cells are macular, central and/or peripheral RPE cells, generated by a method that includes: a) culturing pluripotent stem cells, such as induced pluripotent stem cells (iPSCs), in a retinal induction medium to initiate differentiation of the cells into RPE progenitor cells; b) culturing the RPE progenitor cells in a retinal differentiation medium to further differentiate the RPE progenitor cells into committed RPE cells; c) culturing the committed RPE cells in a retinal medium to form immature RPE cells; and d) culturing the immature RPE cells in a RPE maturation medium including a retinoic acid receptor (RAR) antagonist and/or a canonical Wnt inhibitor, thereby producing macular, central or peripheral human RPE cells. In some embodiments, such a culturing method is performed on the scaffold (e.g., the pluripotent stem cells, such as induced pluripotent stem cells (iPSCs), are added to the scaffold and cultured as described herein to generate macular, central or peripheral human RPE cells. In some embodiments, the method produces macular human RPE cells, and the RPE maturation medium includes the RAR antagonist but not the canonical Wnt inhibitor. In further embodiments, the method produces central human RPE cells, wherein the RPE maturation medium includes both the RAR antagonist and the canonical Wnt inhibitor. In yet other embodiments, the method produces peripheral human RPE cells, wherein the RPE maturation medium includes the canonical Wnt inhibitor but not the RAR antagonist. Such a method can be used to prepare RPE cells from any mammal, including, but not limited to, human RPE cells.

Also provided is a non-biodegradable porous polycarbonate membrane containing a scaffold disclosed herein.

Also provided are kits that include a scaffold disclosed herein. Such kits may further include one or more of vitronectin, laminin, fibronectin, a snap-well culture system, a (polytetrafluoroethylene (PTFE) O-ring, retinal induction media, retinal differentiation media, retinal maturation media, retinal media, a non-biodegradable porous polycarbonate membrane, RPE cells, and PRP cells.

Also provided are pharmaceutical compositions that include a scaffold provided herein, for example for use in treating a retinal degenerative disease, retinal dysfunction, retinal degradation, retinal damage, or loss of retinal pigment epithelium.

Also provided are methods of using the scaffolds provided herein for treating a subject in need thereof, by implanting the scaffold into a retina of the subject. In some examples, the subject has a retinal degenerative disease, retinal dysfunction, retinal degradation, retinal damage, or loss of retinal pigment epithelium. In some examples, following implantation of a scaffold disclosed herein into a retina, the first layer of the scaffold degrades within about 3 months, while the second layer of the scaffold remains longer, for example for up to about 1 year.

Also provided are methods of culturing RPE and PRP cells onto the cells, for example for use in the methods of treatment provided herein.

The foregoing and other features of the disclosure will become more apparent from the following detailed description of several embodiments which proceeds with reference to the accompanying figures.

### BRIEF DESCRIPTION OF THE FIGURES

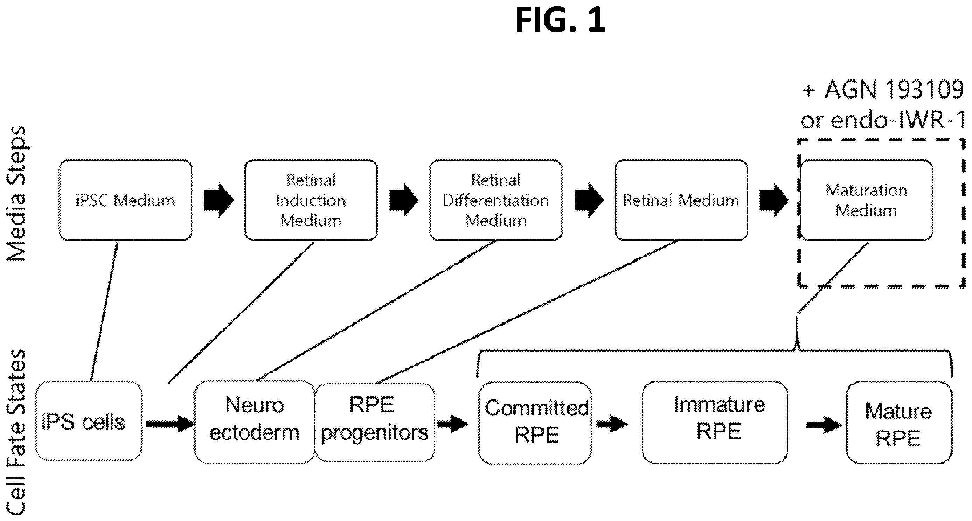

FIG. 1. **Macular and peripheral iPSC-RPE differentiation protocol.** Scheme of macular and peripheral iPSC-RPE differentiation protocol. AGN 193109 and endo-IWR-1 are added to iPSC-RPE in RPE Maturation Medium to obtain macular or peripheral phenotypes, respectively. The flow chart shows that the drugs are added to the RPE Maturation Medium and indicates the cell fate status corresponding to each step.

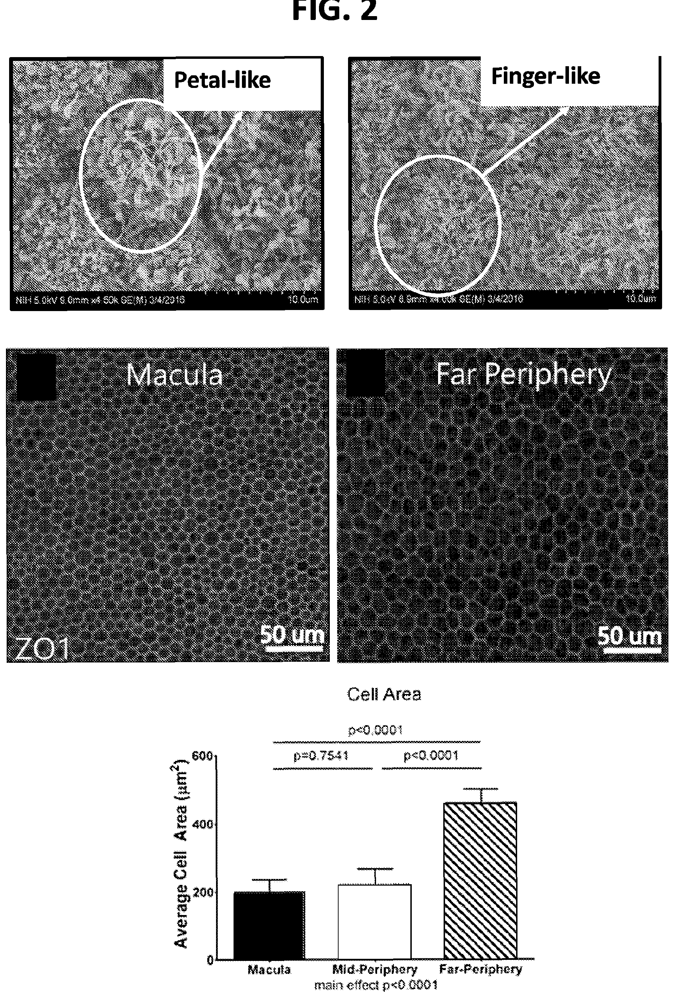

**FIG. 2****. Human macular and peripheral RPE** cells are **phenotypically different.** The human RPE monolayer is a phenotypically and functionally heterogeneous population of cells. This figure shows two phenotypic differences between macular and peripheral RPE. On top, scanning electron microscopy (SEM) images of iPSC-RPE cells grown *ex vivo* show examples of cells enriched in petal- or finger-like apical processes. Petal-like apical processes are sheet-like protrusions (with undulations) of the apical RPE cell membrane, which wrap around cone photoreceptors; these are abundant in the macula. Finger-like apical processes resemble microvilli and selectively support rod photoreceptors, which are rich in the peripheral retina (Steinberg and Wood, Proceedings of the Royal Society of London - Biological Sciences, 187(1089), 461-478, 1974; Fisher and Steinberg, The Journal of Comparative Neurology, 206(2), 131-145, 1982). At the bottom of the figure, two images of RPE cell borders from macular and peripheral regions show the difference in cell area. The bar graph illustrates the quantification of cell area between macula and periphery (Bhatia et al., Molecular Vision, 22, 898-916, 2016).

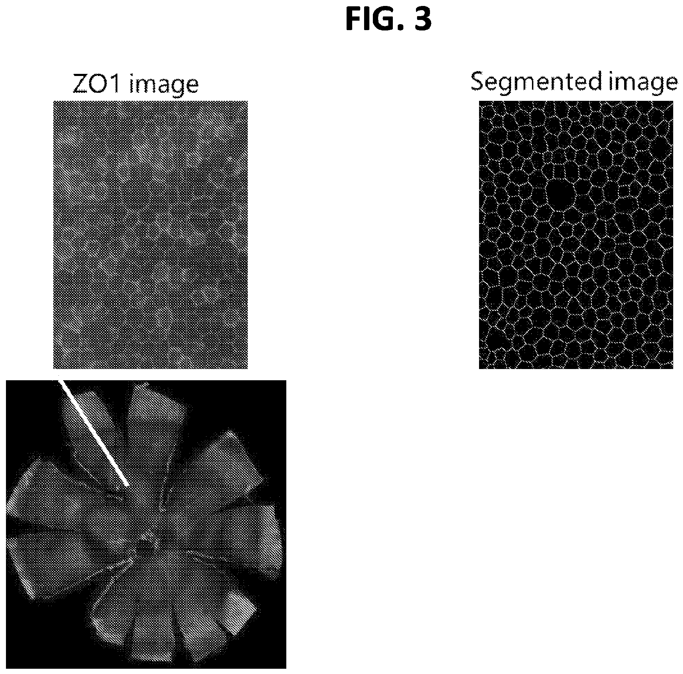

**FIG. 3****. Regional morphometric analysis of human RPE cells.** Entire human RPE flat-mounts were prepared and stained for RPE cell borders to analyze RPE shape metrics (left). A zoomed-in view shows individual RPE cells stained for cell borders with anti-ZO1 antibody. A machine learning algorithm was trained to identify and segment RPE cell borders. The algorithm produces binary images of RPE cells that become the input for the REShAPE software for cell shape analysis.

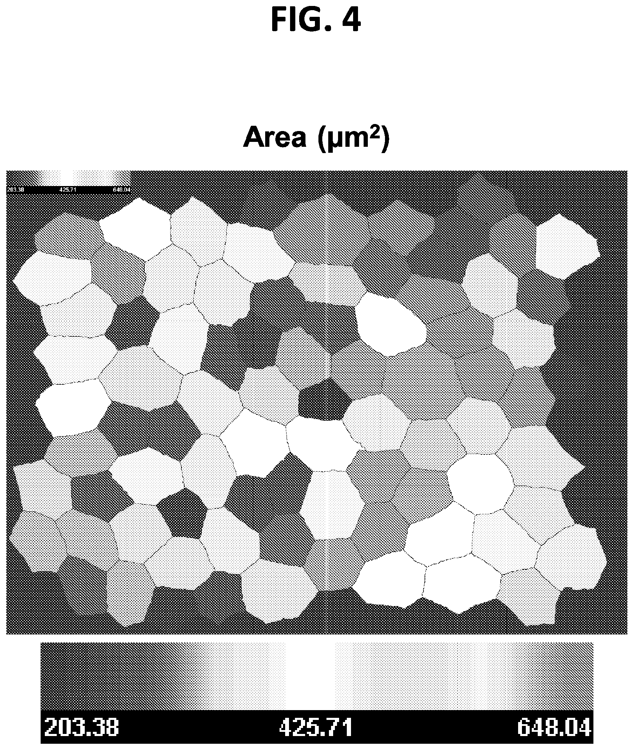

**FIG. 4****. Regional morphometric analysis of human RPE cells.** REShAPE (**R**etinal **E**pithelium **S**hape **A**nd **P**igment **E**valuator) is used to analyze every cell in a field of view that has been successfully segmented, REShAPE provide quantification of more than 25 different shape metrics. The raw data are stored in a spreadsheet to allow for statistical analysis. In addition, the software creates images of segmented cells for every metric analyzed, where every cell is color-coded according to the raw values. In this image, the segmented RPE cells are color-coded according to their area. Small cells appear in darker cells (left side of scale) and big cells in grey (right side of scale). The thermal scale is shown as an insert.

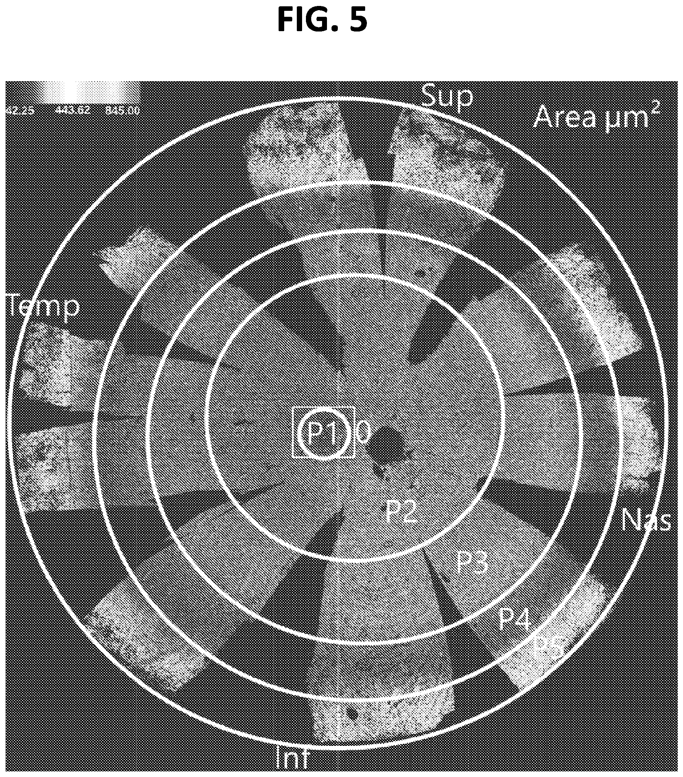

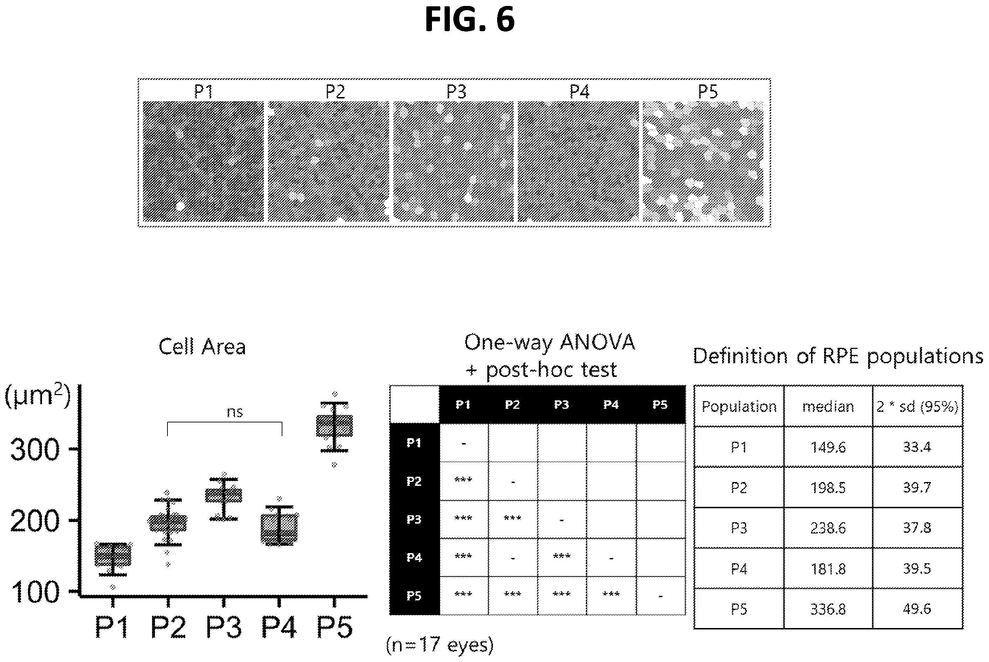

**FIG. 5****. Regional morphometric analysis of human RPE cells.** Low magnification map of an entire human RPE monolayer. It contains about 3-4 million RPE cells, which are color-coded according to cell area. A thermal scale was used. Dark grey corresponds to RPE cells with small area, and the RPE cells with big area are also shown, see the scale. The dark spot in the center of the flat-mount represents macular RPE cells. The RPE cell area grows gradually with eccentricity, except for a peripheral ring of small RPE cells. Using cell area as a reference, five significantly different RPE populations were identified, which are named "Population 1" (P1) to "Population 5" (P5), see FIG. 6, going from macular to peripheral cells, in this figure.
**FIG. 6****. Regional morphometric analysis of human RPE cells.** Higher magnification images to display single cells for each human RPE population (P1 through P5) identified. A thermal scale was used. RPE cells with small area, and RPE cells with big area, were identified. The boxplot shows the values of RPE cell area for each RPE population. Box limits represent the first and third quartile, the central line shows the median and the whiskers indicate the 5^{th} and 95^{th} percentile, so that the range specifies 90% of the data. The table of one-way ANOVA shows the multiple comparison between each RPE population. RPE populations are defined according to cell size as population median ± 2 standard deviations, which encompasses 95% of the data. The values are shown in the table on the right.

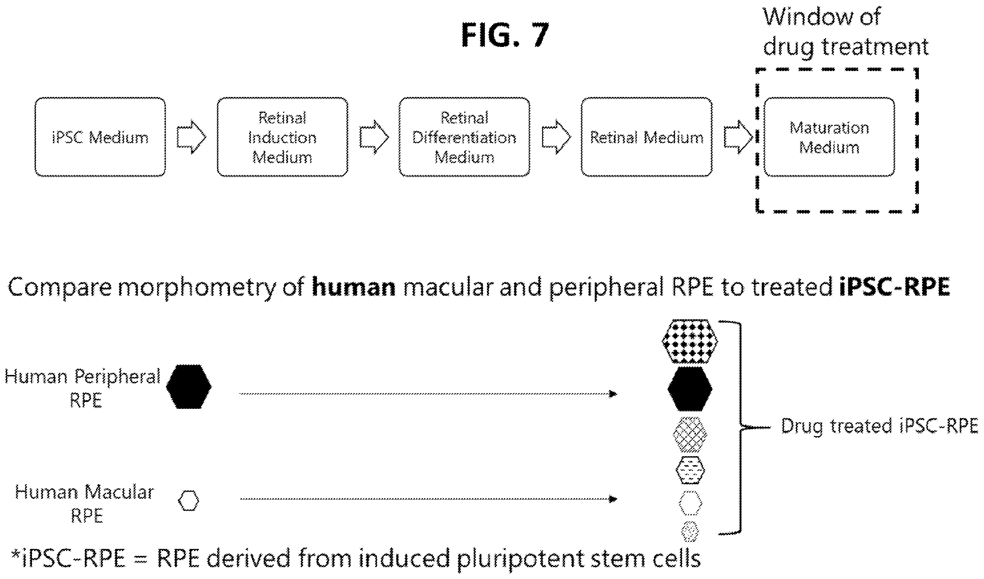

**FIG. 7****. Comparison of morphometry between human RPE cells and iPSC-RPE.** 115 compounds were screened on iPSC-RPE to reproduce morphometries of human RPE populations. The compounds are activators and inhibitors of developmental pathways. All the compounds were tested by adding them to the RPE maturation medium for 30 days (as shown in the schematic flow chart). At the end of the treatment, cell areas were quantified using REShAPE. The results were compared to the measurements of macular and peripheral RPE cells to select the compounds that recapitulate human RPE dimensions. The bottom of the figure shows a schematic of the comparison of cell areas between macular and peripheral human RPE (from flat-mounts) and iPSC-RPE cells treated with different compounds.

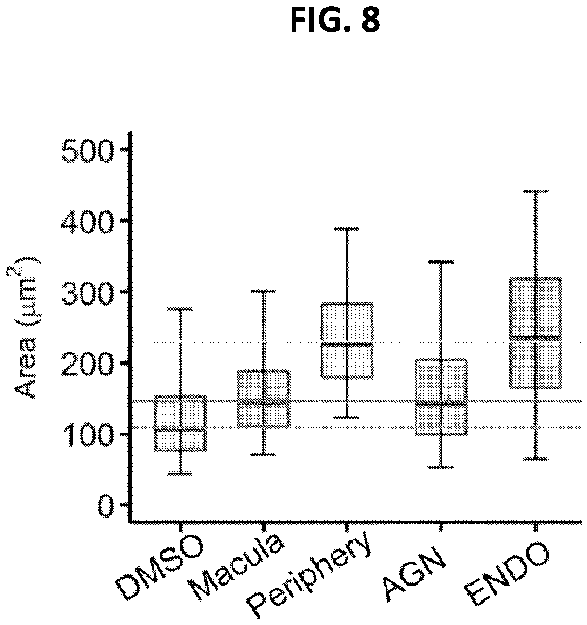

**FIG. 8****. Comparison of morphometry between human RPE cells and iPSC-RPE.** The results of the screening of compounds indicate that adding retinoic acid inhibitors, such as AGN 193109, or canonical Wnt inhibitors, such as endo-IWR-1, to iPSC-RPE reproduces macular and peripheral cell sizes *in vitro,* respectively. Human RPE population P3 was chosen as reference for peripheral RPE cells. One-way ANOVA and a post-hoc test for multiple comparisons were used for statistical analysis. iPSC-RPE cells treated with DMSO are also shown in the graph as a control for untreated iPSC-RPE. Data is displayed as boxplots, where box limits represent the first and third quartile, the central line shows the median and the whiskers indicate the 5^{th} and 95^{th} percentile, so that the range specifies 90% of the data.

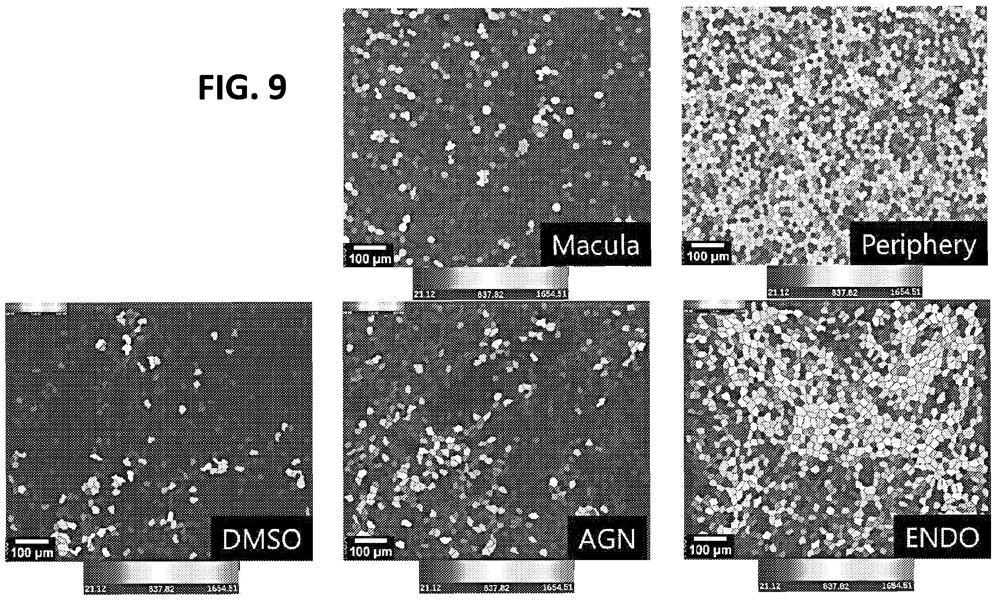

**FIG. 9****. Comparison of morphometry between human RPE cells and iPSC-derived RPE.** Images color-coded by cell area demonstrate the comparison between human RPE and iPSC-RPE cells. Human RPE population P3 was chosen as reference for peripheral RPE cells. Adding retinoic acid inhibitors, such as AGN 193109, or canonical Wnt inhibitors, such as endo-IWR-1, to iPSC-RPE reproduces macular and peripheral cell sizes *in vitro,* respectively. The same range of parameters was used for the thermal scale in the color-coded images; thus, the area can be directly compared between the images. This data complements the boxplot graph of the previous figure. Scale bar = 100 um.

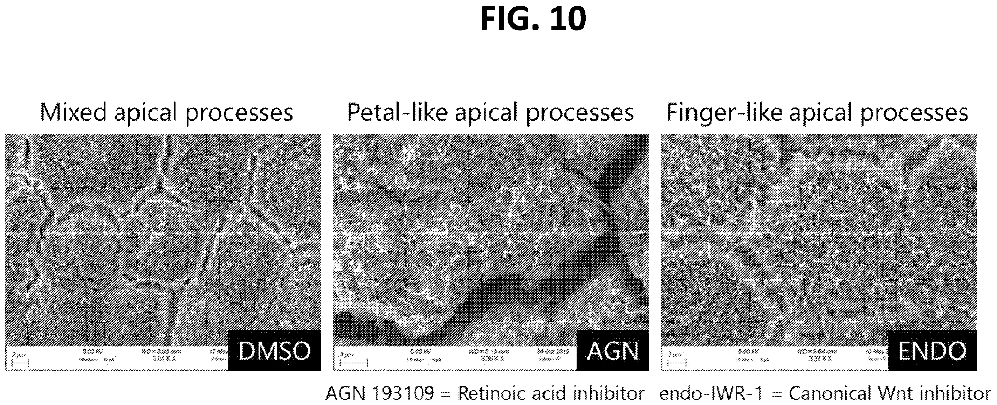

**FIG. 10****. Comparison of apical structures between treated iPSC-derived RPE.** Top-to-bottom view of iPSC-RPE cells shows the structures of apical processes. AGN 193109 enriched cells with petal-like apical processes with undulation, a characteristic of macular RPE cells. Endo-1WR-1 enriched cells with finger-like apical processes, a characteristic of peripheral RPE cells. Both types of apical processes can be seen in the DMSO control.

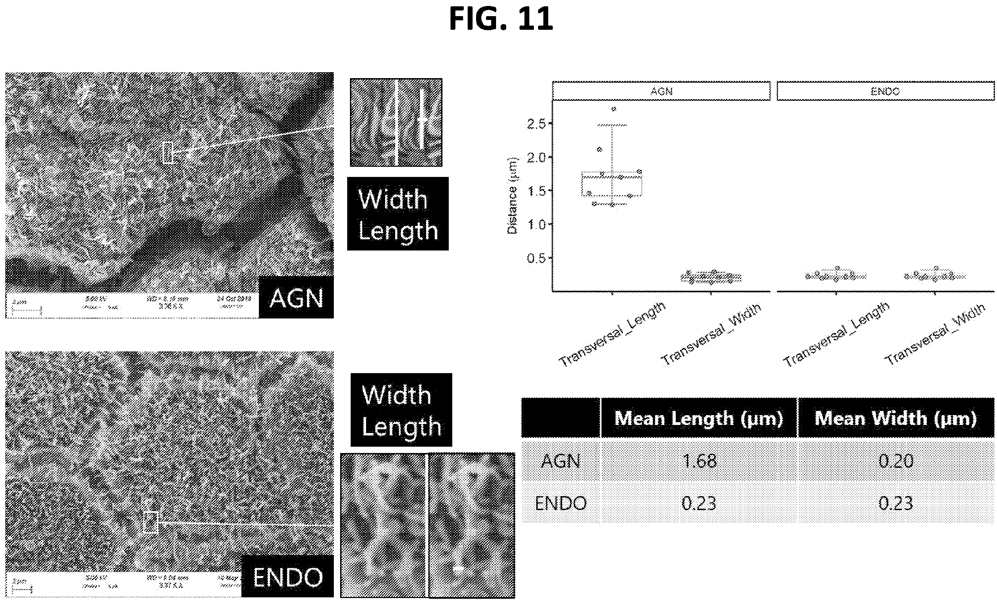

**FIG. 11****. Defining petal (undulated) and finger -like apical processes.** The transversal section of apical processes was measured to precisely define their shape. Petal-like apical processes, obtained by treating RPE cells with AGN 193109, have an average transversal length of 1.68 µm (length min 0.5, max 4 µm) and width of 0.20 µm (width min 0.1, max 1 µm). The petal-like apical processes are also wavy along their length. The width of the undulation ranges from 0.2 to 2 µm. Finger-like apical processes, obtained by treating RPE cells with endo-IWR-1, are cylindrical and have the same average transversal length and width of 0.23 µm (min 0.1, max 1 microns). An example of how measurements of the apical processes was done is shown for each type of apical processed.

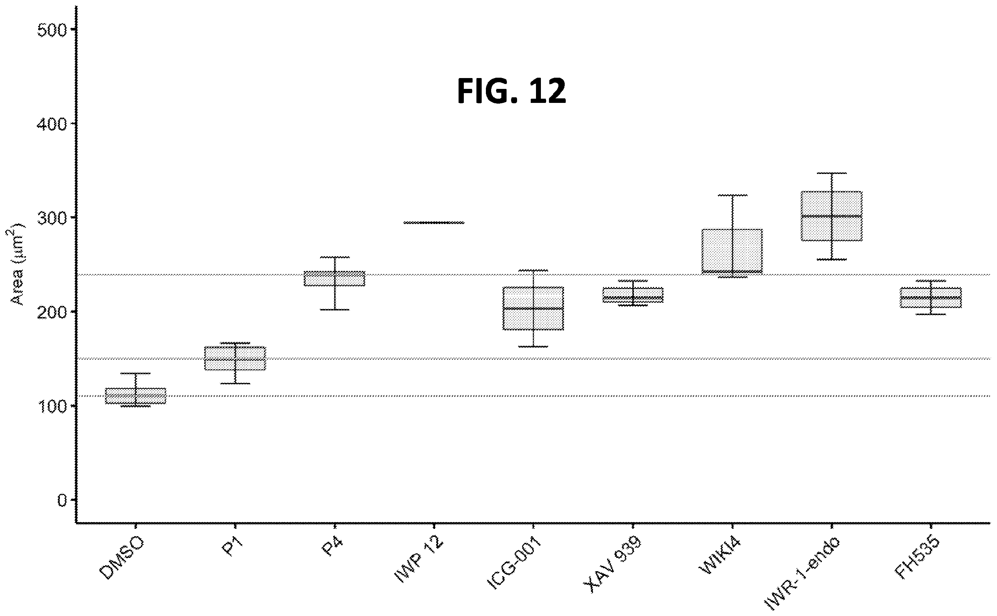

**FIG. 12****. Comparison of cell area.** The graph shows the comparison of cell area between macular and far peripheral RPE cells (P1 and P4 respectively) and iPSC-RPE cells treated with different drugs which inhibit the canonical Wnt pathway. This data comes from the screening of compounds that led to the identification of AGN 193109 and endo-IWR-1. iPSC-RPE cells treated with DMSO are also shown in the graph as a control for untreated iPSC-RPE. The graph shows that other canonical Wnt inhibitors can be used to recapitulate peripheral RPE phenotype. Indeed, all of the canonical Wnt inhibitors increased iPSC-RPE cell area to the dimension of peripheral RPE cells (P3). The results indicated that canonical Wnt inhibition generates peripheral RPE cells. Data is displayed as boxplots, where box limits represent the first and third quartile, the central line shows the median and the whiskers indicate the 5^{th} and 95^{th} percentile, so that the range specifies 90% of the data. One-way ANOVA and a post-hoc test for multiple comparisons were used for statistical analysis.

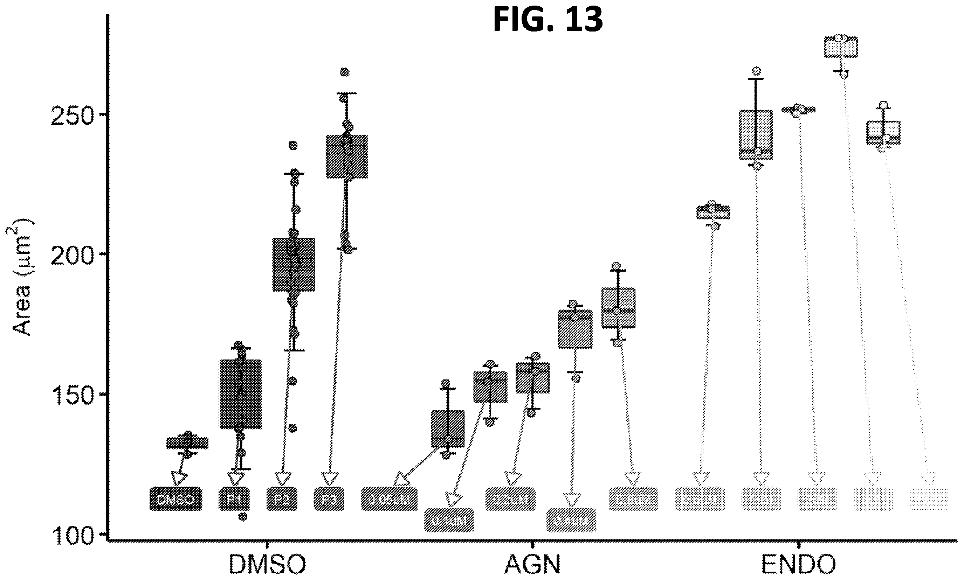

**FIG. 13****. Testing different concentrations of AGN 193109 and endo-IWR-1 to reproduce macular (P1), central (P2) and peripheral (P3) cells.** A range of concentrations of AGN 193109 and endo-IWR-1 were tested that could be used to reproduce macular, central and peripheral RPE populations (labeled P1, P2 and P3, respectively). Population 1 (P1, macular cells) can be reproduced with 0.1mM-0.2mM of AGN 193109. Population 3 (P3, peripheral) can be reproduced with 1mM-4mM of endo-IWR-1. A gradient of AGN 193109 and endo-IWR-1 could be used to replicate human RPE population 2 (P2, central) - central RPE cells. In one example, this can be reproduced with 25nM-50nM of AGN 193109 and 0.1mM-0.5mM of endo-IWR-1. Boxplots relative to DMSO, P1, P2 and P3 are plotted on the left side of the graph as a reference for cell size comparison. Five concentrations were tested for AGN 193109 and endo-IWR-1.

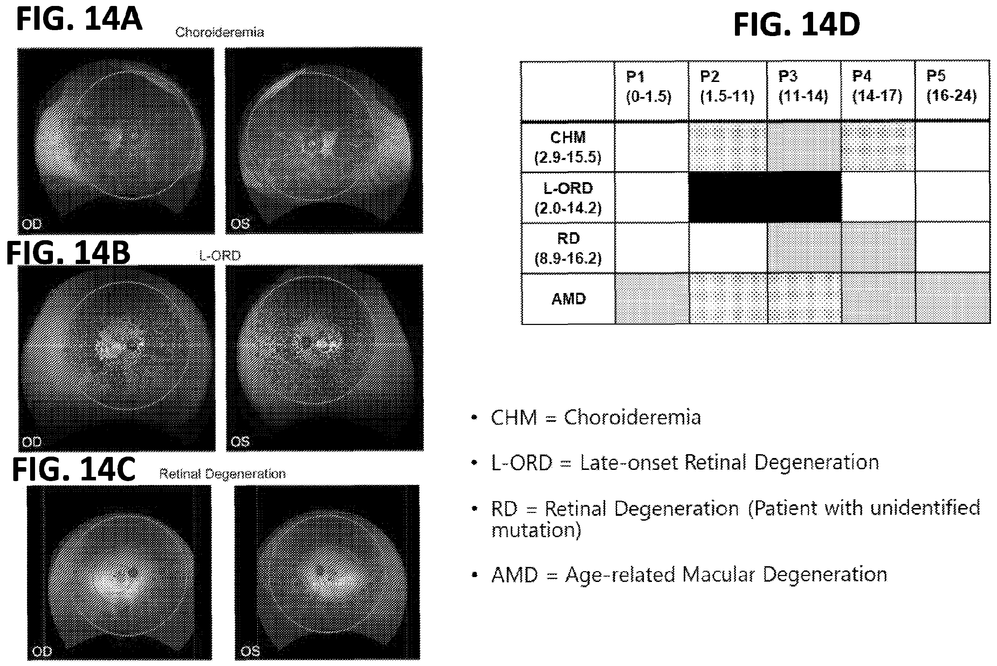

**FIGS. 14A-14D****. Different RPE populations are affected in different retinal diseases.** Fundus images of patient eyes showing damage in different regions of RPE in different ocular diseases (A) Choroideremia; (B) late onset retinal degeneration (LORD); (C) undiagnosed retinal degeneration. Different types of retinal degenerative diseases appear to affect a different subset of RPE populations. RPE degeneration in different diseases is observed in fundus images and their location can be quantified. (D) The table summarizes which RPE population is mostly affected in the different diseases. Filled-in squares signify that the entire RPE population is affected. Dotted squares indicate that the population is partially affected. For RPE populations, the numbers in parenthesis indicate the distance of each RPE population from the center of the eye (in millimeters). For the diseases, the numbers in parenthesis specify the location of RPE degeneration, expressed as millimeters from the center of the eye. AMD is acute macular degeneration and RD is retinal degeneration in a patient with an unidentified mutation. Populations with dots or filled in squares can be used for the treatment of the indicated subject. P1 is macular, P2 is central, P3 is peripheral RPE, P4 is far-peripheral, and P5 is ora serrata RPE cells. Dots show degeneration in later disease stages (also means partial degeneration in earlier stages). Squares show degeneration in earlier disease stages. The square areas will be transplanted earlier in the disease process than dot areas. Thus, the dots and squares provide information on the timing that specific cell populations can be used.

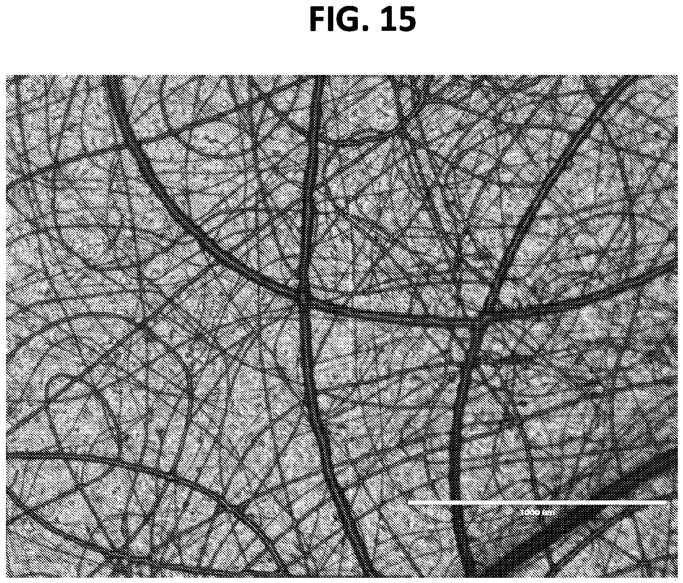

**FIG. 15****.** Digital scanning electron microscope (SEM) image of an example bi-layer "fuzzy" scaffold construct provided herein, showing the mesh-like structure of darker and thinner PCL loops on top of a PLGA spun scaffold (grey appearance between darker PCL loops).

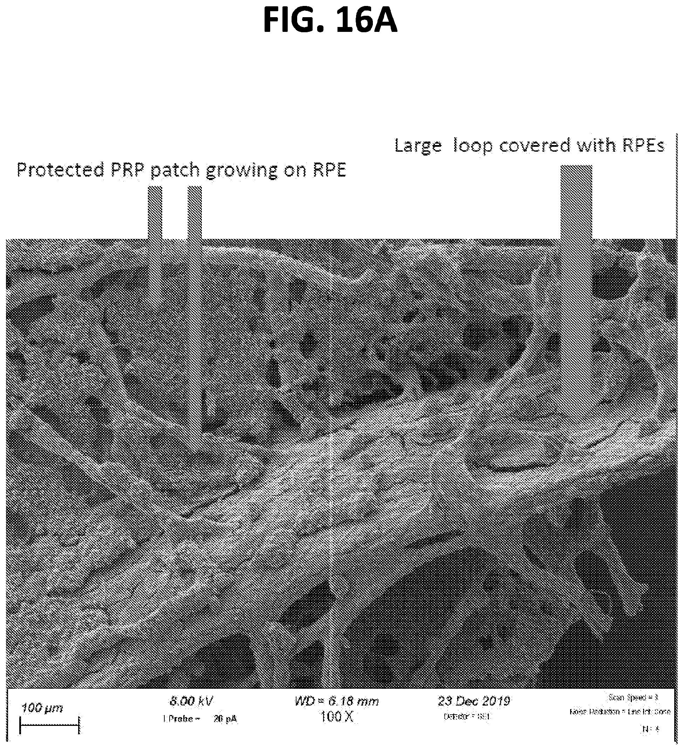

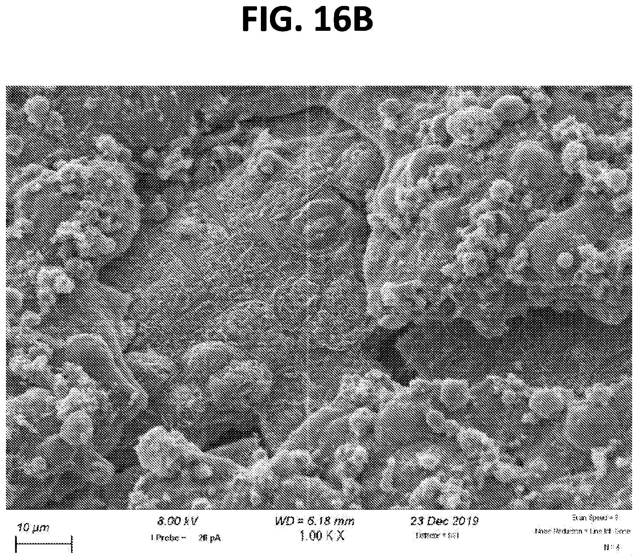

**FIGS. 16A-16B****.** Digital scanning electron microscope (SEM) images (A) 100X magnification, (B) 1000X magnification, of an example bi-layer "fuzzy" scaffold construct provided herein, seeded with RPE and photoreceptor progenitor (PRP) cells. In (B) arrows indicate RPE apical processes, an indication of healthy and mature RPE cells.

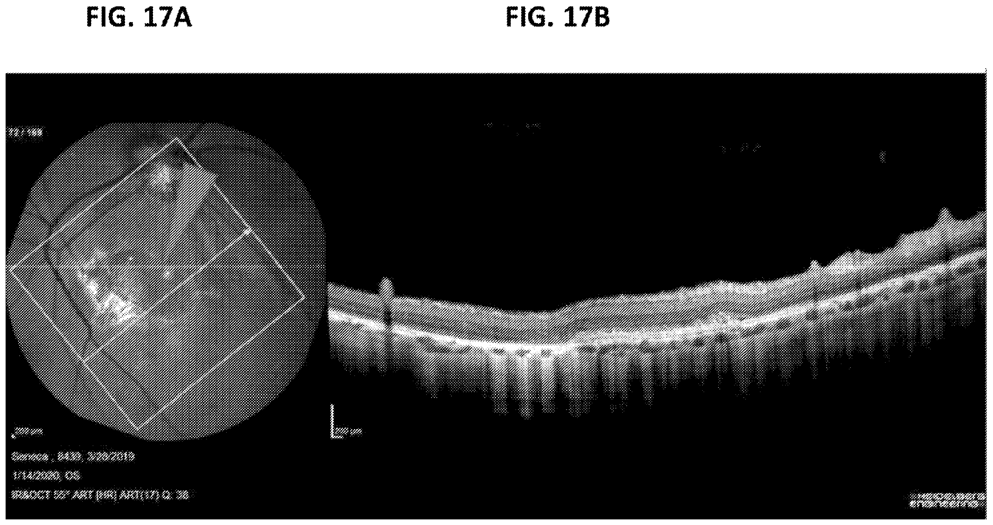

**FIGS. 17A and 17B****.** Digital (A) scanning laser ophthalmoscopy (SLO) image showing the implanted scaffold (arrow) and (B) optical coherence tomography (OCT) B-scan across the implanted scaffold.

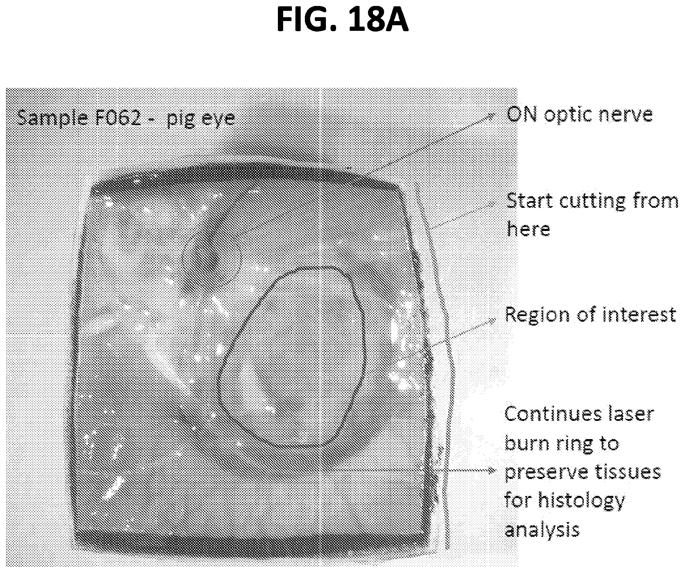

**FIGS. 18A - 18C****.** (A) Digital image of a dissected and fixed eye tissue showing area where implant was placed two-months earlier (region of interest). (B and C) Digital image of H&E stained paraffin embedded section taken across the scaffold. Images were collected with 20X objective using AxioScan (Zeiss) with 0.173 µm/ pixel resolution. This device produces images that can be zoomed in and out as needed.

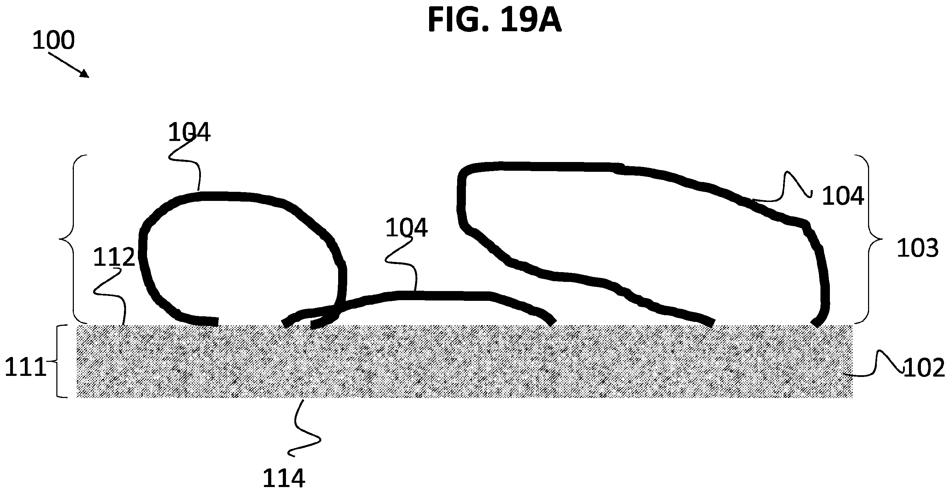

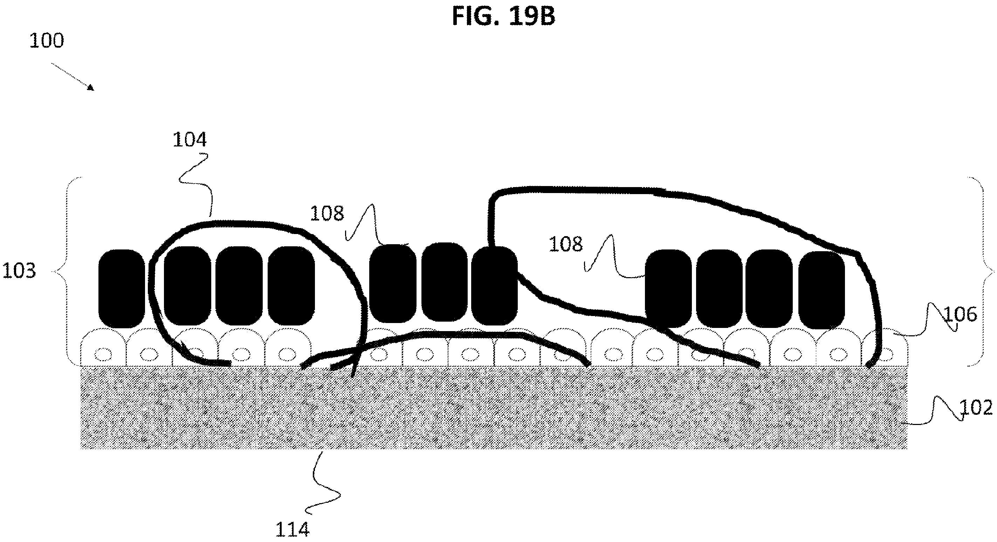

**FIGS. 19A** **and** **19B****.** Schematic drawing (not to scale) showing (A) exemplary bi-layer scaffold 100 comprising a first layer PLGA scaffold 102, and a second layer 103 containing PCL loops 104. Here, loops 104 are shown "closed", but they can be open (e.g., linear). The first layer 102 includes a upper surface 112, and a lower surface 114. (B) Bi-layer scaffold 100 seeded with RPE cells 106 (which can be generated on the scaffold from pluripotent stem cells, such as iPScs, using the methods provided herein) and PRP cells 108.

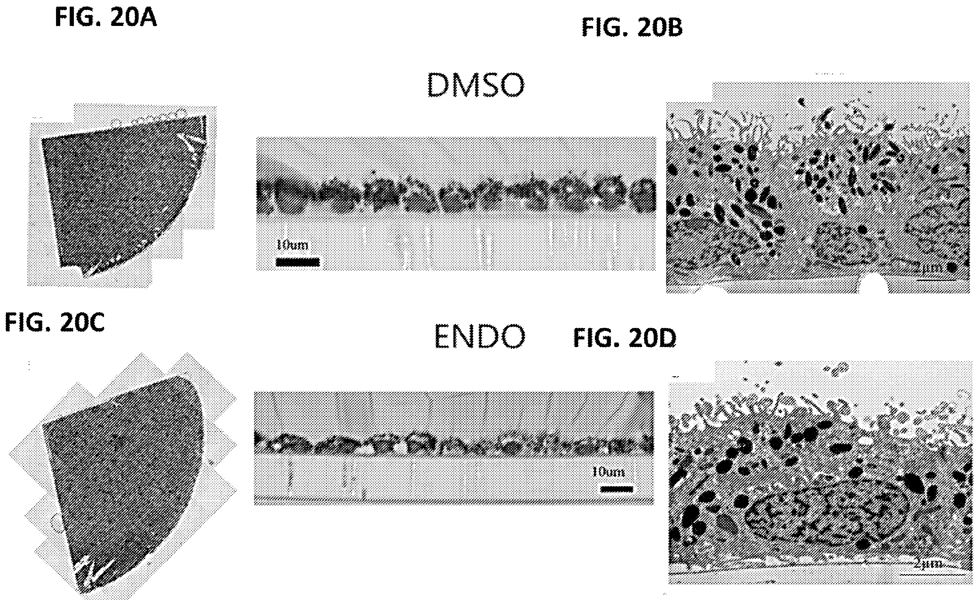

**FIGS. 20A-20F****. Pigmentation and cell morphology.** iPSC-RPE pigmentation levels (A, C, E) were examined after treatment with DMSO/control (A, B), endo-IWR-1 (C, D) or AGN 193109 (E, F), to ensure that the compounds are not detrimental. Gross cell morphology was also analyzed (center) to detect possible abnormalities. Fine intracellular structures were examined for alterations with transmission electron microscopy (right). Endo-IWR-1 and AGN 193109 did not alter pigmentation levels nor gross or fine cell morphology.

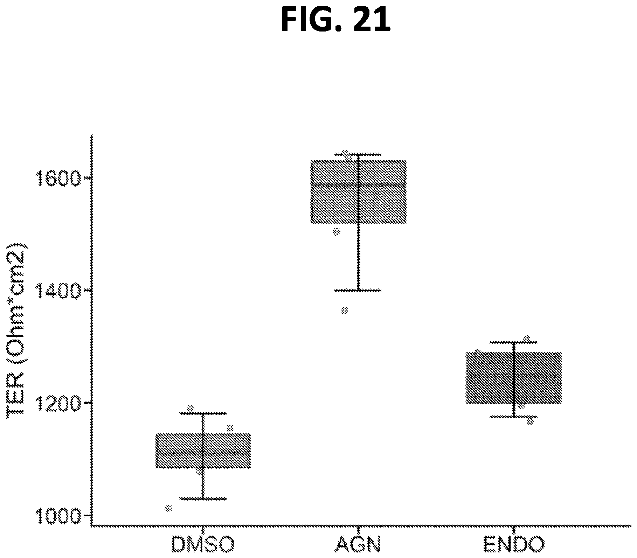

**FIG. 21****. Trans-epithelial electrical resistance (TER).** Graph showing TER (a measure of the tightness of the monolayer) observed for AGN 193109- and endo-IWR-1-treated cells. A cutoff of 400 ohm*centimeter squared was used to exclude cells with low TER. AGN 193109- and endo-IWR-1-treated cells were well above the threshold. Data is displayed as boxplots, where box limits represent the first and third quartile, the central line shows the median and the whiskers indicate the 5th and 95th percentile, so that the range specifies 90% of the data.

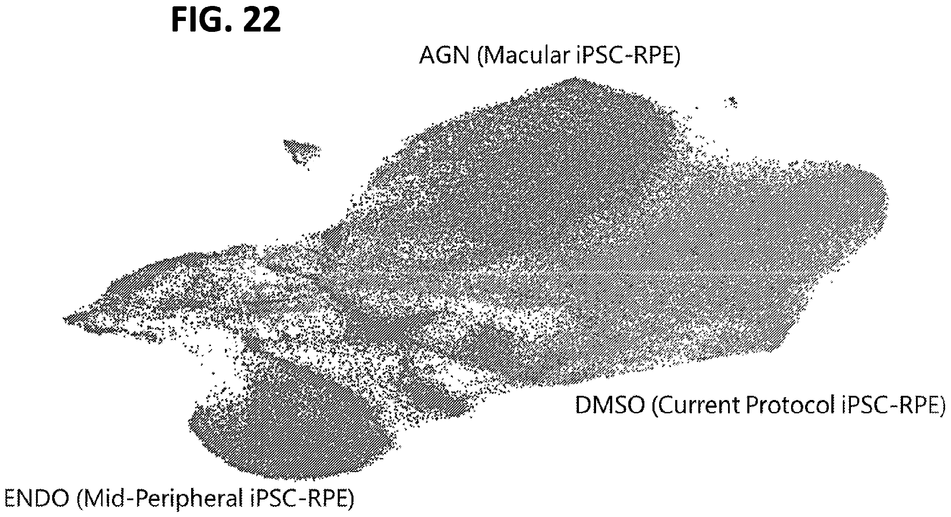

**FIG. 22****. Single Cell RNA Sequencing,** Each dot represents the transcriptome of a single cell. The distance between dots shows how different these cells are (the closer, the more similar). The three sets cluster separately from each other; therefore, their transcriptome is fairly distinct. AGN 193109-treated cells; endo-IWR-1-treated cells, and DMSO-treated cells are shown.

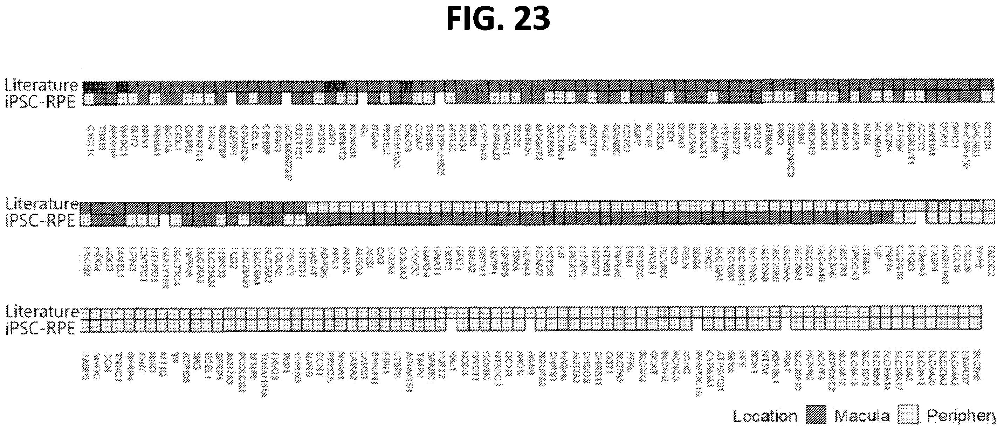

**FIG. 23****. Bulk RNA sequencing.** The three heatmaps show expression levels of specific genes from the literature (Radeke et al., Experimental Eye Research 85 (3), 366-380, doi: 10.1016/j.exer.2007.05.006 (2007); Whitmore et al., Experimental Eye Research 129, 93-106, doi: 10.1016/J.EXER.2014.11.001 (2014); Voigt et al., Experimental Eye Research 184, 234-242, doi: 10.1016/J.EXER.2019.05.001 (2019). van Soest et al., Molecular Vision 13, 1608-17, ncbi.nlm.nih.gov/pubmed/17893662 (2007); Li et al. iScience 23 (11), 101672, doi: 10.1016/j.isci.2020.101672 (2020); Ishibashi et al., Investigative Ophthalmology & Visual Science 45 (9), 3291, doi: 10.1167/iovs.04-0168 (2004) in the top row and the bulk RNA sequencing in the bottom row. The X axis is labelled with genes of interest. Dark Grey corresponds to genes that are more expressed in the macula (literature) or AGN 193109-treated cells (current data, iPSC-RPE). Light grey corresponds to genes that are more expressed in the periphery (literature) or endo-IWR-1-treated cells (current data, iPSC-RPE).

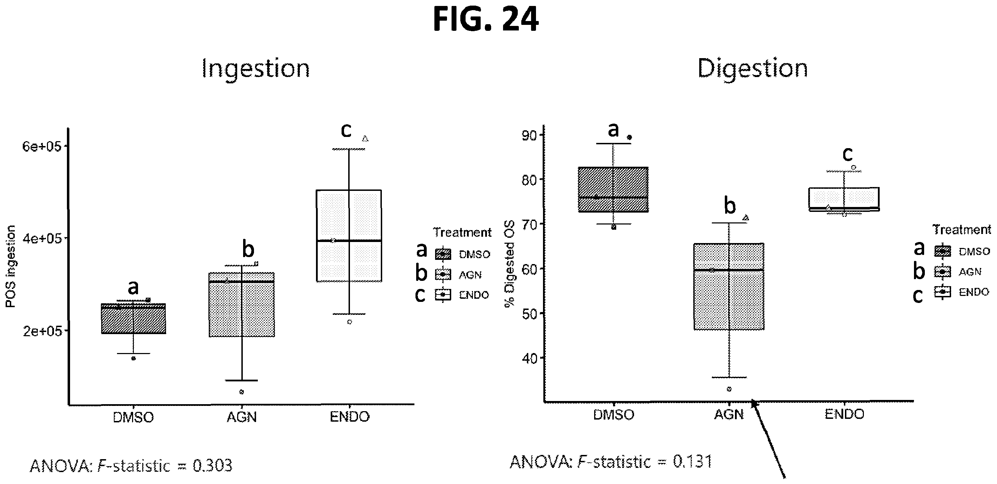

**FIG. 24****. Phagocytosis test.** Data is displayed as boxplots, where box limits represent the first and third quartile, the central line shows the median and the whiskers indicate the 5^{th} and 95^{th} percentile, so that the range specifies 90% of the data.

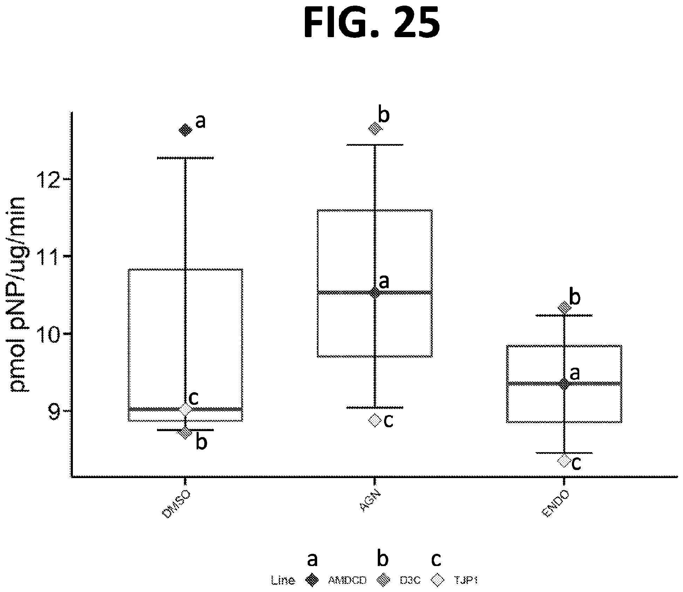

**FIG. 25****. Acid Phosphatase Activity.** Graph showing higher acid phosphatase activity in AGN 193109-treated cells (macular iPSC-RPE) than endo-IWR-1. Data is displayed as boxplots, where box limits represent the first and third quartile, the central line shows the median and the whiskers indicate the 5^{th} and 95^{th} percentile, so that the range specifies 90% of the data.

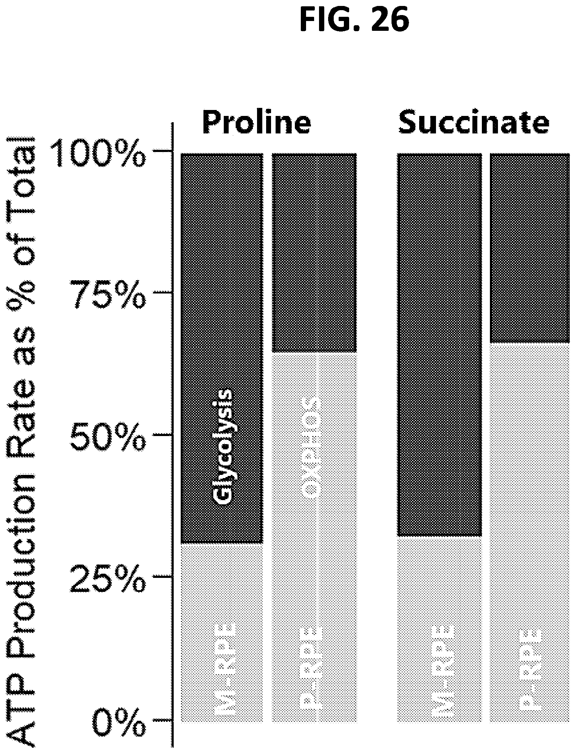

**FIG. 26****. Metabolic Processes of two types of RPE cells.** P-RPE have higher ability to undergo OXPHOS (lower bars) as compared to M-RPE that show higher rate of glycolysis (upper bars).

### DETAILED DESCRIPTION OF SEVERAL EMBODIMENTS

It is disclosed herein that several different types of RPE cells are present in the retina. Methods are disclosed herein for preparing macular, central, and peripheral RPE cells from pluripotent stem cells, such as iPSC. These methods include culturing immature RPE cells in a RPE maturation medium comprising a retinoic acid receptor (RAR) antagonist and/or a canonical Wnt inhibitor, to produce macular, central or peripheral human RPE cells. In some examples, the immature RPE cells are cultured on a scaffold provided herein, such as one including PLGA and PCL loops, such that the scaffold eventually includes the macular, central or peripheral human RPE cells produced from the culturing steps provided herein. The macular, central and peripheral RPE cells generated with the disclosed methods can be used as cell therapies to treat retinal degeneration, to discover drugs specific for cell types, to test drug toxicity for specific cell types, and to perform high throughput drug screens specific for different RPE regions. The macular, central and peripheral RPE cells also provide a model system to study regional RPE effects in different conditions.

### Terms

Unless otherwise noted, technical terms are used according to conventional usage. Definitions of many common terms in molecular biology may be found in Krebs et al. (eds.), Lewin's genes XII, published by Jones & Bartlett Learning, 2017. As used herein, the singular forms "a," "an," and "the," refer to both the singular as well as plural, unless the context clearly indicates otherwise. For example, the term "a canonical Wnt inhibitor" includes singular or plural canonical Wnt inhibitors and can be considered equivalent to the phrase "at least one canonical Wnt inhibitor." As used herein, the term "comprises" means "includes." Throughout this application, the term "about" is used to indicate within five percent, or that a value includes the inherent variation of error for a device, when the device is specified.

It is further to be understood that any and all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described herein. In case of conflict, the present specification, including explanations of terms, will control. In addition, the materials, methods, and examples are illustrative only and not intended to be limiting. To facilitate review of the various embodiments, the following explanations of terms are provided:
**Age Related Macular Degeneration (AMD):** AMD is caused by damage to the macula of the retina. Onset of AMD may be asymptomatic, but AMD gradually worsens over time and generally results in blurred or no vision in the center of the visual field in one or both eyes. It can be hard to recognize faces, drive, read, or perform other activities of daily life, and visual hallucinations may also occur. AMD typically occurs in older people, such as subjects about 50 years and older. Genetic factors and smoking can play a role. Diagnosis includes a complete eye exam, and severity can range from early, intermediate, and late types, in which the late type can further include "dry" and "wet" forms.

Dry AMD occurs over time, wherein macular tissue thins and breaks down. Symptoms can include visual distortions, reduced central vision in one or both eyes, a need for brighter light for reading or close work, increased difficulty adapting to low light levels, increased blurriness of printed words, decreased intensity or brightness of colors, and difficulty recognizing faces. Dry AMD is diagnosed by examining the back of the eye for drusen; testing for defects in the center of the vision (such as using an Amsler grid to identify whether straight lines in the grid to look faded, broken, or distorted, indicating the presence of dry AMD); fluorescein or indocyanine green angiography (examining for abnormal blood vessel or retinal changes); and/or optical coherence tomography (examining for retinal thinning, thickening, or swelling). Currently available treatments include rehabilitation for adapting to the loss of central vision (low vision rehabilitation) and implanting a telescopic lens.

Wet AMD follows dry AMD and includes abnormal blood vessel growth as well as fluid build-up in the back of the eye, which can produce a bump in the macula, causing vision loss of distortion. In addition to the symptoms of dry AMD, wet AMD symptoms can also include a well-defined blurry spot or blind spot in the field of vision, general haziness in overall vision, and abrupt onset and rapid worsening of symptoms. Currently available treatments include medications directed to stopping the growth of new blood vessels, such as bevacizumab (AVASTIN^{®}), ranibizumab (LUCENTIS^{®}), and aflibercept (EYLEA^{®}); photodynamic therapy; photocoagulation; and low vision rehabilitation.

In some examples, one or more of the cell populations generated using the methods provided herein (such as the macular, central, and/or peripheral RPE cells) are used to treat wet or dry AMD.

**Allele:** One of two or more forms of a gene. Diploid organisms such as humans contain two copies of each chromosome, and thus carry one allele on each.

The term **"homozygous"** is defined as containing two of the same alleles at a particular locus. The term **"heterozygous"** refers to as containing two different alleles at a particular locus. A **"haplotype"** refers to a combination of alleles at multiple loci along a single chromosome. A haplotype can be based upon a set of single-nucleotide polymorphisms (SNPs) on a single chromosome and/or the alleles in the major histocompatibility complex. As used herein, the term **"haplotype-matched"** is defined as the cell (*e.g.,* iPSC cell) and the subject being treated share one or more major histocompatibility locus haplotypes. The haplotype of the subject can be readily determined using assays well known in the art. The haplotype-matched iPSC cell can be autologous or allogeneic. The autologous cells which are grown in tissue culture (e.g., *ex vivo*) and differentiated to RPE cells inherently are haplotype-matched to the subject. **"Substantially the same HLA type"** indicates that the HLA type of donor matches with that of a patient to the extent that the transplanted cells, which have been obtained by inducing differentiation of iPSCs derived from the donor's somatic cells, can be engrafted when they are transplanted to the patient. **"Super donors" are** referred to herein as individuals that are homozygous for certain MHC class I and II genes. These homozygous individuals can serve as super donors and their cells, including tissues and other materials comprising their cells, can be transplanted in individuals that are either homozygous or heterozygous for that haplotype. The super donor can be homozygous for the HLA-A, HLA-B, HLA-C, HLA-DR, HLA-DP or HLA-DQ locus/loci alleles, respectively.

**Alter:** A change in an effective amount of a substance or parameter of interest, such as a polynucleotide, polypeptide or a property of a cell. An alteration in polypeptide or polynucleotide or activity can affect a physiological property of a cell, such as the differentiation, proliferation or survival of a cell. The amount of the substance can be changed by a difference in the amount of the substance produced, by a difference in the amount of the substance that has a desired function, or by a difference in the activation of the substance. The change can be an increase or a decrease. The alteration can be *in vivo* or *in vitro.* In several embodiments, altering is at least about a 50%, 60%, 70%, 80%, 90%, 95%, 96%, 97%, 98%, 99%, or 100% increase or decrease in the effective amount (level) of differentiation, proliferation and/or survival of a cells.

**Animal:** Living multi-cellular vertebrate organisms, a category that includes, for example, mammals and birds. The term mammal includes both human and non-human mammals. Similarly, the term "subject" includes both human and veterinary subjects, for example, non-human primates, dogs, cats, horses, rabbits, pigs, mice, rats, and cows.

**Antagonist or Inhibitor:** An agent that blocks or dampens a biochemical or biological response when bound to a receptor or a ligand of the receptor. Antagonists mediate their effects through receptor interactions by preventing agonist-induced responses. In one embodiment, a Frizzled (Fzd) antagonist binds to a Fzd receptor or to a Fzd ligand (such as Wnt) and reduces or inhibits the Wnt/beta-catenin signaling pathway, for example a reduction of at least 50%, at least 60%, at least 70%, at least 80%, at least 90%, at least 95%, at least 96%, at least 97%, at least 98%, at least 99%, or 100%.

**Bear track dystrophy:** A condition, which forms part of the disorder known as the grouped congenital hypertrophy of the retinal pigment epithelium (CHRPE), that is a peculiar congenital anomaly of the retinal pigment epithelium diagnosed by its characteristic ophthalmoscopic appearance. This disorder is usually without any functional consequence with patients typically having a normal vision, color vision, normal visual fields, dark adaptation, electroretinography, and electrooculography findings. The main differential diagnosis of CHRPE includes choroidal naevus, choroidal melanoma, chorioretinal scar, subretinal hematoma, pigmented epiretinal membrane, and reactive retinal pigment epithelial hyperplasia.

**Cell:** A structural and functional unit of an organism that can replicate independently, is enclosed by a membrane, and contains biomolecules and genetic material. Cells used herein may be naturally-occurring cells or artificially modified cells (*e.g.,* fusion cells, genetically modified cells, *etc.*)*.*

The term "cell population" refers to a group of cells, typically of a common type. The cell population can be derived from a common progenitor or may comprise more than one cell type. An "enriched" cell population refers to a cell population derived from a starting cell population (*e.g*., an unfractionated, heterogeneous cell population) that contains a greater percentage of a specific cell type, such as macular, central or peripheral RPE cells, than the percentage of that cell type in the starting population. The cell populations may be enriched for one or more cell types and depleted of one or more cell types.

**Cellular Adhesion Protein:** A protein involved in the binding of a cell to other cells or in the extracellular matrix in the process called cellular adhesion. Cell adhesion proteins include vitronectin, fibrin and laminin, but also include the integrins, which mediats cell-ECM interactions with collagen, fibrinogen, fibronectin, and vitronectin, cadherins, which are homophilic calcium-dependent glycoproteins, and the selectins, which are a family of heterophilic proteins (E-selectin, L-selectin, and P-selectin) that are dependent on fucosylated carbohydrate. In some examples, the cellular adhesion protein is recombinant.

**Chorioderemia:** A rare, X-linked recessive form of hereditary retinal degeneration that affects roughly 1 in 50,000 males. The disease causes a gradual loss of vision, starting with childhood night blindness, followed by peripheral vision loss and progressing to loss of central vision later in life. Progression continues throughout the individual's life, but both the rate of change and the degree of visual loss are variable among those affected, even within the same family. The first symptom many individuals with choroideremia notice is a significant loss of night vision, which begins in youth. Peripheral vision loss occurs gradually, starting as a ring of vision loss, and continuing on to "tunnel vision" in adulthood. Individuals with choroideremia tend to maintain good visual acuity into their 40s, but eventually lose all sight at some point in the 50-70 age range. In some examples, one or more of the cell populations generated using the methods provided herein (such as the central and/or peripheral, RPE cells) are used to treat choroideremia.

**Defined or Fully Defined:** When used in relation to a medium, an extracellular matrix, or a culture condition, refers to a medium, an extracellular matrix, or a culture condition in which the chemical composition and amounts of approximately all the components are known. For example, a defined medium does not contain undefined factors such as in fetal bovine serum, bovine serum albumin or human serum albumin. Generally, a defined medium comprises a basal media (*e.g.,* Dulbecco's Modified Eagle's Medium (DMEM), F12, or Roswell Park Memorial Institute Medium (RPMI) 1640, containing amino acids, vitamins, inorganic salts, buffers, antioxidants and energy sources) which is supplemented with recombinant albumin, chemically defined lipids, and recombinant insulin. An exemplary fully defined medium is ESSENTIAL 8^{™} medium.

**Diabetic retinopathy:** Diabetic retinopathy is a diabetes complication in which the blood vessels of the retinal tissue is damaged, symptoms of which can range from absent or mild, such as at onset, to blindness. In some embodiments, diabetic retinopathy is diagnosed with a comprehensive dilated eye exam, for example, to identify abnormal blood vessels; swelling, blood or fatty deposits in the retina; growth of new blood vessels and scar tissue; bleeding in the clear, jelly-like substance that fills the center of the eye (vitreous); retinal detachment; and abnormalities in the optic nerve. Additional diagnostic examinations include vision, glaucoma, and cataract tests as well as a fluorescein angiography or optical coherence tomography, for example, to determine whether fluid has leaked into the retinal tissue. Currently available treatments include photocoagulation, focal laser treatment, panretinal photocoagulation, vitrectomy, and intravitreal administration of medications, such as vascular endothelial growth factor (VEGF) inhibitors. In some examples, one or more of the cell populations generated using the methods provided herein (such as the macular, central, and/or peripheral RPE cells) are used to treat diabetic retinopathy.

**Differentiation:** The process by which an unspecialized cell becomes a more specialized type with changes in structural and/or functional properties. The mature cell typically has altered cellular structure and tissue-specific proteins. More specifically, in the context of the present methods indicates the process of a stem cell acquiring the cell characteristics of a RPE cell with features indicative that said RPE cell is a macular, central or peripheral RPE cell.

As used herein, "undifferentiated" refers to cells that display characteristic markers and morphological characteristics of undifferentiated cells that clearly distinguish them from terminally differentiated cells of embryo or adult origin.

**Embryo:** A cellular mass obtained by one or more divisions of a zygote or an activated oocyte with an artificially reprogrammed nucleus without regard to whether it has been implanted into a female. A "morula" is the preimplantation embryo 3-4 days after fertilization, when it is a solid mass, generally composed of 12-32 cells (blastomeres). A "blastocyst" refers to a preimplantation embryo in placental mammals (about 3 days after fertilization in the mouse, about 5 days after fertilization in humans) of about 30-150 cells. The blastocyst stage follows the morula stage, and can be distinguished by its unique morphology. The blastocyst is generally a sphere made up of a layer of cells (the trophectoderm), a fluid-filled cavity (the blastocoel or blastocyst cavity), and a cluster of cells on the interior (the inner cell mass, ICM). The ICM, consisting of undifferentiated cells, gives rise to what will become the fetus if the blastocyst is implanted in a uterus.

**Embryoid Bodies:** Three-dimensional aggregates of pluripotent stem cells. These cells can undergo differentiation into cells of the endoderm, mesoderm and ectoderm. In contrast to monolayer cultures, the spheroid structures that are formed when pluripotent stem cells aggregate enables the non-adherent culture of EBs in suspension, which is useful for bioprocessing approaches. The three-dimensional structure, including the establishment of complex cell adhesions and paracrine signaling within the EB microenvironment, enables differentiation and morphogenesis.

**Expand:** A process by which the number or amount of cells in a cell culture is increased due to cell division. Similarly, the terms "expansion" or "expanded" refer to this process. The terms "proliferate," "proliferation" or "proliferated" may be used interchangeably with the words "expand," "expansion", or "expanded." Typically, during an expansion phase, the cells do not differentiate to form mature cells, but divide to form more cells.

**Embryoid bodies (EBs):** Aggregates of pluripotent stem cells that can undergo differentiation into cells of the endoderm, mesoderm, and ectoderm germ layers. The spheroid structures form when pluripotent stem cells aggregate and enable the non-adherent culture of EBs in suspension.

**Embryonic stem cells:** Non-human embryonic cells derived from the inner cell mass of blastocysts or morulae, optionally that have been serially passaged as cell lines. The term includes cells isolated from one or more blastomeres of an embryo, without destroying the remainder of the embryo when the embryo is human. The term also includes cells produced by non-human somatic cell nuclear transfer. "Human embryonic stem cells" (hES cells) includes embryonic cells derived from the inner cell mass of human blastocysts or morulae, optionally that have been serially passaged as cell lines. The hES cells may be derived from parthenogenesis. Human ES cells can be produced or derived from blastomeres, or blastocyst-staged mammalian embryo produced by parthenogenesis. Human embryonic stem cells include, for reference only, but are not limited to, MAO1, MAO9, ACT-4, No. 3, H1, H7, H9, H14 and ACT30 embryonic stem cells. Human embryonic stem cells, regardless of their source or the particular method used to produce them, can be identified based on (i) the ability to differentiate into cells of all three germ layers, (ii) expression of at least Oct-4 and alkaline phosphatase, and (iii) ability to produce teratomas when transplanted into immunocompromised animals.

**Essentially Free:** In terms of a specified component, essentially free is used herein to mean that none of the specified component has been purposefully formulated into a composition and/or is present only as a contaminant or in trace amounts. The total amount of the specified component resulting from any unintended contamination of a composition is therefore well below 0.05%, such as below 0.01%. In some embodiments, no amount of the specified component can be detected with standard analytical methods.

**Feeder layers:** A coating layer of cells such as on the bottom of a culture dish. The feeder cells can release nutrients into the culture medium and provide a surface to which other cells, such as pluripotent stem cells, can attach.

**Feeder-free** or **feeder-independent:** A culture supplemented with cytokines and growth factors as a replacement for the feeder cell layer. Thus, "feeder-free" or feeder-independent culture systems and media may be used to culture and maintain pluripotent cells in an undifferentiated and proliferative state. In some cases, feeder-free cultures utilize an animal-based matrix (e.g. MATRIGEL^{™}) or are grown on a substrate such as fibronectin, collagen or vitronectin. These approaches allow human stem cells to remain in an essentially undifferentiated state without the need for mouse fibroblast "feeder layers."

**Fibroblast growth factor (FGF):** Any suitable fibroblast growth factor, derived from any animal, and functional fragments thereof, such as those that bind the receptor and induce biological effects related to activation of the receptor. Exemplary FGFs include, but are not limited to, FGF-1 (acidic fibroblast growth factor), FGF-2 (basic fibroblast growth factor, bFGF), FGF-3 (int-2), FGF-4 (hst/K-FGF), FGF-5, FGF-6, FGF-7, FGF-8, FGF-9 and FGF-98. "FGF" refers to a fibroblast growth factor protein such as FGF-1, FGF-2, FGF-4, FGF-6, FGF-8, FGF-9 or FGF-98, or a biologically active fragment or mutant thereof. The FGF can be from any animal species. In one embodiment, the FGF is mammalian FGF, including but not limited to, rodent, avian, canine, bovine, porcine, equine and human. The amino acid sequences and method for making many of the FGFs are known.

The amino acid sequence of human bFGF and methods for its recombinant expression are disclosed in U.S. Patent No. 5,439,818. The amino acid sequence of bovine bFGF and various methods for its recombinant expression are disclosed in U.S. Patent No. 5,155,214.

When the 146 residue forms are compared, their amino acid sequences are nearly identical, with only two residues that differ. Recombinant bFGF-2, and other FGFs, can be purified to pharmaceutical quality (98% or greater purity) using the techniques described in detail in U.S. Patent No. 4,956,455.

An FGF inducer includes an active fragment of FGF. In its simplest form, the active fragment is made by the removal of the N-terminal methionine, using well-known techniques for N-terminal methionine removal, such as a treatment with a methionine aminopeptidase. A second desirable truncation includes an FGF without its leader sequence. Those skilled in the art recognize the leader sequence as the series of hydrophobic residues at the N-terminus of a protein that facilitate its passage through a cell membrane but that are not necessary for activity and that are not found on the mature protein. Human and murine bFGF are commercially available.

**Growth factor:** A substance that promotes cell growth, survival, and/or differentiation. Growth factors include molecules that function as growth stimulators (mitogens), factors that stimulate cell migration, factors that function as chemotactic agents or inhibit cell migration or invasion of tumor cells, factors that modulate differentiated functions of cells, factors involved in apoptosis, or factors that promote survival of cells without influencing growth and differentiation. Examples of growth factors are a fibroblast growth factor (such as FGF-2), epidermal growth factor (EGF), cilliary neurotrophic factor (CNTF), and nerve growth factor (NGF), and activin-A.

**Inducer:** A molecule that regulates gene expression such as activating genes within a cell. An inducer can bind to repressors or activators. Inducers function by disabling repressors.

**Isolated:** An "isolated" cell has been substantially separated or purified from other cells in an organism or culture. Isolated cells can be, for example, at least 99%, at least 98% pure, at least 95% pure or at least 90% pure.

**KNOCKOUT^{™} serum replacement:** A serum-free formulation optimized to grow and maintain undifferentiated cells, such as stem cell, in culture.

**Late onset retinal degeneration:** A rare autosomal dominant disorder, characterized by the presence of thick, lipid-rich deposits between the RPE and Bruch's membrane. The disease was first seen in the mid- 1990s and has been linked to a mutation in the *C1QTVF5* gene.

The *C1QTNF5* gene, located on 11q23 encodes a 281 amino acid protein and is highly expressed in the RPE, lens, and ciliary epithelium.

Individuals with L-ORD often show no ophthalmic disturbances until midlife, around 50-60 years old. Clinically, early symptoms of the disease process include difficulty with light and dark adaptation with inability to see in dim light or at night. Progression of the disease then leads to loss of central and peripheral vision, choroidal neovascularization and retina-wide pigmentary retinopathy. Ultimately, the decline in visual acuity leads to complete vision loss.

Ophthalmic examinations may initially be normal even after decline in night vision begins. However, as the disease progresses, the appearance of fine yellow-white drusen-like dots in the mid-periphery is the first ophthalmic indication of L-ORD. These drusen-like "dots" or deposits then form atrophic areas that spread throughout the retina. Fundus auto fluorescence depicts extensive, well-defined scalloped areas of RPE and chorioretinal atrophy predominantly in the mid-periphery and in the posterior pole of the retina. The macula usually becomes atrophic but can sometimes form a disc form scar. The optic disc also changes into a pale color.

In some examples, one or more of the cell populations generated using the methods provided herein (such as the central and/or peripheral RPE cells) are used to treat L-ORD.

**Leber's Congenital Amaurrosis (LCA):** A rare inherited eye disease that appears at birth or in the early stages of life (infancy or early childhood) and primarily affects the retina. The presentation can vary because is it associated with multiple genes. However, it is characterized by characterized by nystagmus, photophobia, sluggish or absent pupillary response, and severe vision loss or blindness. The common modes of inheritance are autosomal recessive and autosomal dominant.

The pupils, which usually expand and contract in response to the amount of light entering the eye, do not react normally to light. Instead, they expand and contract more slowly than normal, or they may not respond to light at all. Additionally, the clear front covering of the eye (the cornea) may be cone-shaped and abnormally thin, a condition known as keratoconus. A specific behavior referred to as Franceschetti's oculo-digital sign is characteristic of LCA. This sign consists of poking, pressing, and rubbing the eyes with a knuckle or finger.

In some examples, one or more of the cell populations generated using the methods provided herein (such as the macular, central, and/or peripheral RPE cells) are used to treat LCA.

**Mammal:** This term includes both human and non-human mammals. Examples of mammals include but are not limited to: humans and veterinary and laboratory animals, such as pigs, cows, goats, cats, dogs, rabbits and mice.

**Membrane potential:** The electrical potential of the interior of the cell with respect to the environment, such as an external bath solution. One of skill in the art can readily assess the membrane potential of a cell, such as by using conventional whole cell techniques. The membrane potential can be assessed using many approaches, such as using conventional whole cell access, or using, for example, perforated-patch whole-cell and cell-attached configurations.

**Medium:** A synthetic set of culture conditions with the nutrients necessary to support the growth (cell proliferation/expansion) and/or differentiation of a specific population of cells. In one embodiment, the cells are stem cells, such as iPSCs. In another embodiment, the cells are RPE cells. Media generally include a carbon source, a nitrogen source and a buffer to maintain pH. In one embodiment, growth medium contains a minimal essential media, such as DMEM, supplemented with various nutrients to enhance stem cell growth. Additionally, the minimal essential media may be supplemented with additives such as horse, calf or fetal bovine serum.

**Noggin:** A protein which is encoded by the *NOG* gene. Noggin inhibits TGF-β signal transduction by binding to TGF-β family ligands and preventing them from binding to their corresponding receptors. Noggin plays a key role in neural induction by inhibiting BMP4, along with other TGF-β signaling inhibitors such as chordin and follistatin. Exemplary sequences for Noggin are GENBANK^{®} Accession Nos. NP_005441.1 and NM_005450.4, January 13, 2013.

**Oct-4:** A protein also known as POU5-F1 or MGC22487 or OCT3 or OCT4 or OTF3 or OTF4, which is the gene product of the Oct-4 gene. The term includes Oct-4 from any species or source and includes analogs and fragments or portions of Oct-4 that retain the ability to be used for the production of iPSCs. The Oct-4 protein may have any published sequence for Oct-4 which can be obtained from public sources such as GENBANK^{®}. An example of such a sequence includes, but is not limited to, GENBANK^{®} Accession No. NM_002701.

**Pharmaceutically acceptable carriers:** Conventional pharmaceutically acceptable carriers are useful for practicing the methods and forming the compositions disclosed herein. Remington's Pharmaceutical Sciences, by E. W. Martin, Mack Publishing Co., Easton, PA, 15th Edition, 1975, describes examples of compositions and formulations suitable for pharmaceutical delivery of the compounds herein disclosed.

In general, the nature of the carrier will depend on the particular mode of administration being employed. For example, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g.,* powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Pre-confluent:** A cell culture in which the entire tissue culture surface is not covered with cells, so that cell division can occur. The proportion of the culture surface which is covered by cells can be about 60-80%. Usually, pre-confluent refers to a culture in which about 70% of the culture surface is covered by cells.

**Purified:** A purified composition does not require absolute purity; rather, it is intended as a relative term. Thus, a purified population of cells is greater than about 90%, 91%, 92%, 93%, 94%, 95%, 96%, 97%, 98%, 99% or 100% pure, for example essentially free other cell types.

**Retinoic acid receptor (RAR) Antagonist:** The RAR receptor is a nuclear receptor that is activated by all-trans retinoic acid and 9-cis retinoic acid. The best-known mechanism of action of these receptors involves their binding to RA response elements (RARE) in the promoters of retinoid-responsive genes. Retinoid receptors also affect transcription through RARE-independent mechanisms, such as repression of transcription factor activator protein. There are three subtypes of RAR receptors; pan-RAR antagonists inhibit all of the subtypes.

**Retina:** A light-sensitive layer of tissue which lines the inner surface of the eye.

**Retinal detachment:** Retinal detachment is a condition in which the retina is pulled away from its normal position with symptoms that include a sudden increase in the number of specks floating in your vision (floaters), flashes of light in one eye or both eyes, a "curtain" or shadow over your field of vision, and, without immediate treatment, permanent vision loss.

In some examples, one or more of the cell populations generated using the methods provided herein (such as the peripheral RPE cells) are used to treat retinal detachment.

**Retinal diseases and disorders:** Retinal diseases include disease in which the function or structure of the retina is damaged or decreased. Retinal degenerative diseases are included, in which the retinal structure or function changes for the worse over time. Retinal vascular diseases are included, such as retinal diseases in which the structure or function of the blood vessels in the eye are affected.

In some examples, one or more of the cell populations generated using the methods provided herein (such as the macular, central, and/or peripheral RPE cells) are used to treat a retinal disease.

**Retinal lineage cells:** Cells that can give rise or differentiate to RPE cells.

**Retinal Induction Medium (RIM):** A growth media that comprises a WNT pathway inhibitor and a BMP pathway inhibitor and can result in the differentiation of pluripotent stem cells (PSCs) to retinal lineage cells. The RIM also comprises a TGFβ pathway inhibitor.

**Retinal Differentiation Medium (RDM):** A medium that includes a WNT pathway inhibitor, a BMP pathway inhibitor and a mitogen-activated protein kinase (MEK) (or FGF) inhibitor and differentiates retinal cells. The RDM also includes a TGFβ pathway inhibitor.

**Retinal Medium (RM):** A growth medium for the culture of retinal cells that includes activin A and nicotinamide.

**RPE-Maturation Medium (RPE-MM):** A medium for the maturation of RPE cells that includes taurine and hydrocortisone. The RPE-MM also includes triiodothyronine. The RPE-MM may also include PD0325901 or PGE2.

**Retinal pigment epithelial (RPE) cell:** RPE cells can be recognized based on pigmentation, epithelial morphology, and apical-basal polarized cells. RPE cells express, both at the mRNA and protein level, one or more of the following: Pax6, MITF, RPE65, CRALBP, PEDF, Bestrophin and/or Otx2. In certain other embodiments, the RPE cells express, both at the mRNA and protein level, one or more of Pax-6, MitF, and tyrosinase. RPE cells do not express (at any detectable level) the embryonic stem cell markers Oct-4, nanog, or Rex-1. Specifically, expression of these genes is approximately 100-1000 fold lower in RPE cells than in ES cells or iPSCs, when assessed by quantitative RT-PCR. Differentiated RPE cells also can be visually recognized by their morphology and the initial appearance of pigment. In addition, differentiated RPE cells have trans-epithelial resistance/TER, and trans epithelial potential/TEP across the monolayer (TER >100 Ohms*cm²; TEP >2 mV), transport fluid and CO₂ from apical to basal side, and regulate a polarized secretion of cytokines. RPE cells include peripheral, central and macular RPE cells.

In humans, a "macular" RPE cell has an area of about 150 µm² ± 33 µm² and petal-like apical process, which have a length of about 0.5 to about 4 µm, a width of about 0.1 to about 1µm, and a width of undulation of about 0.2 to about 2 µm. A central RPE cell has an area of about 199 µm² + 40 µm² with mixed petal-like and finger-like apical processes. A "peripheral" RPE cell has an area of about 239 µm²-± 38 µm²-and finger-like apical process, which have a length of about 0.1 to about 1 µm and a width of about 0.1 to about 1µm.

"Mature" RPE cells are referred to herein as RPE cells which have downregulated expression of immature RPE markers such as Pax6 and upregulated expression of mature RPE markers such as RPE65.

RPE cell "maturation" refers herein to the process by which RPE developmental pathways are modulated to generate mature RPE cells. For example, modulation of cilia function can result in RPE maturation. "Retinal lineage cells" herein refer to cells that can give rise or differentiate to RPE cells.

**Retinitis Pigmentosa (RP):** RP is an inherited, degenerative eye disease that causes severe vision impairment due to the progressive degeneration of the rod photoreceptor cells in the retina. This form of retinal dystrophy manifests initial symptoms independent of age. The initial retinal degenerative symptoms of RP are characterized by decreased night vision (nyctalopia) and the loss of the mid-peripheral visual field. The rod photoreceptor cells, which are responsible for low-light vision and are orientated in the retinal periphery, are the retinal processes affected first during non-syndromic forms of this disease. Visual decline progresses relatively quickly to the far peripheral field, eventually extending into the central visual field as tunnel vision increases. Visual acuity and color vision can become compromised due to accompanying abnormalities in the cone photoreceptor cells, which are responsible for color vision, visual acuity, and sight in the central visual field. The progression of disease symptoms occurs in a symmetrical manner, with both the left and right eyes experiencing symptoms at a similar rate.

In some examples, one or more of the cell populations generated using the methods provided herein (such as the macular, central, and/or peripheral RPE cells) are used to treat RP.

**Retinoic acid receptor (RAR) Antagonist:** The RAR receptor is a nuclear receptor that is activated by all-trans retinoic acid and 9-cis retinoic acid. One mechanism of action of these receptors involves their binding to RA response elements (RARE) in the promoters of retinoid-responsive genes. Retinoid receptors also affect transcription through RARE-independent mechanisms, such as repression of transcription factor activator protein. There are three subtypes of RAR receptors; pan-RAR antagonists inhibit all of the subtypes.

In some examples, a RAR antagonist reduces activity by at least 50%, at least 60%, at least 70%, at least 80%, at least 90%, at least 95%, at least 96%, at least 97%, at least 98%, at least 99%, or 100%.

Examples of RAR antagonists include AGN 193109 (4-[2-[5,6-Dihydro-5,5-dimethyl-8-(4-methylphenyl)-2-naphthalenyl]ethynyl]-benzoic acid), CE 2665, ER 5081, LE 135, LY 2955303, MM 11253, and liarozole dihydrochloride.

**Scaffold:** A solid support to which cells, such as pluripotent stem cells, RPE progenitor cells, committed RPE cells, immature RPE cells, mature RPE and PRP cells can be attached. In some examples, the scaffold is biodegradable. In some examples, the scaffold includes two layers, a PLGA layer and a layer of PCL loops attached to the PLGA layer. In some examples, includes pluripotent stem cells, such as iPSC, and is cultured in the presence of particular reagents provided herein to generate mature macular, central and/or peripheral RPE cells (such as human RPE cells).

**Stem Cell:** A cell that under suitable conditions is capable of differentiating into a diverse range of specialized cell types, while under other suitable conditions is capable of self-renewing and remaining in an essentially undifferentiated pluripotent state. The term "stem cell" also encompasses a pluripotent stem cell, multipotent stem cell, precursor cell and progenitor cell. Exemplary human stem cells can be obtained from hematopoietic or mesenchymal stem cells obtained from bone marrow tissue, embryonic stem cells obtained from embryonic tissue, or embryonic germ cells obtained from genital tissue of a fetus. Exemplary pluripotent stem cells can also be produced from somatic cells by reprogramming them to a pluripotent state by the expression of certain transcription factors associated with pluripotency; these cells are called "induced pluripotent stem cells" or "iPSCs".

An "embryonic stem (ES) cell" is an undifferentiated pluripotent cell which can give rise to any differentiated cell type in an embryo or an adult, including germ cells (*e.g.,* sperm and eggs).

"Induced pluripotent stem cells (iPSCs)" are cells generated by reprogramming a somatic cell by expressing or inducing expression of a combination of factors (herein referred to as reprogramming factors). iPSCs can be generated using fetal, postnatal, newborn, juvenile, or adult somatic cells. In certain embodiments, factors that can be used to reprogram somatic cells to pluripotent stem cells include, for example, Oct4 (sometimes referred to as Oct 3/4), Sox2, c-Myc, and Klf4, Nanog, and Lin28. In some embodiments, somatic cells are reprogrammed by expressing at least two reprogramming factors, at least three reprogramming factors, or four reprogramming factors to reprogram a somatic cell to a pluripotent stem cell.

The term **"pluripotent"** refers to the property of a cell to differentiate into all other cell types in an organism, with the exception of extraembryonic, or placental, cells. Pluripotent stem cells are capable of differentiating to cell types of all three germ layers (e.g., ectodermal, mesodermal, and endodermal cell types) even after prolonged culture. A pluripotent stem cell is an embryonic stem cell derived from the inner cell mass of a blastocyst. In other embodiments, the pluripotent stem cell is an induced pluripotent stem cell derived by reprogramming somatic cells.

**Therapeutically effective amount:** The amount of a compound or a cell, such as an RPE cell, that, when administered to a subject for treatment of a disease or condition, is sufficient to effect such treatment, or to reduce a symptom of the disease or condition. Malfunction of the retinal pigment epithelium is associated with a number of vision-altering conditions, such as retinal pigment epithelium detachment, dysplasia, atrophy, retinopathy, retinitis pigmentosa, macular dystrophy, and degeneration.

**Tissue Replacement Implant:** A biological compatible structure including a both a matrix and cells that is created *in vitro,* that can be used to replace a tissue in vivo. A tissue replacement implant can include macular, central, and/or peripheral RPE cells, and combinations thereof. Tissue replacement implants can also include rods, cones and/or vascular cells, or combinations thereof. An exemplary tissue replacement implant is a scaffold disclosed herein containing RPE cells (such as mature macular, central and/or peripheral RPE cells) and PRP cells.

**Treatment:** Therapeutic measures that cure, slow down, lessen symptoms of, and/or halt progression of a diagnosed pathologic condition or disorder. In certain embodiments, treating a subject with a retinal disorder results in a decline in the deterioration of the retinal; an increase in the number of retinal pigment epithelial cells, an improvement in vision, or some combination of effects. The scaffolds disclosed herein containing RPE cells (such as mature macular, central and/or peripheral RPE cells) and PRP cells can be implanted into the retina of a subject to treat retinal degenerative disease, retinal dysfunction, retinal degradation, retinal damage, or loss of retinal pigment epithelium.

**Undifferentiated:** Cells that display characteristic markers and morphological characteristics of undifferentiated cells, distinguishing them from differentiated cells of embryo or adult origin. Thus, in some embodiments, undifferentiated cells do not express cell lineage specific markers, including, but no limited to, RPE markers.

**Wnt:** A family of highly conserved secreted signaling molecules that regulate cell-to-cell interactions and are related to the *Drosophila* segment polarity gene, wingless. In humans, the Wnt family of genes encodes 38 to 43 kDa cysteine rich glycoproteins. The Wnt proteins have a hydrophobic signal sequence, a conserved asparagine-linked oligosaccharide consensus sequence (see *e.g.,* Shimizu et al Cell Growth Differ 8:1349-1358 (1997)) and 22 conserved cysteine residues. Because of their ability to promote stabilization of cytoplasmic beta-catenin, Wnt proteins can act as transcriptional activators and inhibit apoptosis. Overexpression of particular Wnt proteins is associated with certain cancers.

The Wnt family contains at least 19 mammalian members. Exemplary Wnt proteins include Wnt-1, Wnt-2, Wnt2b, Wnt-3, Wnt-3a, Wnt-4, Wnt-5a, Wnt5b, Wnt-6, Wnt-7a, Wnt-7b, Wnt-8a, Wnt-8b, Wnt9a, Wnt9b, Wnt10a, Wnt-10b, Wnt-11, and Wnt 16. These secreted ligands activate at least three different signaling pathways. In the canonical (or Wnt/beta-catenin) Wnt signaling pathway, Wnt activates a receptor complex consisting of a Frizzled (Fzd) receptor family member and low-density lipoprotein (LDL) receptor-related protein 5 or 6 (LRP5/6). To form the receptor complex that binds the Fzd ligands, Fzd receptors interact with LRP5/6, single pass transmembrane proteins with four extracellular EGF-like domains separated by six YWTD amino acid repeats (Johnson et al., 2004, J. Bone Mineral Res. 19:1749). The canonical Wnt signaling pathway activated upon receptor binding is mediated by the cytoplasmic protein Dishevelled (Dvl) interacting directly with the Fzd receptor and results in the cytoplasmic stabilization and accumulation of beta-catenin. In the absence of a Wnt signal, beta-catenin is localized to a cytoplasmic destruction complex that includes the tumor suppressor proteins adenomatous polyposis coli (APC) and Axin. These proteins function as critical scaffolds to allow glycogen synthase kinase (GSK)-3beta to bind and phosphorylate beta-catenin, marking it for degradation via the ubiquitin/proteasome pathway. Activation of Dvl results in the dissociation of the destruction complex. Accumulated cytoplasmic beta-catenin is then transported into the nucleus where it interacts with the DNA-binding proteins of the TCF/LEF family to activate transcription.

The non-canonical WNT pathway is regulated by three of these WNT ligands - WNT4, WNT5a, and WNT11. These ligands bind to the WNT receptor Frizzled in the absence of the co-receptors (LRP5/6). This leads to the activation of the RHO GTPase and ROCK kinase without activating cytoplasmic beta-catenin. ROCK regulates cytoskeleton to regulate apical-basal polarity of the cell. Because of competition for the same receptor, non-canonical WNT ligands also lead to inhibition of canonical WNT signaling.

**Xeno-Free (XF):** When used in relation to a medium, an extracellular matrix, or a culture condition, refers to a medium, an extracellular matrix, or a culture condition which is essentially free from heterogeneous animal-derived components. For culturing human cells, any proteins of a non-human animal, such as mouse, would be xeno components. In certain aspects, the Xeno-free matrix may be essentially free of any non-human animal-derived components, therefore excluding mouse feeder cells or MATRIGEL^{™}. MATRIGEL^{™} is a solubilized basement membrane preparation extracted from the Engelbreth-Holm-Swarm (EHS) mouse sarcoma, a tumor rich in extracellular matrix proteins to include laminin (a major component), collagen IV, heparan sulfate proteoglycans, and entactin/nidogen.

**Vasculature:** The vasculature can include the circulatory system (such as an arrangement of blood vessels) or a portion thereof, such as a supply of vessels to a specific region.

### Scaffolds

The present disclosure provides scaffolds which can further include pluripotent stem cells (e.g., iPSCs), RPE progenitor cells, committed RPE cells, immature RPE cells, or RPE cells. For example, scaffolds which include pluripotent stem cells can be cultured in the presence of reagents as described herein us the disclosed methods to obtain a scaffold including mature mammalian (e.g., human) RPE cells (such as mature macular, central and/or peripheral RPE cells). Such mature RPE cell-containing scaffolds can be implanted into the retina of a subject, such as a human, to treat a retinal disorder. In some examples, the scaffold is biodegradable, for example within at least 20 days following its implantation into a retina, such as within at least 30 days, at least 6 weeks, at least 2 months, at least 3 months, at least 4 months, at least 5 months, at least 6 months, at least 9 months, or at least 12 months, such as within about 20 days to 6 months, about 30 days to 3 months, about 30 to 60 days, or about 3 months to 1 year, following its implantation into a retina. In some examples, the first PLGA layer of the scaffold degrades more quickly than the second PCL-loop containing layer. In some examples the first layer degrades within about 3 months of implantation into a retina, for example within about 30 days, about 60 days, about 90 days, about 100 days, or about 120 days (such as about 30-90 days, about 60 to 100 days, or about 70-120 days) following implantation of the scaffold into a retina. In some examples the second layer degrades within about 1 year of implantation into a retina, for example within about 6 months, about 8 months, about 9 months, about 10 months, about 11 months, about 12 months, about 13 months, or about 14 months (such as about 6-14 months, about 9 to 12 months, or about 10-14 months) following implantation of the scaffold into a retina.

An exemplary scaffold is shown in FIG. 19A. Although shown in two dimensions (e.g., a cross section), one skilled in the art will appreciate that a scaffold is three dimensional. Scaffold 100 includes two layers 102, 103, having height/thickness 111. The first layer 102 (e.g., the bottom portion, see FIG. 19A) includes poly(lactic-co-glycolic acid) (PLGA) (see Lu et al., J. Biomater Sci Polym Ed. 9(11): 1187-205, 1998), and the second layer 103 (e.g., the top surface, see FIG. 19A) includes polycaprolactone (PCL) loops 104. Thus, the material of the scaffold is generally physiologically acceptable and suitable for use *in vivo* applications. In some examples, scaffold 100 can be about at least about 1 micron in height (e.g., height of scaffold 111 can be at least about 1 micron), at least about 1 micron in length, and at least about 1 micron in width (such as at least about 20 microns in height, at least about 20 microns in length, and at least about 20 microns in width, such as at least about 100 microns in height, at least about 100 microns in length, and at least about 100 microns in width, such as at least about 200 microns in height, at least about 200 microns in length, and at least about 200 microns in width, such as at least about 1 mm in height, at least about 1 mm in length, and at least about 1 mm in width, such as at least about 10 mm in height, at least about 10 mm in length, and at least about 10 mm in width, or such as at least about 100 mm in height, at least about 100 mm in length, and at least about 100 mm in width). The scaffold 100 can be any shape, such as rectangular, oval, circular, square, or irregular.

PLGA is a copolymer of poly-lactic acid (PLA) and poly-glycolic acid (PGA). Poly-lactic acid contains an asymmetric *α-*carbon which is typically described as the D or L form in classical stereochemical terms and sometimes as R and S form, respectively. The enantiomeric forms of the polymer PLA are poly D-lactic acid (PDLA) and poly L-lactic acid (PLLA). PLGA is poly D, L-lactic-co-glycolic acid where D- and L- lactic acid forms are generally in equal ratio. PLGA biodegrades by hydrolysis of its ester linkages. In some embodiments, the PLGA scaffold is cultured for a sufficient time such that the bulk of lactic acid release from the scaffold occurs *in vitro.* In some embodiments, greater than 50%, 60%, 70%, 80%, 90% or 95% of the lactic acid release occurs *in vitro.* The lactic acid release occurs over time.

The first layer 102 can be at least 5 microns in height, at least 10 microns in height, at least 20 microns in height, at least 30 microns in height, or at least 40 microns in height, such as about 5 to about 40 microns in height, such as about 5 to about 25 microns in height, about 10 to about 20 microns in height, or about 15 to about 25 microns in height, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 microns in height. In this context, "about" indicates within 5%.

The PLGA layer of the scaffold can include nanofibers that intersect each other, such that they intersect and form junctions. The scaffold can be treated to fuse fibers of the scaffold at the junctions of fiber intersections within the PLGA scaffold to increase mechanical strength. The average pore size is the space between the fibers in the PLGA scaffold. Thus, in some examples, first layer 102 is composed of PLGA having an average pore size of at least about 0.2 microns in diameter, such as a diameter of at least 0.3, at least 0.4, at least 0.5, at least 0.6, at least 0.7, at least 0.8, at least 0.9, at least 1, at least 1.1, at least 1.2, at least 1.3, at least 1.4, at least 1.5, at least 1.6, at least 1.7, at least 1.8, at least 1.9, or at least 2 microns, such as about 0.2 to about 2 microns, about 0.5 to about 2 microns, about 0.5 to about 1.5 microns, about 1 to about 2 microns, such as 0.2, 0.3, 0.4, 0.5, 0.6, 0.7, 0.8, 0.9, 1, 1.1, 1.2, 1.3, 1.4, 1.5, 1.6, 1.7, 1.8, 1.9, or 2 microns. In some embodiments, the PLGA layer of the scaffold has a pore size of less than about 2 microns, such as less than about 1.5 microns, less than about 1.25 microns, less than about 1 micron, less than about 0.5 micron, or less than about 0.3 micron. In one example the PLGA of first layer 102 has an average pore size of about 1 micron or less in diameter. In this context, "about" indicates within 5%.

The PLGA of first layer 102 has a DL-lactide/glycolide ratio of about 0.25:3 to about 3:0.25, such as about 0.25:3; about 0.5:3, about 1:3, about 1:2.5, about 1:2, about 1:1.5, about 1:1, about 1.5:1, about 1.5:2, about 1.5:3, about 2:1, about 2.5:1, or about 3:1. In one example the PLGA of first layer 102 has a DL-lactide/glycolide ratio of about 1:1. In this context, "about" indicates within 5%.

The PLGA of first layer 102 has a fiber diameter of at least 150 nm, such as at least 200 nm, at least 250 nm, at least 300 nm, at least 350 nm, at least 400 nm, at least 450 nm, at least 500 nm, at least 550 nm, or at least 600 nm diameter, such as about 150 to about 650 nm diameter, about 150 to about 500, nm about 200 to about 600 nm, about 400 to about 500 nm diameter, such as about 150 nm, about 200 nm, about 250 nm, about 300 nm, about 350 nm, about 400 nm, about 450 nm, about 500 nm, about 550 nm, about 600 nm diameter, such as about 450 nm diameter. In one example the PLGA of first layer 102 has a fiber diameter of about 450 nm. In this context, "about" indicates within 5%.

Any of the features of the PLGA layer of the scaffold of thickness, pore size and fiber diameter described herein can be combined. Varying a DL-lactide/glycolide ratio can alter the pore size and fiber diameter. Thus, one can produce PLGA scaffolds of the disclosed thickness (e.g., height), pore sizes and fiber diameters. Any features can be combined to arrive at specific combinations produced by varying DL-lactide/glycolide ratio. These are all understood to be disclosed herein. In a specific non-limiting example, the PLGA layer of the scaffold has a DL-lactide/glycolide ratio of 1:1, an average pore size of less than 1 microns, and a fiber diameter of 150 to 600 nm.

In some embodiments, PLGA layer of the scaffold is treated with heat to fuse fibers of the PLGA layer of scaffold at the junctions (fiber intersections) within the PLGA layer of scaffold to increase mechanical strength of the PLGA layer of scaffold. This heat treatment also reduces pore size by fusing the fibers at the junctions and thus allows the cells to form a monolayer on the upper layer of the scaffold. In some non-limiting examples, PLGA is placed on an appropriate surface, for example, a metal surface such aluminum foil, such as in the form of an envelope, that is placed inside an oven set to the desired temperature for treatment. Suitable temperatures include about 35 °C to about 55 °C, such as about 40 °C to about 50 °C, such as about 43 °C, 44 °C, 45 °C, 46 °C, or 47 °C. The PLGA can be heated for about 5 to about 20 minutes, such as about 10 to about 15 minutes, such as about 10, 11, 12, 13, 14 or 15 minutes. In one embodiment, the PLGA is treated at about 45 °C to for about 10 minutes. The temperature can then be increased relative to the first temperature, such as to about 50 ° C to about 70 °C, such as about 55 °C to about 60 °C, such as about 55 °C, 56 °C, 57 °C, 58 °C, 59 °C or 60 °C. The higher temperature treatment can be for about 45 minutes to about 75 minutes, such as about 50 minutes to about 70 minutes, or about 55 minutes to about 65 minutes. The higher temperature treatment can be applied for about 55, 56, 57, 58, 59, 60, 61, 62, 63, 64 or 65 minutes. In some embodiments, the scaffold is treated at about 60 °C for about 60 minutes. In one non-limiting example, the scaffold can be treated at about 45 °C to for about 10 minutes and then at about 60 °C for about 60 minutes. Following heat treatment, the PLGA layer of the scaffold can be stored.

The second layer 103 is attached to one surface of the first layer 102, such as a surface with a larger surface area than another surface of the first layer. The first layer 102 includes an upper surface 112 and lower surface 114. In some examples, the second layer 103 is on the upper surface 112 of first layer 102. In some examples, the second layer 103 is at least about 10 microns in height, such as at least about 20 microns, at least about 50 microns, at least about 100 microns, at least about 150 microns, at least about 200 microns, at least about 500 microns, or at least about 1000 microns, such as about 10 to 200 microns, about 50 to 200 microns, about 100 to 200 microns, about 10 to 1000 microns, or 100 to 500 microns. In this context, "about" indicates within 5%.

The second layer 103 is composed of a plurality of PCL loops 104. In some examples, the fiber of the loop 104 has a diameter of at least 5 microns, such as a diameter of at least 10, at least 20, at least 50, at least 150, at least 200, at least 250, or at least 300 microns, such as about 5 to about 300 microns, about 5 to about 10 microns, about 100 to about 300 microns, about 10 to about 50 microns, such as 5, 10, 20, 25, 30, 40, 50, 60, 70, 80, 90, 100, 125, 150, 175, 200, 225, 250, 275, or 300 microns. In one example the fiber of the PCL loop is about 5 microns in diameter. In this context, "about" indicates within 5%.

The density and number of PCL loops 104 of the second layer 103 can be determined to provide sufficient gaps for multiple RPE cells 106 (*e.g.,* 10-1000 cells) to form a monolayer. In one example, the density of PCL loops 104 of the second layer 103 is at least about 10 PCL loops/mm, such as at least about 20 PCL loops/mm, at least about 50 PCL loops/mm, at least about 75 PCL loops/mm, or at least about 100 PCL loops/mm, such as 10-100 PCL loops/mm, 10-50 PCL loops/mm, 10-200 PCL loops/mm, or 50-100 PCL loops/mm. In one example, at least about 10 PCL loops 104 are present in the second layer 103, such as at least about 20, at least about 30, at least about 50, at least about 75, or at least about 100 PCL loops. In this context, "about" indicates within 5%.

The PCL loops 104 of the second layer 103 deposited on the PLGA first layer 102 can be completely closed (that is both ends of the PCL fiber are in contact with the first layer 102, e.g., as shown in FIG. 19B), can be open (e.g., one end of the PCL fiber is not in contact with the first layer 102, and in some examples forms a hook-like structure), can be closed on itself (e.g., like the letter "p" or "b"), or the second layer 103 can include a mixture of open and closed PCL loops. In some examples, a closed PCL loop 104 has a diameter of at least about 10 microns, such as at least about 20 microns, at least about 50 microns, at least about 150 microns, or at least about 200 microns, such as about 10 to about 200 microns, about 10 to about 100 microns, about 50 to about 20 microns, about 10 to about 50 microns, such as 10, 20, 25, 30, 40, 50, 60, 70, 80, 90, 100, 125, 150, 175, or 200 microns. In this context, "about" indicates within 5%.

Any of the features of the PCL loop layer of the scaffold of thickness, density, and fiber diameter described herein can be combined. In a specific non-limiting example, the PCL layer of the scaffold have a fiber diameter of 5 to 300 nm, and are attached to the first layer using electrospinning.

In some examples, a scaffold (e.g., 100 of FIG. 19A or 19B) is coated with a cellular adhesion protein, such as a recombinant cellular adhesion protein. Exemplary cellular adhesion proteins that can be used include one or more of vitronectin, laminin, and fibronectin. In some embodiments, the PLGA scaffold is coated with vitronectin. In some examples, a scaffold (e.g., 100 of FIG. 19A or 19B)is coated with at least about 20 ng/ml cellular adhesion protein, such as at least about 30 ng/ml, at least about 40 ng/ml, at least about 50 ng/ml, at least about 60 ng/ml, at least about 70 ng/ml, at least about 80 ng/ml, at least about 90 ng/ml, or at least about 95 ng/ml cellular adhesion protein, such as about 20-95 ng/ml, about 20-80 ng/ml, about 20-70 ng/ml, about 30-60 ng/ml, about 30-50 ng/ml, about 40-50 ng/ml cellular adhesion protein, such as 20 ng/ml, 30 ng/ml, 40 ng/ml, 45.5 ng/ml, 50 ng/ml, 60 ng/ml, 70 ng/ml, 80 ng/ml, or 95 ng/ml cellular adhesion protein. In a specific example, scaffold 100 is coated with 45.5 ng/ml cellular adhesion protein, such as 45.5 ng/ml vitronectin, 45.5 ng/ml laminin, or 45.5 ng/ml fibronectin.

In some examples, a scaffold (e.g., 100 of FIG. 19A or 19B)is coated with an extracellular matrix or gelatin. An extracellular matrix is a complex mixture of structural and functional biomolecules and/or biomacromolecules including, but not limited to, structural proteins, specialized proteins, proteoglycans, glycosaminoglycans, and growth factors that surround and support cells within mammalian tissues and, unless otherwise indicated, is acellular. Extracellular matrices that can be used are disclosed, for example and without limitation, in U.S. Patent Nos. 4,902,508; 4,956,178; 5,281,422; 5,352,463; 5,372,821; 5,554,389; 5,573,784; 5,645,860; 5,771,969; 5,753,267; 5,762,966; 5,866,414; 6,099,567; 6,485,723; 6,576,265; 6,579,538; 6,696,270; 6,783,776; 6,793,939; 6,849,273; 6,852,339; 6,861,074; 6,887,495; 6,890,562; 6,890,563; 6,890,564; and 6,893,666.

However, an ECM can be produced from any tissue, or from any in vitro source wherein the ECM is produced by cultured cells and includes one or more polymeric components (constituents) of native ECM. ECM preparations can be considered to be "decellularized" or "acellular", meaning the cells have been removed from the source tissue or culture. In some embodiments, the ECM is isolated from a vertebrate animal, for example, from a mammalian vertebrate animal including, but not limited to, human, monkey, pig, cow, sheep, etc. The ECM may be derived from any organ or tissue, including without limitation, urinary bladder, intestine, liver, heart, esophagus, spleen, stomach and dermis. In specific non-limiting examples, the extracellular matrix is isolated from esophageal tissue, urinary bladder, small intestinal submucosa, dermis, umbilical cord, pericardium, cardiac tissue, or skeletal muscle. The ECM can include any portion or tissue obtained from an organ, including, for example and without limitation, submucosa, epithelial basement membrane, tunica propria, etc. In one non-limiting embodiment, the ECM is isolated from urinary bladder.

A scaffold (e.g., 100 of FIG. 19A or 19B)can be seeded with cells. For example, as shown in FIG. 19B. scaffold 100 can be first seeded with RPE cells 106, with photoreceptor progenitor (PRP) 108 cells, or both. In some examples, RPE cells 106 are seeded onto the scaffold 100, coming into contact with both the first layer 102 and second layer 103. In some examples, the RPE cells 106 form a monolayer. In some examples, after attaching RPE cells 106 to the scaffold, PRP cells 108 are added and attach on top of the RPE cells 106. The RPE cells 106 seeded onto the scaffold can be generated by the disclosed methods. In some examples, the RPE cells 106 added to the scaffold 100 are mature RPE cells, such as macular, central or peripheral RPE cells. In some examples, such mature RPE cells are macular, central and/or peripheral human RPE cells. In some examples, the RPE cells 106 are generated from pluripotent stem cells attached to the scaffold 100. Thus, in some examples, RPE cells 106 shown in FIG. 19B are pluripotent stem cells are seeded onto the scaffold 100, coming into contact with both the first layer 102 and second layer 103, and are cultured to generate mature RPE cells using the methods provided herein. Thus in some examples, RPE cells 106 are at some stages (or some examples) pluripotent stem cells, RPE progenitor cells, committed RPE cells, immature RPE cells, or mature RPCE cells (such as macular, central and/or peripheral human RPE cells).

Therefore, provided herein are scaffolds that have at least two layers. The first layer is composed of PLGA having a DL-lactide/glycolide ratio of at least about 0.25:3 to 3:0.25 (such as 1:1), a fiber diameter of at least about 150 nm (such as a diameter of about 150 to about 650 nm, such as a diameter of 450 nm), and an average pore size of at least about 0.2 microns in diameter (such as about 0.2 to 2 microns, such as 1 micron in diameter). The first layer can have a height of at least about 5 microns (such as about 5 microns to about 40 microns, such as 20 microns). The second layer is composed of PCL loops, wherein the fiber of the loops have a diameter of at least 5 microns (such as a diameter of about 5 to about 300 microns). In some examples, the PCL loops are present at a density of about 1 per at least 10 cells, about 1 per at least 25 cells, about 1 per at least 50 cells, about 1 per at least 75 cells, or about 1 per at least 100 cells, such as about 1 per 10 to 100 cells, 1 per 50 to 100 cells. In some examples, the PCL loops are closed (e.g., both ends of the PCL fiber are in contact with the first layer of the scaffold, or one end is in contact with the first layer of the scaffold, and the other end of the PCL fiber is in contact with at least one other region of the same PCL fiber, for example forming a "p" or "b" like structure). In some examples, the PCL loops are open (e.g., one end of the PCL fiber is not in contact with the first layer 102, and in some examples forms a hook-like structure, for example an "j" like structure). In some examples, some PCL loops are open, and some PCL loops are closed.

The second layer is attached to (or deposited onto) the first layer (*e.g.,* PCL loops are attached to the PLGA), for example using electrospinning or electrochemical etching. In one example, electrospinning includes using an electric field voltage of at least about 5 kV (such as at least about 10 kV, at least about 20 kV, at least about 25 kV, at least about 30 kV, at least about 40 kV, or at least about 50 kV, such as about 5 to about 50 kV, about 5 to about 40 kV, about 10 to about 30 kV, about 20 to about 30 kV, such as 10 kV, 20 kV, 25 kV, 30 kV or 40 kV, such as 25 kv), a gas ejection pressure of at least about 10 kPa (such as at least about 50 kPa, at least about 100 kPa, 200 kPa, at least about 300 kPa, at least about 350 kPa, or at least about 400 kPa, such as about 10 kPa to about 400kPa, about 100 kPa to about 400kPa, about 150 kPa to about 300 kPa, about 200 kPa to about 300 kPa, or about 100 kPa to about 200 kPa, such as 10 kPa, 25 kPa, 50 kPa, 75 kPa, 100 kPa, 200 kPa, 300 kPa, or 400 kPa, such as 300 kPa), a working distance between the nozzle and the PLGA of at least about 10 mm (such as at least about 20 mm, at least about 30 mm, or at least about 40 mm, such as about 10 mm to about 40 mm, about 10 mm to about 30 mm, about 20 mm to about 30 mm, such as 10 mm, 20 mm, 27 mm, 30 mm, 35 mm, or 40 mm, such as 27mm], and at least 2 minutes of electrospinning time (such as at least 2 minutes, at least 3 minutes, at least 4 minutes, at least 5 minutes, at least 6 minutes, at least 7 minutes, at least 8 minutes, at least 9 minutes, at least 10 minutes, such as about 2 minutes to about 10 minutes, about 2 minutes to about 8 minutes, about 4 minutes to about 6 minutes, such as about 1, 2, 3, 4, 5, 6, 7, 8, 9 or 10 minutes, such as 5 minutes).

In some examples, a scaffold (e.g., 100 of FIG. 19A or 19B) further includes a coating of a cellular adhesion protein, such as a recombinant cellular adhesion protein. The coating of cellular adhesion protein covers the entire surface of the scaffold, including the first and second layers. Exemplary cellular adhesion proteins that can be used include one or more of vitronectin, laminin, and fibronectin. In some examples, a scaffold is coated with at least about 20 ng/ml cellular adhesion protein, such as about 20-95 ng/ml cellular adhesion protein, such as 45.5 ng/ml cellular adhesion protein, such as 45.5 ng/ml vitronectin, 45.5 ng/ml laminin, or 45.5 ng/ml fibronectin.

In some examples, a scaffold (e.g., 100 of FIG. 19A or 19B) is sterilized before seeding with cells, such as one or more of pluripotent stem cells, RPE progenitor cells, committed RPE cells, immature RPE cells, mature RPCE cells (such as macular, central and/or peripheral human RPE cells), and PRP cells. In some embodiments, gamma irradiation is utilized to sterilize the scaffold. In other embodiments, an electron beam (ebeam) is used to sterilize the scaffold. In some examples oxygen plasma emission is used to sterilize the scaffold. Exemplary methods are disclosed, for example, in Bruyas et al., Tissue Eng. Part A, doi: 10.1089/ten.TEA.2018.0130 (September 20, 2018) and Proffen et al., J. Orthop. Res. 33(7) 1015-1023 (2015)).

In some examples, a scaffold (e.g., 100 of FIG. 19A) is seeded with cells, for example *ex vivo.* Thus, in some examples, a scaffold further includes RPE cells, PRP cells, or both RPE cells and PRP cells. In some examples, the RPE cells are macular, central, and/or peripheral RPE cells. In some examples, the RPE cells are matured on the scaffold, and thus in some examples the scaffold further includes pluripotent stem cells, RPE progenitor cells, committed RPE cells, immature RPE cells, or mature RPE cells (such as macular, central and/or peripheral human RPE cells). In some examples, the pluripotent stem cells, RPE progenitor cells, committed RPE cells, immature RPE cells, or mature RPE cells (such as macular, central and/or peripheral human RPE cells) are a monolayer of cells in contact with both layers of the scaffold. The PRP cells can be seeded on top of the RPE cells, for example on top of a RPE monolayer. For example, pluripotent stem cells, RPE progenitor cells, committed RPE cells, immature RPE cells, or mature RPE cells (such as macular, central and/or peripheral human RPE cells) can be seeded onto the scaffold by adding the cells to the scaffold, and culturing the cells in the presence of appropriate culture media, such as RPE-MM media. In some examples, pluripotent stem cells, RPE progenitor cells, committed RPE cells, immature RPE cells, or mature RPE cells (such as macular, central and/or peripheral human RPE cells) are allowed to grow on the scaffold for at least 1 week, at least 2 weeks, at least 3 weeks, at least 4 weeks, at least 5 weeks, or at least 6 weeks, such as 7-31 days, 10-30 days, 14-21 days, 7-45 days, such as 1, 2, 3, 4, 5, or 6 weeks. Subsequently, (for example after the cells, such as mature RPE cells form a monolayer), PRP cells are added and cultured in the presence of appropriate culture media, such as RPE-MM media. In some examples, PRP cells are allowed to grow on the REP cells for at least 5 days, at least 1 week, at least 2 weeks, or at least 3 weeks, such as 5-31 days, 10-30 days, 7-14 days, 14-21 days, such as 1, 2, or 3 weeks. Thus, in some examples, the scaffold further includes at least 100,000 cells/cm² RPE cells (such as at least 200,000 cells/cm² RPE cells, at least 300,000 cells/cm² RPE cells, at least 400,000 cells/cm² RPE cells, or at least 500,000 cells/cm² RPE cells, such as 100,000 - 500,000 cells/cm² RPE cells, 250,000 - 500,000 cells/cm² RPE cells, or 250,000 - 350,000 cells/cm² RPE cells. In some examples, the scaffold further includes at least 1 million cells/cm² PRP cells (such as at least 2 million cells/cm² PRP cells, at least 3 million cells/cm² PRP cells, at least 4 million cells/cm² PRP cells, at least 5 million cells/cm² PRP cells, or at least 6 million cells/cm² PRP cells, such as 1 to 6 million cells/cm² PRP cells, 2 to 6 million cells/cm² PRP cells, or 3 to 4 million cells/cm² PRP cells). In some examples, the scaffold further includes at least 100,000 cells/cm² RPE cells (such as at least 200,000 cells/cm² RPE cells, at least 300,000 cells/cm² RPE cells, at least 400,000 cells/cm² RPE cells, or at least 500,000 cells/cm² RPE cells, such as 100,000 - 500,000 cells/cm² RPE cells, 250,000 - 500,000 cells/cm² RPE cells, or 250,000 - 350,000 cells/cm² RPE cells) and at least 1 million cells/cm² PRP cells (such as at least 2 million cells/cm² PRP cells, at least 3 million cells/cm² PRP cells, at least 4 million cells/cm² PRP cells, at least 5 million cells/cm² PRP cells, or at least 6 million cells/cm² PRP cells, such as 1 to 6 million cells/cm² PRP cells, 2 to 6 million cells/cm² PRP cells, or 3 to 4 million cells/cm² PRP cells).

A scaffold (e.g., 100 of FIG. 19A or 19B) can include one or more pharmaceutical agents. In some embodiments, the scaffold provides sustained release of one or more pharmaceutical agents. In some embodiments, the pharmaceutical agent is a molecule that inhibits de-differentiation (or epithelial to mesenchymal transition) of RPE cells or inhibit formation of drusen-deposits underneath RPE cells or suppresses reactive oxygen species in RPE cells. In some non-limiting examples, the inhibitor of RPE cell de-differentiation is L,745,870 (3-([4-(4-chlorophenyl)piperazin-1-yl]methyl)-1H-pyrrolo[2,3-b]pyridine) or a dopamine receptor inhibitor. The pharmaceutical agent can be metformin, a Nox4 inhibitor, a reactive oxygen inhibitor aminocaproic acid, Riluzole, or a NK-κβ inhibitor. In a specific non-limiting example, the pharmaceutical agent is L,745,870. In another specific non-limiting example, the pharmaceutical agent is metformin. In a further non-limiting example, the pharmaceutical agent is a Nox4 inhibitor (e.g., VAS2870, GKT 137831 or GLX7013114). In a further non-limiting example, the pharmaceutical agent is a reactive oxygen species inhibitor (e.g., GKT 137831 or GLX7013114 or N-acetylcysteine).

In some examples, the RPE cells on the scaffold are macular, central and/or peripheral RPE cells, such as human macular, central and/or peripheral RPE cells, and are generated by a method provided herein. In some examples, the RPE cells are generated by a) culturing pluripotent stem cells in a retinal induction medium to initiate differentiation of the cells into RPE progenitor cells; b) culturing the RPE progenitor cells in a retinal differentiation medium to further differentiate the RPE progenitor cells into committed RPE cells; c) culturing the committed RPE cells in a retinal medium to form immature RPE cells; and d) culturing the immature RPE cells in a RPE maturation medium comprising a retinoic acid receptor (RAR) antagonist and/or a canonical Wnt inhibitor, thereby producing human RPE cells. In some examples, the stem cells are induced pluripotent stem cells (iPSC). Thus, in some examples, the scaffold is seeded with pluripotent stem cells such that the cells are in contact with both layers of the scaffold, and the pluripotent stem cells matured into mature RPEs on the scaffold in the presence of the media described above. In some examples, the RAR antagonist is AGN 193109, CE 2665, ER 5081, LE 135, LY 2955303, MM 11253, or liarozole dihydrochloride. In some examples, the canonical Wnt inhibitor is 4-(1,3,3a,4,7,7a-Hexahydro-1,3-dioxo-4,7-methano-2H-isoindol-2-yl)-N-8-quinolinyl-Benzamide (Endo-1-IWR), Calphostin C, Cardionogen 1, CCT 031374 hydrobromide, IWP 12, XAV 939, WIKI4, ICG-001, Wnt-C59 (C59), IWR-1-endo, KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, or TC-E 5001. In some examples, the RPE maturation medium includes at least one primary cilium inducer, such as prostaglandin E2 (PGE2) or aphidicolin.

Also provided are non-biodegradable porous polycarbonate membranes that include a scaffold provided herein.

Also provided are kits that include one or more scaffolds provided herein. Such a kit can further include one or more of vitronectin, laminin, fibronectin. In some examples, the kit further includes a snap-well culture system, a polytetrafluoroethylene (PTFE) O-ring, or both. In some examples, the kit further includes culture media, such as retinal induction media, retinal differentiation media, retinal maturation media, and/or retinal media. In some examples, the kit further includes a non-biodegradable porous polycarbonate membrane. In some examples, the kit further includes RPE cells, PRP cells, or both, such as macular, central and/or peripheral RPE cells, such as human macular, central and/or peripheral RPE cells, and are generated by a method provided herein. In some examples, the kit further includes pluripotent stem cells, such as iPSCs, RPE progenitor cells, committed RPE cells, and/or immature RPE cells.

Also provided are methods of using the disclosed scaffolds, for example to treat a subject in need thereof, such as a mammal, such as a human. Such methods can include implanting a scaffold provided herein into a retina of the subject. In some examples, the subject has a retinal degenerative disease, retinal dysfunction, retinal degradation, retinal damage, or loss of retinal pigment epithelium. In some examples, the retina degenerative disease is Stargardt's macular dystrophy, retinitis pigmentosa, age related macular degeneration, glaucoma, diabetic retinopathy, Lebers congenital amaurosis, acquired macular degeneration, hereditary macular degeneration, Best disease, late onset retinal degeneration, bear track dystrophy, retinal detachment, gyrate atrophy, choroideremia, pattern dystrophy. n some examples, the retinal damage is caused by laser, inflammatory, infectious, radiation, neovascular or traumatic injury.

Also provided are pharmaceutical compositions that include one or more scaffolds provided herein, for example for use in a method provided herein.

Also provided are methods for making a scaffold seeded with RPE cells, PRP cells, or both. Such a seeded scaffold can be used in the methods of treatment provided herein. The method can include culturing RPE cells (such as macular, central and/or peripheral RPE cells) onto a scaffold provided herein. In some examples, macular, central and/or peripheral RPE cells are generated by a method comprising a) culturing pluripotent stem cells in a retinal induction medium to initiate differentiation of the cells into RPE progenitor cells; b) culturing the RPE progenitor cells in a retinal differentiation medium to further differentiate the RPE progenitor cells into committed RPE cells; c) culturing the committed RPE cells in a retinal medium to form immature RPE cells; and d) culturing the immature RPE cells in a RPE maturation medium comprising a retinoic acid receptor (RAR) antagonist and/or a canonical Wnt inhibitor, thereby producing macular, central or peripheral RPE cells. In some examples, such culturing is performed on the scaffold itself, such that pluripotent stem cells are seeded onto the scaffold, and are then culture to form mature RPE cells (e.g., macular, central and/or peripheral RPE cells) on the scaffold. In other examples, the culturing is performed separately, and the resulting mature RPE cells (e.g., macular, central and/or peripheral RPE cells) are subsequently seeded onto the scaffold. In some examples the RPE cells are cultured for a period of time to allow formation of a monolayer of RPE cells, such as a monolayer in contact with both layers of the scaffold (e.g., see FIG. 19B). In some examples, the RPE cells are cultured in the presence of the scaffold in retinal maturation media. In some examples, the RPE cells (or pluripotent stem cells (such as iPSCs) RPE progenitor cells, committed RPE cells, and/or immature RPE cells) are cultured on the scaffold for at least 1 week, at least 2 weeks, at least 3 weeks, at least 4 weeks, or at least 5 weeks, such as about 1, 2, 3, 4, or 5 weeks. The method can further include subsequently culturing PRP cells on top of the RPE cells *(e.g.,* see FIG. 19B). The PRP cells need not form a monolayer, and in some examples the confluency is less than about 40%, such as about 5-40% confluency, about 20-40% confluency or about 30-40% confluency. In some examples, the PRP cells are cultured in the presence of the scaffold and RPE cells in retinal maturation media. In some examples, the PRP cells are cultured on the scaffold and RPE cells for at least 1 week, at least 2 weeks, at least 3 weeks, or at least 4 weeks, such as about 1, 2, 3, or 4 weeks. In some examples, the resulting scaffold contains at least 100,000 cells/cm² RPE cells (such as at least 200,000 cells/cm² RPE cells, at least 300,000 cells/cm² RPE cells, at least 400,000 cells/cm² RPE cells, or at least 500,000 cells/cm² RPE cells, such as 100,000 - 500,000 cells/cm² RPE cells, 250,000 - 500,000 cells/cm² RPE cells, or 250,000 - 350,000 cells/cm² RPE cells. In some examples, the scaffold includes at least 1 million cells/cm² PRP cells (such as at least 2 million cells/cm² PRP cells, at least 3 million cells/cm² PRP cells, at least 4 million cells/cm² PRP cells, at least 5 million cells/cm² PRP cells, or at least 6 million cells/cm² PRP cells, such as 1 to 6 million cells/cm² PRP cells, 2 to 6 million cells/cm² PRP cells, or 3 to 4 million cells/cm² PRP cells). In some examples, the scaffold includes at least 100,000 cells/cm² RPE cells (such as at least 200,000 cells/cm² RPE cells, at least 300,000 cells/cm² RPE cells, at least 400,000 cells/cm² RPE cells, or at least 500,000 cells/cm² RPE cells, such as 100,000 - 500,000 cells/cm² RPE cells, 250,000 - 500,000 cells/cm² RPE cells, or 250,000 - 350,000 cells/cm² RPE cells) and at least 1 million cells/cm² PRP cells (such as at least 2 million cells/cm² PRP cells, at least 3 million cells/cm² PRP cells, at least 4 million cells/cm² PRP cells, at least 5 million cells/cm² PRP cells, or at least 6 million cells/cm² PRP cells, such as 1 to 6 million cells/cm² PRP cells, 2 to 6 million cells/cm² PRP cells, or 3 to 4 million cells/cm² PRP cells). In some examples, maturation of the RPE cells from pluripotent stem cells is confirmed by TEER>400 Ohms.cm2 and presence of photoereceptor markers (e.g., recoverin, arrestin, NRL, opsins).

### Pluripotent Stem Cells

### 1. Embryonic Stem Cells

ES cells are derived from the inner cell mass of blastocysts and have a high in vitro differentiating capability. ES cells can be isolated by removing the outer trophectoderm layer of a developing embryo, then culturing the inner mass cells on a feeder layer of non-growing cells. The replated cells can continue to proliferate and produce new colonies of ES cells which can be removed, dissociated, replated again and allowed to grow. This process of "subculturing" undifferentiated ES cells can be repeated a number of times to produce cell lines containing undifferentiated ES cells (U.S. Patent Nos. 5,843,780; 6,200,806; 7,029,913). ES cells have the potential to proliferate while maintaining their pluripotency. For example, ES cells are useful in research on cells and on genes which control cell differentiation. The pluripotency of ES cells combined with genetic manipulation and selection can be used for gene analysis studies in vivo via the generation of transgenic, chimeric, and knockout mice.

Methods for producing mouse ES cells are known. In one method, a preimplantation blastocyst from the 129 strain of mice is treated with mouse antiserum to remove the trophoectoderm, and the inner cell mass is cultured on a feeder cell layer of chemically inactivated mouse embryonic fibroblasts in medium containing fetal calf serum. Colonies of undifferentiated ES cells that develop are subcultured on mouse embryonic fibroblast feeder layers in the presence of fetal calf serum to produce populations of ES cells. In some methods, mouse ES cells can be grown in the absence of a feeder layer by adding the cytokine leukemia inhibitory factor (LIF) to serum-containing culture medium (Smith, 2000). In other methods, mouse ES cells can be grown in serum-free medium in the presence of bone morphogenetic protein and LIF.

Human ES cells can be produced or derived from blastocyst-staged mammalian embryo produced by parthenogenesis.

In said method, human blastocysts are exposed to anti-human serum, and trophectoderm cells are lysed and removed from the inner cell mass which is cultured on a feeder layer of mouse embryonic fibroblasts. Further, clumps of cells derived from the inner cell mass are chemically or mechanically dissociated, replated, and colonies with undifferentiated morphology are selected by micropipette, dissociated, and replated (U.S. Patent No. 6,833,269). In some methods, human ES cells can be grown without serum by culturing the ES cells on a feeder layer of fibroblasts in the presence of basic fibroblast growth factor. In other methods, human ES cells can be grown without a feeder cell layer by culturing the cells on a protein matrix such as MATRIGEL^{®} or laminin in the presence of "conditioned" medium containing basic fibroblast growth factor. Human ES cell lines are available. Pluripotent stem cells according to the claimed invention do not encompass human ES cells which required destruction of a human embryo. These include the use of established ES cell lines.

ES cells can also be derived from other organisms including rhesus monkey and marmoset by previously described methods, as well as from established mouse and human cell lines. For reference only, established human ES cell lines include MAOI, MA09, ACT-4, HI, H7, H9, H13, H14 and ACT30. As a further example, mouse ES cell lines that have been established include the CGR8 cell line established from the inner cell mass of the mouse strain 129 embryos, and cultures of CGR8 cells can be grown in the presence of LIF without feeder layers.

ES stem cells can be detected by protein markers including transcription factor Oct4, alkaline phosphatase (AP), stage-specific embryonic antigen SSEA-1, stage-specific embryonic antigen SSEA-3, stage-specific embryonic antigen SSEA-4, transcription factor NANOG, tumor rejection antigen 1-60 (TRA-1-60), tumor rejection antigen 1-81 (TRA-1-81), SOX2, or REX1.

### a. Somatic Cell Nuclear Transfer

Non-human pluripotent stem cells can be prepared through the method of somatic cell nuclear transfer. Somatic cell nuclear transfer involves the transfer of a donor nucleus into a spindle-free oocyte. In one method, donor fibroblast nuclei from skin fibroblasts of a primate are introduced into the cytoplasm of spindle-free, mature metaphase II primate ooctyes by electrofusion. The fused oocytes are activated by exposure to ionomycin, and then incubated until the blastocyst stage. The inner cell mass of selected blastocysts are then cultured to produce embryonic stem cell lines. The embryonic stem cell lines show normal ES cell morphology, express various ES cell markers, and differentiate into multiple cell types both in vitro and in vivo.

### 2. Induced Pluripotent Stem Cells

The induction of pluripotency was originally achieved in 2006 using mouse cells (Yamanaka et al.) and in 2007 using human cells by reprogramming of somatic cells via the introduction of transcription factors that are linked to pluripotency. Pluripotent stem cells can be maintained in an undifferentiated state and are capable of differentiating into almost any cell type. The use of iPSCs circumvents most of the ethical and practical problems associated with large-scale clinical use of ES cells, and patients with iPSC-derived autologous transplants may not require lifelong immunosuppressive treatments to prevent graft rejection.

With the exception of germ cells, any cell can be used as a starting point for iPSCs. For example, cell types could be keratinocytes, fibroblasts, hematopoietic cells, mesenchymal cells, liver cells, or stomach cells. The cells can be a multipotent cells, such as but not limited to a hematopoietic stem cell, such as, but no limited to, CD34+ cells. T cells may also be used as a source of somatic cells for reprogramming (U.S. Patent No. 8,741,648). There is no limitation on the degree of cell differentiation or the age of an animal from which cells are collected; even undifferentiated progenitor cells (including somatic stem cells) and finally differentiated mature cells can be used as sources of somatic cells in the methods disclosed herein. In one embodiment, the somatic cell is itself a RPE cells such as a human RPE cell. The RPE cell can be an adult or a fetal RPE cell. iPSCs can be grown under conditions that are known to differentiate human ES cells into specific cell types, and express human ES cell markers including: SSEA-1, SSEA-3, SSEA-4, TRA-1-60, and TRA-1-81.

Somatic cells and pluripotent stem cells, such as CD34+ cells, can be reprogrammed to produce induced pluripotent stem cells (iPSCs) using methods known to one of skill in the art. One of skill in the art can readily produce induced pluripotent stem cells, see for example, Published U.S. Patent Application No. 20090246875, Published U.S. Patent Application No. 2010/0210014; Published U.S. Patent Application No. 20120276636; U.S. Patent No. 8,058,065; U.S. Patent No. 8,129,187; U.S. Patent No. 8,278,620; PCT Publication NO. WO 2007/069666 A1, and U.S. Patent No. 8,268,620. Generally, nuclear reprogramming factors are used to produce pluripotent stem cells from a somatic cell. In some embodiments, at least three, or at least four, of Klf4, c-Myc, Oct3/4, Sox2, Nanog, and Lin28 are utilized. In other embodiments, Oct3/4, Sox2, c-Myc and Klf4 are utilized.

The cells are treated with a nuclear reprogramming substance, which is generally one or more factor(s) capable of inducing an iPSC from a somatic cell or a nucleic acid that encodes these substances (including forms integrated in a vector). The nuclear reprogramming substances generally include at least Oct3/4, Klf4 and Sox2 or nucleic acids that encode these molecules. A functional inhibitor of p53, L-myc or a nucleic acid that encodes L-myc, and Lin28 or Lin28b or a nucleic acid that encodes Lin28 or Lin28b, can be utilized as additional nuclear reprogramming substances. Nanog can also be utilized for nuclear reprogramming. As disclosed in published U.S. Patent Application No. 20120196360, exemplary reprogramming factors for the production of iPSCs include (1) Oct3/4, Klf4, Sox2, L-Myc (Sox2 can be replaced with Sox1, Sox3, Soxl5, Soxl7 or Soxl8; Klf4 is replaceable with Klf1, Klf2 or Klf5); (2) Oct3/4, Klf4, Sox2, L-Myc, TERT, SV40 Large T antigen (SV40LT); (3) Oct3/4, Klf4, Sox2, L-Myc, TERT, human papilloma virus (HPV)16 E6; (4) Oct3/4, Klf4, Sox2, L-Myc, TERT, HPV16 E7 (5) Oct3/4, Klf4, Sox2, L- Myc, TERT, HPV16 E6, HPV16 E7; (6) Oct3/4, Klf4, Sox2, L-Myc, TERT, Bmil; (7) Oct3/4, Klf4, Sox2, L-Myc, Lin28; (8) Oct3/4, Klf4, Sox2, L-Myc, Lin28, SV40LT; (9) Oct3/4, Klf4, Sox2, L-Myc, Lin28, TERT, SV40LT; (10) Oct3/4, Klf4, Sox2, L-Myc, SV40LT; (11) Oct3/4, Esrrb, Sox2, L-Myc (Esrrb is replaceable with Esrrg); (12) Oct3/4, Klf4, Sox2; (13) Oct3/4, Klf4, Sox2, TERT, SV40LT; (14) Oct3/4, Klf4, Sox2, TERT, HP VI 6 E6; (15) Oct3/4, Klf4, Sox2, TERT, HPV16 E7; (16) Oct3/4, Klf4, Sox2, TERT, HPV16 E6, HPV16 E7; (17) Oct3/4, Klf4, Sox2, TERT, Bmil; (18) Oct3/4, Klf4, Sox2, Lin28 (19) Oct3/4, Klf4, Sox2, Lin28, SV40LT; (20) Oct3/4, Klf4, Sox2, Lin28, TERT, SV40LT; (21) Oct3/4, Klf4, Sox2, SV40LT; or (22) Oct3/4, Esrrb, Sox2 (Esrrb is replaceable with Esrrg). In one non-limiting example, Oct3/4, Klf4, Sox2, and c-Myc are utilized. In other embodiments, Oct4, Nanog, and Sox2 are utilized, see for example, U.S. Patent No. 7,682,828. These factors include, but are not limited to, Oct3/4, Klf4 and Sox2. In other examples, the factors include, but are not limited to Oct 3/4, Klf4 and Myc. In some non-limiting examples, Oct3/4, Klf4, c-Myc, and Sox2 are utilized. In other non-limiting examples, Oct3/4, Klf4, Sox2 and Sal 4 are utilized. Factors like Nanog, Lin28, Klf4, or c-Myc can increase reprogramming efficiency and can be expressed from several different expression vectors. For example, an integrating vector such as the EBV element-based system can be used (U.S. Patent No. 8,546,140). In a further aspect, reprogramming proteins could be introduced directly into somatic cells by protein transduction. Reprogramming may further comprise contacting the cells with one or more signaling receptors including glycogen synthase kinase 3 (GSK-3) inhibitor, a mitogen-activated protein kinase (MEK) inhibitor, a transforming growth factor beta (TGF-β) receptor inhibitor or signaling inhibitor, leukemia inhibitory factor (LIF), a p53 inhibitor, an NF-kappa B inhibitor, or a combination thereof. Those regulators may include small molecules, inhibitory nucleotides, expression cassettes, or protein factors. It is anticipated that virtually any iPS cells or cell lines may be used.

Mouse and human cDNA sequences of these nuclear reprogramming substances are available with reference to the NCBI accession numbers recited in WO 2007/069666. Methods for introducing one or more reprogramming substances, or nucleic acids encoding these reprogramming substances, are known i, and disclosed for example, in U.S. Patent Application No. 2012/0196360 and U.S. Patent No. 8,071,369.

Once derived, iPSCs can be cultured in a medium sufficient to maintain pluripotency. The iPSCs may be used with various media and techniques developed to culture pluripotent stem cells, more specifically, embryonic stem cells, as described in U.S. Patent No. 7,442,548 and U.S. Patent Pub. No. 2003/0211603. In the case of mouse cells, the culture is carried out with the addition of Leukemia Inhibitory Factor (LIF) as a differentiation suppression factor to an ordinary medium. In the case of human cells, it is desirable that basic fibroblast growth factor (bFGF) be added in place of LIF. Other methods for the culture and maintenance of iPSCs, may be used.

In certain embodiments, undefined conditions may be used; for example, pluripotent cells may be cultured on fibroblast feeder cells or a medium that has been exposed to fibroblast feeder cells in order to maintain the stem cells in an undifferentiated state. In some embodiments, the cell is cultured in the co-presence of mouse embryonic fibroblasts treated with radiation or an antibiotic to terminate the cell division, as feeder cells. Alternately, pluripotent cells may be cultured and maintained in an essentially undifferentiated state using a defined, feeder-independent culture system, such as a TESR^{™} medium or E8^{™} medium.

In some embodiments, the iPSCs can be modified, such as to express an exogenous gene, increase expression of an endogenous gene, increase copy number of a gene, to correct a gene mutation, or to silence the expression of a mutant gene. In some specific non-limiting examples, a mutation or a deletion in an endogenous gene is corrected. The gene can encode, for example, retinoid isomerohydrase (RPE65), bestrophin (BEST)1, MER tyrosine kinase proto-oncogene (MERTK), RAB escort protein (REP1), cellular retinaldyehyde binding protein (CRALBP), pre-mRNA processing factor (PPRF), complement factor H (CFH), complement component 3a receptor (C3aR)1, complement component 5 receptor (C5aR1), vascular endothelial growth factor (VEGF), pigment epithelium-derived factor (PEDF), complement factor I (CFI), complement factor 2B (C2B), ATP-binding cassette, subfamily A, member 4 (ABCA4), ATP-binding cassette, subfamily A, member 1 (ABCA1), membrane-type frizzeled related protein (MFRP), C1q and tumor necrosis factor related protein 5 (C1qTNF5), spermatogenesis-associated protein 7 (SPATA7), centrosomal protein, 290 kd (CEP290), myosin VIIA (MYO7A), cilliary neurotrophic factor (CNTF), FMS-related tyrosine kinase 1 (FLT-1), Usher syndrome, type I (USH1A), tyrosinase, plasminogen (PLG), collagen, type XVIII, alpha-1 (COL18A1), TTRA serine peptidase (HTRA)1, ARMS2, tissue inhibitor of metalloproteinase (TIMP)3, epithelial growth factor (EGF) containing fibulin-like extracellular matrix protein (EFEMP)1, microphthalmia-associated transcription factor (MITF), transcription factor EC (TFEC), orthodenticle, drosophila, homolog of, 2 (OTX2), zinc finger protein 503 (ZNF503 or NLZ2). In one non-limiting example, the gene is RPE65. In another non-limiting example, the gene is BEST1. If a further non-limiting example, the gene encodes METRK. Methods for performing gene editing in iPSCs are disclosed, for example, in Hockenmeyer and Jaenisch, "Induced Pluripotent Stem Cell Meets Genome Editing," Cell Stem Cell 18: 573-586, 2016. Any of the methods disclosed therein are of use. The method can include the use of a viral vector, such as an adeno-associated viral vector or a lentiviral vector ending a transgene of interest. The method can include the use of CRISPR/Cas9, TALEN nuclease, Zinc-finger nuclease, lentiviral mediated correction, adeno-associated virus mediated correction, shRNA, siRNA, or F-prime editing.

In some embodiments, the iPSC can be modified to express exogenous nucleic acids, such as to include a tyrosinase enhancer operably linked to a promoter and a nucleic acid sequence encoding a first marker. The tyrosinase gene is disclosed, for example, in GENBANK^{®} Accession No. 22173, as available on January 1, 2013. This sequence aligns to chromosome 7 of mouse strain C57BL/6 location 5286971-5291691 (invert orientation). A 4721 base pair sequence is sufficient for expression in RPE cells, see Murisier et al., Dev. Biol. 303: 838-847, 2007. This construct is expressed in RPE cells. Other enhancers can be utilized. Other RPE-specific enhancers include D-MITF, DCT, TYRP1, RPE65, VMD2, MERTK, MYRIP, and RAB27A. Suitable promoters include, but are not limited to, any promoter expressed in RPE cells including the tyrosinase promoter. The construct can also include other elements, such as a ribosome binding site for translational initiation (internal ribosomal binding sequences), and a transcription/translation terminator. Generally, it is advantageous to transfect cells with the construct. Suitable vectors for stable transfection include, but are not limited to retroviral vectors, lentiviral vectors and Sendai virus.

Plasmids can achieve regulated high copy number and are compatible with use in mammalian cells, including human cells. In some examples, plasmids, they are suitable for maintenance and fermentation in *E. coli,* so that large amounts of DNA can be produced and purified. Plasmids can be safe and suitable for use in human patients and animals. High copy number plasmids can be selected for and stably maintained relatively easily during bacterial fermentation. Elements such as selectable markers and other coding sequences can be included in a plasmid. In some embodiments plasmids that encode a marker include: (1) a high copy number replication origin, (2) a selectable marker, such as, but not limited to, the neo gene for antibiotic selection with kanamycin, (3) transcription termination sequences, including the tyrosinase enhancer and (4) a multicloning site for incorporation of various nucleic acid cassettes; and (5) a nucleic acid sequence encoding a marker operably linked to the tyrosinase promoter. There are numerous plasmid vectors that are known in the art for inducing a nucleic acid encoding a protein, such as the vectors disclosed in U.S. Patent No. 6,103,470; U.S. Patent No. 7,598,364; U.S. Patent No. 7,989,425; and U.S. Patent No 6,416,998.

A viral gene delivery system can be an RNA-based or DNA-based viral vector. An episomal gene delivery system can be a plasmid, an Epstein-Barr virus (EBV)-based episomal vector, a yeast-based vector, an adenovirus-based vector, a simian virus 40 (SV40)-based episomal vector, a bovine papilloma virus (BPV)-based vector, or a lentiviral vector.

In some embodiments, the cells are transfected with a nucleic acid molecule encoding a marker. Markers include, but are not limited to, fluorescence proteins (for example, green fluorescent protein or red fluorescent protein), enzymes (for example, horse radish peroxidase or alkaline phosphatase or firefly/renilla luciferase or nanoluc), or other proteins. A marker may be a protein (including secreted, cell surface, or internal proteins; either synthesized or taken up by the cell); a nucleic acid (such as an mRNA, or enzymatically active nucleic acid molecule) or a polysaccharide. Included are determinants of any such cell components that are detectable by antibody, lectin, probe or nucleic acid amplification reaction that are specific for the marker of the cell type of interest. The markers can also be identified by a biochemical or enzyme assay or biological response that depends on the function of the gene product. Nucleic acid sequences encoding these markers can be operably linked to the tyrosinase enhancer. In addition, other genes can be included, such as genes that may influence stem cell to RPE differentiation, or RPE function, or physiology, or pathology. Thus, in some embodiments, a nucleic acid is included that encodes one or more of MITF, PAX6, TFEC, OTX2, LHX2, VMD2, CFTR, RPE65, MFRP, CTRP5, CFH, C3, C2B, APOE, APOB, mTOR, FOXO, AMPK, SIRT1-6, HTRP1, ABCA4, TIMP3, VEGFA, CFI, TLR3, TLR4, APP, CD46, BACE1, ELOLV4, ADAM 10, CD55, CD59, and ARMS2.

### a. MHC Haplotype Matching

Major Histocompatibility Complex is the main cause of immune-rejection of allogeneic organ transplants. There are three major class I MHC haplotypes (A, B, and C) and three major MHC class II haplotypes (DR, DP, and DQ). The HLA loci are highly polymorphic and are distributed over 4 Mb on chromosome 6. The ability to haplotype the HLA genes within the region is clinically important since this region is associated with autoimmune and infectious diseases and the compatibility of HLA haplotypes between donor and recipient can influence the clinical outcomes of transplantation. HLAs corresponding to MHC class I present peptides from inside the cell and HLAs corresponding to MHC class II present antigens from outside of the cell to T-lymphocytes. Incompatibility of MHC haplotypes between the graft and the host triggers an immune response against the graft and leads to its rejection. Thus, a subject can be treated with an immunosuppressant to prevent rejection. HLA-matched stem cell lines may overcome the risk of immune rejection.

Because of the importance of HLA in transplantation, the HLA loci are usually typed by serology and PCR for identifying favorable donor-recipient pairs. Serological detection of HLA class I and II antigens can be accomplished using a complement mediated lymphocytotoxicity test with purified T or B lymphocytes. This procedure is predominantly used for matching HLA-A and -B loci. Molecular-based tissue typing can often be more accurate than serologic testing. Low resolution molecular methods such as SSOP (sequence specific oligonucleotide probes) methods, in which PCR products are tested against a series of oligonucleotide probes, can be used to identify HLA antigens, and currently these methods are the most common methods used for Class II-HLA typing. High resolution techniques such as SSP (sequence specific primer) methods which utilize allele specific primers for PCR amplification can identify specific MHC alleles.

MHC compatibility between a donor and a recipient increases significantly if the donor cells are HLA homozygous, i.e. contain identical alleles for each antigen-presenting protein. Most individuals are heterozygous for MHC class I and II genes, but certain individuals are homozygous for these genes. These homozygous individuals can serve as super donors and grafts generated from their cells can be transplanted in all individuals that are either homozygous or heterozygous for that haplotype. Furthermore, if homozygous donor cells have a haplotype found in high frequency in a population, these cells may have application in transplantation therapies for a large number of individuals.

Accordingly, iPSCs can be produced from cells, such as CD34⁺ cells, of the subject to be treated, or another subject with the same or substantially the same HLA type as that of the patient. In one case, the major HLAs (e.g., the three major loci of HLA-A, HLA-B and HLA-DR) of the donor are identical to the major HLAs of the recipient. In some cases, the somatic cell donor may be a super donor; thus, iPSCs derived from a MHC homozygous super donor may be used to generate RPE cells. Thus, the iPSCs derived from a super donor may be transplanted in subjects that are either homozygous or heterozygous for that haplotype. For example, the iPSCs can be homozygous at two HLA alleles such as HLA-A and HLA-B. As such, iPSCs produced from super donors can be used in the methods disclosed herein, to produce RPE cells that can potentially "match" a large number of potential recipients.

### b. Episomal Vectors

In certain aspects, reprogramming factors are expressed from expression cassettes comprised in one or more exogenous episiomal genetic elements (see U.S. Patent Publication 2010/0003757. Thus, iPSCs can be essentially free of exogenous genetic elements, such as from retroviral or lentiviral vector elements. These iPSCs are prepared by the use of extra-chromosomally replicating vectors (i.e., episomal vectors), which are vectors capable of replicating episomally to make iPSCs essentially free of exogenous vector or viral elements (see U.S. Patent No. 8,546 140; Yu et al., 2009). A number of DNA viruses, such as adenoviruses, simian virus 40 (SV40) or bovine papilloma virus (BPV), or budding yeast ARS (Autonomously Replicating Sequences)-containing plasmids replicate extra-chromosomally or episomally in mammalian cells. These episomal plasmids are intrinsically free from all these disadvantages (Bode et al., 2001) associated with integrating vectors. For example, a lymphotrophic herpes virus-based including or Epstein Barr Virus (EBV) as defined above may replicate extra-chromosomally and help deliver reprogramming genes to somatic cells. Useful EBV elements are OriP and EBNA-1, or their variants or functional equivalents. One advantage of episomal vectors is that the exogenous elements will be lost with time after being introduced into cells, leading to self-sustained iPSCs essentially free of these elements.

Other extra-chromosomal vectors include other lymphotrophic herpes virus-based vectors. Lymphotrophic herpes virus is a herpes virus that replicates in a lymphoblast (e.g., a human B lymphoblast) and becomes a plasmid for a part of its natural life-cycle. Herpes simplex virus (HSV) is not a "lymphotrophic" herpes virus. Exemplary lymphotrophic herpes viruses include, but are not limited to EBV, Kaposi's sarcoma herpes virus (KSHV); herpes virus saimiri (HS) and Marek's disease virus (MDV). Additional sources of episome-based vectors are contemplated, such as yeast ARS, adenovirus, SV40, or BPV.

### Methods for Producing RPE Cells

Methods for producing macular, central or peripheral RPE cells are disclosed herein. In some embodiments, cells can be produced from pluripotent stem cells, such as ESCs or iPSCs. In some examples, macular, central and/or peripheral RPE cells are produced on a scaffold provided herein, for example by seeding the scaffold with pluripotent stem cells, such as ESCs or iPSCs, and performing the culturing steps provided herein.

RPE cells can be characterized based upon their pigmentation, epithelial morphology, and apical-basal polarity. Differentiated RPE cells can be visually recognized by their morphology and the initial appearance of pigment. In humans, a "macular" RPE cell has an area of about 150 µm² ± 33 µm² and petal-like apical process, which have a length of about 0.5 to about 4µm, a width of about 0.1 to about 1µm, and a width of undulation of about 0.2 to about 2µm. A central RPE cell has an area of about 199 µm² + 40 µm² with mixed petal-like and finger-like apical processes. A "peripheral" RPE cell has an area of about 239 µm²-± 33 µm²-and finger-like apical process, which have a length of about 0.1 to about1 µm and a width of about 0.1 to about 1µm. Methods are disclosed for producing these types of RPE cells.

In addition, all differentiated RPE cells have transepithelial resistance/TER, and trans-epithelial potential/TEP across the monolayer (TER >100 Ohms*cm²; TEP >2 mV), transport fluid and CO₂ from the apical to basal side, and regulate a polarized secretion of cytokines. RPE cells express several proteins that can serve as markers for detection by the use of methodologies, such as immunocytochemistry, Western blot analysis, flow cytometry, and enzyme-linked immunoassay (ELISA). For example, RPE-specific markers may include: cellular retinaldehyde binding protein (CRALBP), microphthalmia-associated transcription factor (MITF), tyrosinase-related protein 1 (TYRP-1), retinal pigment epithelium-specific 65 kDa protein (RPE65), premelanosome protein (PMEL17), bestrophin 1 (BEST1), and c-mer proto-oncogene tyrosine kinase (MERTK). RPE cells do not express (at any detectable level) the embryonic stem cells markers Oct-4, nanog or Rex-2. Specifically, expression of these genes is approximately 100-1000 fold lower in RPE cells than in ES cells or iPSCs, when assessed by quantitative RT-PCR. RPE cell markers may be detected at the mRNA level, for example, by reverse transcriptase polymerase chain reaction (RT-PCR), Northern blot analysis, or dot-blot hybridization analysis using sequence-specific primers in standard amplification methods using publicly available sequence data (GENBANK^{®}). Expression of tissue-specific markers as detected at the protein or mRNA level is considered positive if the level is at least or about 2-, 3-, 4-, 5-, 6-, 7-, 8-, or 9-fold, and more particularly more than 10-, 20-, 30, 40-, 50-fold or higher above that of a control cell, such as an undifferentiated pluripotent stem cell or other unrelated cell type.

Exemplary methods for producing macular, central and peripheral RPE cells from iPSCs and ESCs are disclosed below. A description of inhibitors that can be used in the media are disclosed at the end of this section. It should be noted that any of the specific inhibitors can be used wherein a reference is made to the class of inhibitors.

### 1. Culturing pluripotent stem cells to product RPE progenitor cells

The disclosed methods include culturing pluripotent stem cells (PSCs), such as ES cells or iPSCs in a retinal induction medium to initiate differentiation of the cells into RPE progenitor cells. PCT Publication No. 2014 12 1077 discloses methods wherein embryoid bodies (EBs) produced from iPSCs can be utilized in culture methods to produce RPE cells. For example, embryoid bodies are produced from iPSCs by the addition of a rho-associated coiled-coil kinase (ROCK) inhibitor and cultured in a first medium comprising two WNT pathway inhibitors and a Nodal pathway inhibitor.

In some embodiments, an essentially single cell suspension of PSCs, such as human iPSCs can be utilized in the present methods. In some embodiments, the PSCs are cultured to pre-confluency to prevent any cell aggregates. In certain aspects, the PSCs are dissociated by incubation with a cell dissociation enzyme, such as exemplified by TRYPSIN^{™} or TRYPLE^{™}. PSCs can also be dissociated into an essentially single cell suspension by pipetting. In addition, Blebbistatin (e.g., about 2.5 µM) can be added to the medium to increase PSC survival after dissociation into single cells while the cells are not adhered to a culture vessel. A ROCK inhibitor instead of Blebbistatin may alternatively be used to increase PSC survival after dissociated into single cells.

In order to efficiently differentiate RPE cells from the single cell PSCs, an accurate count of the input density can increase RPE differentiation efficiency. Thus, the single cell suspension of PSCs can be counted before seeding. For example, the single cell suspension of PSCs can be counted by a hemocytometer or an automated cell counter, such as VICELL^{®} or TC20. The cells may be diluted to a cell density of at least 10,000 cells/mL, such as at least 20,000 cells/mL, at least 50,000 cells/mL, at least 75,000 cells/mL, at least 100,000 cells/mL, at least 150,000 cells/mL, or at least 200,000 cells/mL, such as about 10,000 to about 500,000 cells/mL, about 50,000 to about 200,000 cells/mL, or about 75,000 to about 150,000 cells/mL. In a non-limiting example, the single cell suspension of PSCs is diluted to a density of about 100,000 cells/mL in a fully defined cultured medium such as ESSENTIAL 8^{™} (E8^{™}) medium.

In other embodiments, the PSCs, such as iPSCs, are plated at a cell density of about 1,000 to about 75,000 cells/cm2, such as of about 5,000 to about 40,000 cells/cm2. In other embodiments, the PSCs, such as iPSCs, are plated at a cell density of at least 1,000 cells/cm2, at least 2,000 cells/cm2, at least 5,000 cells/cm2, at least 10,000 cells/cm2, at least 20,000 cells/cm2, or at least 50,000 cells/cm2, In a 6 well plate, the cells may be seeded at a cell density of about 50,000 to about 400,000 cells per well. In exemplary methods, the cells are seeded at a cell density of about 100,000, about 150,00, about 200,000, about 250,000, about 300,000 or about 350,000 cells per well, such as about 200,00 cells per well.

Once a single cell suspension of PSCs is obtained at a known cell density, the cells are generally seeded in an appropriate culture vessel, such as a tissue culture plate, such as a flask, 6-well, 24-well, or 96-well plate. A culture vessel used for culturing the cell(s) can include, but is particularly not limited to: flask, flask for tissue culture, dish, petri dish, dish for tissue culture, multi dish, micro plate, micro-well plate, multi plate, multi-well plate, micro slide, chamber slide, tube, tray, CELLSTACK^{®} chambers, culture bag, and roller bottle, as long as it is capable of culturing the stem cells therein. The cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture. In a certain embodiment, the culture vessel may be a bioreactor, which may refer to any device or system ex vivo that supports a biologically active environment such that cells can be propagated. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable

The PSCs, such as iPSCs, are generally cultured on culture plates coated by a matrix including one or more cellular adhesion proteins to promote cellular adhesion while maintaining cell viability. For example, exemplary cellular adhesion proteins include extracellular matrix proteins such as vitronectin, laminin, collagen and/or fibronectin which may be used to coat a culturing surface as a means of providing a solid support for pluripotent cell growth. In some embodiments, iPSC are cultured on matrix that includes at least one cellular adhesion protein, such as laminin, vitronectin or fibronectin. Combinations of laminin, vitronectin and/or fibronectin are also of use. In other embodiments, the PSCs are grown on culture plates coated with vitronectin or fibronectin. In some embodiments, the cellular adhesion proteins are human proteins.

An extracellular matrix can be utilized. The term "extracellular matrix" is recognized in the art. Its components include one or more of the following proteins: fibronectin, laminin, vitronectin, tenascin, entactin, thrombospondin, elastin, gelatin, collagen, fibrillin, merosin, anchorin, chondronectin, link protein, bone sialoprotein, osteocalcin, osteopontin, epinectin, hyaluronectin, undulin, epiligrin, and kalinin.

The extracellular matrix (ECM) proteins may be of natural origin and purified from human or animal tissues or, alternatively, the ECM proteins may be genetically engineered recombinant proteins or synthetic in nature. The ECM proteins may be a whole protein or in the form of peptide fragments, native or engineered. Examples of ECM protein that may be useful in the matrix for cell culture include laminin, collagen I, collagen IV, fibronectin and vitronectin. In some embodiments, the matrix composition includes synthetically generated peptide fragments of fibronectin or recombinant fibronectin. In some embodiments, the matrix composition is xeno-free. For example, in the xeno-free matrix to culture human cells, matrix components of human origin may be used, wherein any non-human animal components may be excluded.

In some aspects, the total protein concentration in the matrix composition may be about 1 ng/mL to about 1 mg/mL. In some embodiments, the total protein concentration in the matrix composition is about 1 µg/mL to about 300 µg/mL. In other embodiments, the total protein concentration in the matrix composition is about 5 µg/mL to about 200 µg/mL.

### a. Culture conditions

Cells, such as RPE cells or PSC, can be cultured with the nutrients necessary to support the growth of each specific population of cells, such as RPE progenitor cells. Generally, the cells are cultured in growth media including a carbon source, a nitrogen source and a buffer to maintain pH. The medium can also contain fatty acids or lipids, amino acids (such as non-essential amino acids), vitamin(s), growth factors, cytokines, antioxidant substances, pyruvic acid, buffering agents, and inorganic salts. An exemplary growth medium contains a minimal essential media, such as Dulbecco's Modified Eagle's medium (DMEM) or ESSENTIAL 8^{™} (E8^{™}) medium, supplemented with various nutrients, such as non-essential amino acids and vitamins, to enhance stem cell growth. Examples of minimal essential media include, but are not limited to, Minimal Essential Medium Eagle (MEM) Alpha medium, Dulbecco's modified Eagle medium (DMEM), RPMI-1640 medium, 199 medium, and F12 medium. Additionally, the minimal essential media may be supplemented with additives such as horse, calf or fetal bovine serum. Alternatively, the medium can be serum free. In other cases, the growth media may contain "knockout serum replacement," referred to herein as a serum-free formulation optimized to grow and maintain undifferentiated cells, such as stem cell, in culture. KNOCKOUT^{™} serum replacement is disclosed, for example, in U.S. Patent Application No. 2002/0076747.

In some embodiments, the PSCs are cultured in a fully defined and feeder free media.

Accordingly, the single cell PSCs are generally cultured in a fully defined culture medium after plating. In certain aspects, about 18-24 hours after seeding, the medium is aspirated and fresh medium, such as E8^{™} medium, is added to the culture. In certain aspects, the single cell PSCs are cultured in the fully defined culture medium for about 1, 2 or 3 days after plating. In some non-limiting examples, the single cells PSCs are cultured in the fully defined culture medium for about 2 days before proceeding with the differentiation process.

In some embodiments, the medium may contain alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolgiycerol, or equivalents thereto. The alternatives to serum can be prepared by the method disclosed in International Publication No. WO 98/30679, for example. Alternatively, any commercially available materials can be used for more convenience. The commercially available materials include KNOCKOUT^{™} Serum Replacement (KSR), Chemically-defined Lipid concentrated (Gibco), and GLUTAMAX^{™} (Gibco). The medium can be serum free.

Other culturing conditions can be appropriately defined. For example, the culturing temperature can be about 30 to 40°C, for example, at least or about 31, 32, 33, 34, 35, 36, 37, 38, 39°C but particularly not limited to them. In one embodiment, the cells are cultured at 37°C. The CO₂ concentration can be about 1 to 10%, for example, about 2 to 5%, or any range derivable therein. The oxygen tension can be at least, up to, or about 1, 2, 3, 4, 5, 6, 7, 8, 9, 10, 20%, or any range derivable therein.

### 2. Differentiation Media and Step-Wise Culture

### a. Retinal Induction Medium

After the PSCs, such as ES cells or iPSC, have adhered to the culture plate, the cells can be cultured in Retinal Induction Medium to start the differentiation process into RPE progenitor cells. The Retinal Induction Medium (RIM) includes a WNT pathway inhibitor and can result in the differentiation of PSCs to retinal lineage cells. The RIM additionally includes a TGFβ pathway inhibitor and a BMP pathway inhibitor. In some embodiments, the RIM includes a WNT pathway inhibitor, a TGFβ pathway inhibitor, a BMP pathway inhibitor and insulin growth factor 1 (IGF1).

The RIM can include DMEM and F12 at about a 1:1 ratio. In exemplary methods, a WNT pathway inhibitor is included in the RIM, such as CKI-7, a BMP pathway inhibitor is included, such as LDN193189, and the TGFβ pathway inhibitor is included, such as SB431542. For example, the RIM comprises about 5 nM to about 50 nM, such as about 10 nM, of LDN193189, about 0.1 µM to about 5 µM, such as about 0.5 µM, of CKI-7, and about 0.5 µM to about 10 µM, such as about 1 µM, of SB431542. Additionally, the RIM can include knockout serum replacement, such as about 1% to about 5%, MEM non-essential amino acids (NEAA), sodium pyruvate, N-2 supplement, B-27 supplement, ascorbic acid, and insulin growth factor 1 (IGF1). In some embodiments, the IGF1 is animal free IGF1 (AF-IGF1) and is included in the RIM at a concentration of about 0.1 ng/mL to about 10 ng/mL, such as about 1 ng/mL. The media can be aspirated each day and replaced with fresh RIM. The cells are generally cultured in the RIM for about 1 to about 5 days, such as about 1, 2, 3, 4 or 5 days, such as for about 2 days to produce RPE progenitor cells.

### b. Retinal Differentiation Medium

The resulting RPE progenitor cells can subsequently be cultured in Retinal Differentiation Medium (RDM) for further differentiation into committed RPE cells. The RDM includes a WNT pathway inhibitor, a BMP pathway inhibitor, a TGFβ pathway inhibitor and a MEK inhibitor. The RDM can include a WNT pathway inhibitor, a TGFβ pathway inhibitor, a BMP pathway inhibitor, a MEK inhibitor and IGF1.

In one embodiment, the RDM comprises a WNT pathway inhibitor, such as CKI-7, a BMP pathway inhibitor, such as LDN193189, a TGFβ pathway inhibitor, such as SB431542, and a MEK inhibitor, such as PD032590. Alternatively, the RDM can comprise a WNT pathway inhibitor, a BMP pathway inhibitor, a TGFβ pathway inhibitor and a bFGF inhibitor. Generally, the concentrations of the Wnt pathway inhibitor, BMP pathway inhibitor and TGFβ pathway inhibitor are higher in the RDM as compared to the RIM, such as about 9 to about 11 times higher, such as about 10 times higher. In exemplary methods, the RDM includes about 50 nM to about 200 nM, such as about 100 nM of LDN193189, about 1 µM to about 10 µM, such as about 5 µM, of CKI-7, about 1 µM to about 50 µM, such as about 10 µM, of SB431542, and about 0.1 µM to about 10 µM, such as about 1 µM, 2 µM, 3 µM, 4 µM, 5 µM, 6 µM, 7 µM, 8 µM, or 9 µM of PD0325901.

Generally, the RDM includes DMEM and F12 at about a 1:1 ratio, knockout serum replacement (e.g., about 1% to about 5%, such as about 1.5%), MEM NEAA, sodium pyruvate, N-2 supplement, B-27 supplement, ascorbic acid and IGF1 (e.g., about 1 ng/mL to about 50 ng/mL, such as about 10 ng/mL). In particular methods, the cells are given fresh RDM each day after aspiration of the media from the previous day. Generally, the cells are cultured in the RDM for about 2, 3, 4, 5, 6, 7, 8, 9, 10, 11, 12, 13, 14, 15, or 16 days, such as for about 7 days to derive committed RPE cells.

### c. Retinal Medium

The resulting committed RPE cells are further differentiated by subsequent culturing in Retinal Medium (RM) to form immature RPE cells. The Retinal Medium includes activin A, and can additionally include nicotinamide. The RM can include about 50 to about 200 ng/mL, such as about 100 ng/mL, of activin A, and about 1 mM to about 50 mM, such as about 10 mM, of nicotinamide. Alternatively, the RM can include other TGF-β pathway activators such as GDF1 and/or WNT pathway activators such as WAY-316606, IQ1, QS11, SB-216763, BIO (6-bromoindirubin-3'-oxime), or 2-amino-4-[3,4-(methylenedioxy)benzyl-amino]-6-(3-methoxyphenyl) pyrimidine. In some examples, the RM additionally includes WNT3a.

The RM can include DMEM and F12 at about a 1:1 ratio, knockout serum replacement at about 1% to about 5%, such as about 1.5%, MEM non-essential amino acids (NEAA), sodium pyruvate, N-2 supplement, B-27 supplement, and ascorbic acid. The medium can be changed daily with room temperature RM. The cells are generally cultured in the RM for about 8, 9, 10, 11, 12, 13, 14, 15, 16 or 17 days, such as for about 10 days to derive or produce immature RPE cells.

### d. RPE-Maturation Medium

For further differentiation of immature RPE cells, the cells are subsequently cultured in RPE Maturation Medium (RPE-MM) to form macular, central or peripheral RPE cells. The RPE-Maturation Medium can include about 100 µg/mL to about 300 µg/mL, such as about 250 µg/mL, of taurine, about 10 µg/L to about 30 µg/L, such as about 20 µg/L, of hydrocortisone and about 0.001 µg/L to about 0.1 µg/L, such as about 0.013 µg/L, of triiodothyronine. In some examples, the RPE-MM includes MEM Alpha, N-2 supplement, MEM non-essential amino acids (NEAA), sodium pyruvate, and fetal bovine serum (e.g., about 0.5% to about 10%, such as about 1% to about 5%).

The medium can be changed every other day with room temperature RPE-MM. The RPE-MM can include a RAR antagonist, a canonical Wnt inhibitor, or both. Exemplary RAR antagonists include AGN 193109, CE 2665, ER 5081, LE 135, LY 2955303, MM 11253, and liarozole dihydrochloride. In some non-limiting examples, the RAR antagonist is AGN 193109. Exemplary canonical Wnt inhibitors include 4-(1,3,3a,4,7,7a-Hexahydro-1,3-dioxo-4,7-methano-2H-isoindol-2-yl)-N-8-quinolinyl-Benzamide (Endo-1-IWR), Calphostin C, Cardionogen 1, CCT 031374 hydrobromide, IWP 12, XAV 939, WIKI4, ICG-001, Wnt-C59 (C59), IWR-1-endo, KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, and TC-E 5001. In some non-limiting examples, the canonical Wnt inhibitor is Endo-1-IWR.

The immature cells are generally cultured in RPE-MM for about 5 to about 10 days, such as about 5 days. The cells can then be dissociated, such as with a cell dissociation enzyme, reseeded, and cultured for an additional period of time, such as an additional about 5 to about 50 days, about 5 to about 40 days, or about 5 to about 30 days. The cells can be cultured, for example, for about 15 to about 20 days, about 15 to about 25 days, or about 20 to about 25 days, or about 20 to about 30 days, about 30 to about 40 days, about 30 to about 50 days, or about 40 to about 50 days, for further differentiation into RPE cells in the presence of the RAR antagonist, the Wnt inhibitor, or both. In specific non-limiting examples, to produce macular, central, or peripheral RPE cells, cells are cultured in RPE-MM in the presence of the RAR antagonist, the canonical Wnt inhibitor, or both, for about 15 to about 50 days, such as bout 20 to about 50 days, about 30 to about 50 days, or about 40 to about 50 days.

### i. Production of macular RPE Cells

In some embodiments, the method produces macular RPE cells, and the RPE-MM includes a RAR antagonist but does not include a canonical Wnt inhibitor. The macular RPE cells can be human macular RPE cells. Exemplary RAR antagonists that can be present in the RPE-MM include one or more of AGN 193109, CE 2665, ER 5081, LE 135, LY 2955303, MM 11253, and liarozole dihydrochloride.

In some non-limiting examples, the RAR antagonist in the RPE-MM is AGN 193109. The RPE-MM can include about 0.05 to about 0.4 µM of AGN 193109, such as about 0.1 to about 0.2 µM of AGN 193109. The RPE-MM can include for example, about 0.05 to about 0.3 µM, about 0.05 to about 0.2 µM, or about 0.05 to about 0.1 µM of AGN 193109. The RPE-MM can include, for example, about 0.05 to about 0.4 µM, about 0.1 to about 0.2 µM, about 0.15 to about 0.4 µM, about 0.2 to about 0.4 µM, about 0.25 to about 0.4 µM, about 0.3 to about 0.4 µM, or about 0.35 to about 0.4 µM of AGN 193109. The RPE-MM can include, for example, about 0.1 µM, about 0.15, or about 0.2 µM of AGN 193109.

The immature RPE cells are generally cultured in RPE-MM containing a RAR antagonist but not a canonical Wnt inhibitor for about 5 to about 50 days, such as about 5 to about 25 days, such as about 5 to about 10 days, such as about 5 days. The cells can then be dissociated, such as with a cell dissociation enzyme, reseeded, and cultured for an additional period of time, such as an additional about 5 to about 50 days, such as about 5 to about 40 days, for example about 5 to about 30 days, such as about 15 to about 20 days, about 15 to about 25 days, or about 20 to about 25 days, about 20 to about 30 days, about 30 to about 40 days, about 40 to about 50 days, or about 5, 10, 15, 20, 25, 30, 35, 40, 45 or 50 days, for further differentiation into central RPE cells in the presence of the RAR antagonist. In specific non-limiting examples, to produce central RPE cells, the immature RPE cells are cultured in RPE-MM containing the RAR antagonist for about 15 to about 25 days.

In a specific non-limiting example, the RAR antagonist is AGN 193109.

### ii. Production of central RPE Cells

In other embodiments, the method produces central RPE cells, and the RPE-MM includes both a RAR antagonist and a canonical Wnt inhibitor. The central RPE cells can be human central RPE cells. Exemplary RAR antagonists that can be present in the RPE-MM include AGN 193109, CE 2665, ER 5081, LE 135, LY 2955303, MM 11253, and liarozole dihydrochloride. Exemplary canonical Wnt inhibitors that can be present in the RPE-MM include 4-(1,3,3a,4,7,7a-Hexahydro-1,3-dioxo-4,7-methano-2H-isoindol-2-yl)-N-8-quinolinyl-Benzamide (Endo-1-IWR), Calphostin C, Cardionogen 1, CCT 031374 hydrobromide, IWP 12, XAV 939, WIKI4, ICG-001, Wnt-C59 (C59), IWR-1-endo, KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, and TC-E 5001.

In some non-limiting examples, the RAR antagonist is AGN 193109 and the canonical Wnt inhibitor is ENDO1-1WR. The RPE-MM can include, for example, about 10 nM to about 50 nM of AGN 193109 and about 0.025 to about 0.5 µM Endo-1-IWR, such as about 25 nM to about 50 nM of AGN 193109, and about 0.1 µM to about 0.2 µM Endo-1-IWR.

The RPE-MM can include about 10 nM to about 40 nM, about 10 nM to about 30 nM, about 10 nM to about 20 nM, about 20 nM to about 50 nM, about 30 nM to about 50 nM or about 40 nM to about 50 nM of AGN 193109. The RPE-MM can include, for example, about 0.025 to about 0.4 µM, about 0.025 to about 0.3 µM, about 0.025 to about 2 µM, or about 0.025 to about 1 µM ANG 193109. The RPE-MM can include, for example, about 0.05 to about 0.5 µM, about 0.1 to about 0.5 µM, about 0.15 to about 0.5 µM, about 0.2 to about 0.5 µM, about 0.25 to about 0.5 µM, about 0.3 to about 0.5 µM, about 0.35 to about 0.5 µM, about 0.4 to about 0.5 µM, or about 0.45 to about 0.5 µM Endo-1-IWR.

The RPE-MM can include about 25 to about 45 nM, about 25 to about 40 nM, about 25 to about 35 nM, or about 25 to about 30 nM AGN 193109. The RPE-MM can include about 0.1 to about 0.125 µM, about 0.1 to about 0.15 µM, or about 0.1 to about 0.175 µM Endo-1-IWR.

The immature RPE cells are generally cultured in RPE-MM containing a RAR antagonist and a canonical Wnt inhibitor for about 5 to about 50 days, such as about 5 to about 25 days, such as about 5 to about 10 days, such as about 5 days. The cells can then be dissociated, such as with a cell dissociation enzyme, reseeded, and cultured for an additional period of time, such as an additional about 5 to about 50 days, such as about 5 to about 40 days, for example about 5 to about 30 days, such as about 15 to about 20 days, about 15 to about 25 days, or about 20 to about 25 days, about 20 to about 30 days, about 30 to about 40 days, about 40 to about 50 days, or about 5, 10, 15, 20, 25, 30, 35, 40, 45 or 50 days, for further differentiation into central RPE cells in the presence of the RAR antagonist and the canonical Wnt inhibitor. In specific non-limiting examples, to produce central RPE cells, the immature RPE cells are cultured in RPE-MM containing the RAR antagonist and the canonical Wnt inhibitor for about 15 to about 25 days.

### iii. Production of peripheral RPE cells

In further embodiments, the method produces peripheral RPE cells, and the RPE maturation medium includes the canonical Wnt inhibitor but not a RAR antagonist. The peripheral RPE cells can be human peripheral RPE cells.

Exemplary canonical Wnt inhibitors that can be present in the RRPE MM include 4-(1,3,3a,4,7,7a-Hexahydro-1,3-dioxo-4,7-methano-2H-isoindol-2-yl)-N-8-quinolinyl-Benzamide (Endo-1-IWR), Calphostin C, Cardionogen 1, CCT 031374 hydrobromide, IWP 12, XAV 939, WIKI4, ICG-001, Wnt-C59 (C59), IWR-1-endo, KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, and TC-E 5001. In a non-limiting example, the canonical Wnt inhibitor present in the RRPE MM is Endo-1-IWR.

In some non-limiting examples, the RPE-MM includes about 0.5 to about 8 µM Endo-1-IWR, such as about 1 to about 4 µM Endo-1-IWR. The RPE-MM can include, for example, about 0.5 to about 7 µM, about 0.5 to about 6 µM, about 0.5 to about 5 µM, about 0.05 to about 4 µM, about 0.05 to about 3 µM, about 0.05 to about 2 µM, or about 0.05 to about 1 µM Endo-1-IWR. The RPE-MM can include, for example, about 1 to about 2 µM or about 1 to about 3 µM Endo-1-IWR.

The immature RPE cells are generally cultured in RPE-MM containing the canonical Wnt inhibitor but no RAR antagonist for about 5 to about 50 days, such as about 5 to about 25 days, such as about 5 to about 10 days, such as about 5 days. The cells can then be dissociated, such as with a cell dissociation enzyme, reseeded, and cultured for an additional period of time, such as an additional about 5 to about 50 days, such as about 5 to about 40 days, for example about 5 to about 30 days, such as about 15 to about 20 days, about 15 to about 25 days, or about 20 to about 25 days, about 20 to about 30 days, about 30 to about 40 days, about 40 to about 50 days, or about 5, 10, 15, 20, 25, 30, 35, 40, 45 or 50 days, for further differentiation into central RPE cells in the presence of the canonical Wnt inhibitor. In specific non-limiting examples, to produce central RPE cells, the immature RPE cells are cultured in RPE-MM containing the canonical Wnt inhibitor for about 15 to about 25 days.

### 3. Cryopreservation

The macular, central, and/or peripheral RPE cells produced by the methods disclosed herein can be cryopreserved, see for example, PCT Publication No. 2012/149484 A2. The cells can be cryopreserved with or without a substrate. In several embodiments, the storage temperature ranges from about -50°C to about - 60°C, about -60°C to about -70°C, about -70°C to about -80°C, about -80°C to about -90°C, about - 90°C to about - 100°C, and overlapping ranges thereof. In some embodiments, lower temperatures are used for the storage (e.g., maintenance) of the cryopreserved cells. In several embodiments, liquid nitrogen (or other similar liquid coolant) is used to store the cells. In further embodiments, the cells are stored for greater than about 6 hours. In additional embodiments, the cells are stored about 72 hours. In several embodiments, the cells are stored 48 hours to about one week. In yet other embodiments, the cells are stored for about 1, 2, 3, 4, 5, 6, 7, or 8 weeks. In further embodiments, the cells are stored for 1, 2, 3, 4, 5, 67, 8, 9, 10, 11 or 12 months. The cells can also be stored for longer times. The cells can be cryopreserved separately or on a substrate, such as any of the substrates disclosed herein.

In some embodiments, additional cryoprotectants can be used. For example, the cells can be cryopreserved in a cryopreservation solution comprising one or more cryoprotectants, such as DM80, serum albumin, such as human or bovine serum albumin. The cryoprotectant can intercalate into the cell membrane and change the properties of the cells so that it survives freezing. Central, peripheral, and macular RPE cells can be cryopreserved as isolated populations, or can be mixed in a ratio of interest prior to cryopreservation.

In certain embodiments, the solution includes about 1%, about 1.5%, about 2%, about 2.5%, about 3%, about 4%, about 5%, about 6%, about 7%·, about 8%, about 9%, or about 10% DMSO. In other embodiments, the solution includes about 1% to about 3%, about 2% to about 4%, about 3% to about 5%, about 4% to about 6%, about 5% to about 7%, about 6% to about 8%, about 7% to about 9%, or about 8%· to about 10% dimethylsulfoxide (DMSO) or albumin. In a specific embodiment, the solution includes 2.5% DMSO. In another specific embodiment, the solution includes 10% DMSO.

Cells may be cooled, for example, at about 1° C minute during cryopreservation. In some embodiments, the cryopreservation temperature is about -80° C to about -180° C, or about -125° C to about -140° C. In some embodiments, the cells are cooled to 4 °C prior to cooling at about 1 °C/minute. Cryopreserved cells can be transferred to vapor phase of liquid nitrogen prior to thawing for use. In some embodiments, for example, once the cells have reached about -80° C, they are transferred to a liquid nitrogen storage area. Cryopreservation can also be done using a controlled-rate freezer. Cryopreserved cells may be thawed, e.g., at a temperature of about 25° C to about 40° C, such as at a temperature of about 37° C. The cells can then be matured on a scaffold, as discussed below.

### 4. Maturation of RPE Cells on a Solid Support

RPE cells can be cultured in RPE-MM (such as one containing a canonical Wnt inhibitor, RAR antagonist, or both) for a continued period of time for maturation, for example in or on a solid support, such as a scaffold provided herein containing PLGA ad PCL loops. In some embodiments, RPE cells are grown in wells (such as with a plastic non-permeable or semi-permeable surface), such as a 6-well, 12-well, 24-well, or 10 cm plate. In some embodiments, the RPE cells are grown and matured on a scaffold disclosed herein, such as a biodegradable scaffold composed of a PLGA layer and a layer of PCL loops attached to a top surface of the PLGA layer. RPE cells can be maintained in RPE-MM on a scaffold for about two to about ten weeks, such as for about three to four weeks, about six to eight weeks, such as for two, three, four, five, six, seven, or eight weeks. In some non-limiting examples, the RPE cells are cultured in a medium on a scaffold for about two to six weeks, such as about five weeks, to obtain mature and functional macular, central and/or peripheral RPE cell monolayers. This culturing produces polarized macular, central or peripheral RPE cells on the scaffold, which together form the tissue implant.

In some embodiments, the scaffold is cultured for a sufficient time such that the bulk of lactic acid release from the scaffold occurs in vitro. In some embodiments, greater than 50%, 60%, 70%, 80%, 90% or 95% of the lactic acid release occurs in vitro. Thus, in some embodiments, maintaining the RPE in RPE-MM on the scaffold for about two to about ten weeks, such as for about six to eight weeks or 3 to 5 weeks, such as for three, four, five, six, seven, or eight weeks achieves this effect. In some non-limiting examples, culturing the RPE cells in a medium on a scaffold for about two to six weeks, such as about three, four or five weeks achieves this effect.

The scaffold can be sterilized prior to seeding RPE cells, PRP cells, or both, (such as macular, central, and/or peripheral RPE cells) on the scaffold (*e.g.,* using gamma irradiation, an electron beam (ebeam)).

In some embodiments, pluripotent stem cells, RPE progenitor cells, committed RPE cells, r immature RPE cells, are seeded onto a scaffold at about 125,000 to about 500,000 cells per 12 mm diameter, such as about 150,000 cells per 12 mm diameter, about 200,000 cells per 12 mm diameter, about 250,000 cells per 12 mm diameter, about 300,000 cells per 12 mm diameter, about 350,000 cells per 12 mm diameter of PLGA scaffold, about 400,000 cells per 12 mm diameter, or about 450,000 cells per 12 mm diameter of scaffold (such as a PLGA scaffold).

In some embodiments, mature macular, central or peripheral RPE cells are developed into monolayers that behave as intact macular, central or peripheral RPE tissue by continued culture in the RPE-MM (such as one containing a canonical Wnt inhibitor, RAR antagonist, or both) on the scaffold. Additional small molecules can be included in the RPE-MM (such as one containing a canonical Wnt inhibitor, RAR antagonist, or both). In some embodiments, these small molecules are primary cilium inducers such as prostaglandin E2 (PGE2) or aphidicolin. The PGE2 can be added to the RPE-MM at a concentration of about 25 µM to about 250 µM, such as about 50 µM to about 100 µM.

In some embodiments, for the continued maturation of the RPE cells, immature RPE cells can be dissociated in a cell dissociated enzyme such as TRYPLE^{™} and reseeded onto the scaffold, such as in a specialized SNAPWELL^{™} design, for at least about one to two weeks in RPE-MM (such as one containing a canonical Wnt inhibitor, RAR antagonist, or both) with a MEK inhibitor such as PD0325901. Alternatively, the RPE-MM (such as one containing a canonical Wnt inhibitor, RAR antagonist, or both) can include a bFGF inhibitor instead of the MEK inhibitor. Suitable methods for culturing RPE cells on the degradable scaffold are taught and described in PCT Publication No. WO 2014/121077 and PCT Publication No. WO2020/106622. Briefly, the main components of this method are a CORNING^{®} COSTAR^{®} SNAPWELL^{™} plate, a bioinert 0-ring, and a biodegradable scaffold, such as any of the PLGA scaffold disclosed above. SNAPWELL^{™} plates provide the structure and platform for biodegradable scaffolds. The microporous membrane that creates an apical and basal side provides support to the scaffold as well as isolating the distinct sides of the polarized layer of cells. The ability of the SNAPWELL^{™} insert to detach the membrane allows the support ring of the insert to be used an anchor for the scaffold (see below). The resulting monolayers of macular, central or peripheral RPE cells on the scaffold can then be isolated and used as the tissue replacement implant.

In yet other embodiments the resulting mature macular, central or peripheral RPE cells on the scaffold have a resting potential of about -50 to about -60 mV, and a fluid transport rate of about 5 to about 10 µl cm⁻²h⁻¹. In additional embodiments, the macular, central or peripheral RPE cells express MITF, PAX6, LHX2, TFEC, CDH1, CDH3, CLDN10, CLDN16, CLDN19, BEST1, TIMP3, TRPM1, TRPM3, TTR, VEGFA, CSPG5, DCT, TYRP1, TYR, SILV, SIL1, MLANA, RAB27A, OCA2, GPR143, GPNMB, MYO6, MYRIP, RPE65, RBP1, RBP4, RDH5, RDH11, RLBP1, MERTK, ALDH1A3, FBLN1, SLC16A1, KCNV2, KCNJ13, and CFTR, express miR204 and miR211, have a resting potential of about -50 to about -60 mV and have a fluid transport rate of about 5 to about 10 µl cm⁻²h⁻¹. In other embodiments, the RPE cells have a transepitelial resistance of greater than 100Ω*cm², such as greater than 200Ω*cm². In further embodiments, the macular, central or peripheral RPE cells have a transepitelial resistance of 100Ω*cm² to 500Ω*cm², such as a transepitelial resistance of 200Ω*cm² to 400Ω*cm².

In one non-limiting example, a method is provided that includes: a) providing a scaffold provided herein that includes a PLGA first layer and a PCL loop second layer attached thereto, which is coated with vitronectin, wherein the PLGA portion of the scaffold includes fibers that form a mesh structure and wherein the PLGA portion of the scaffold has an upper surface and a lower surface, wherein the PLGA scaffold is about 5 to about 40 microns in thickness (e.g., height), has a DL-lactide/glycolide ratio of about 1:1, an average pore size of less than about 1 microns, and a fiber diameter of about 150 to about 650 nm and wherein the PCL loops have a diameter of about 5 to about 300 microns; b) seeding pluripotent stem cells, RPE progenitor cells, committed RPE cells, or immature RPE cells onto the scaffold at about 100,000 to about 500,000 cells per cm diameter of scaffold; c) culturing the i cells on the scaffold in a tissue culture medium *in vitro,* with medium present on both the upper surface and the lower surface of the scaffold, for a time that is sufficient for i) polarization of the RPE cells and ii) bulk degradation of the scaffold; d) seeding PRP cells onto the layer of mature macular, central or peripheral RPE generated from the immature RPE previously seeded onto the scaffold at about 1,000,000 to about 6,000,000 PRP cells per cm diameter of scaffold; and e) culturing the mature macular, central and/or peripheral RPE cells and PRP cells on the scaffold in a tissue culture medium *in vitro,* with medium present on both the upper surface and the lower surface of the scaffold, for a time that is sufficient for TEER>400 Ohms.cm2 and presence of photoereceptor markers (e.g., recoverin, arrestin, NRL, opsins).

### 5. Inhibitors of Use in Differentiation of Peripheral, Central and Macular RPE Cells

Disclosed below are inhibitors that are of use in preparing RPE cells. These inhibitors can be included in the retinal induction medium, retinal differentiation medium, retinal medium, and/or RPE maturation medium, as described above.

### a. WNT Pathway Inhibitors

WNT is a family of highly conserved secreted signaling molecules that regulate cell-to-cell interactions and are related to the *Drosophila* segment polarity gene, wingless. In humans, the WNT family of genes encodes 38 to 43 kDa cysteine rich glycoproteins. The WNT proteins have a hydrophobic signal sequence, a conserved asparagine-linked oligosaccharide consensus sequence (see e.g., Shimizu et al., Cell Growth Differ 8: 1349-1358 (1997)) and 22 conserved cysteine residues. Because of their ability to promote stabilization of cytoplasmic beta-catenin, WNT proteins can act as transcriptional activators and inhibit apoptosis. Overexpression of particular WNT proteins has been shown to be associated with certain cancers.

A WNT inhibitor herein refers to WNT inhibitors in general. Thus, a WNT inhibitor refers to any inhibitor of a member of the WNT family proteins including Wnt1, Wnt2, Wnt2b, Wnt3, Wnt4, Wnt5A, Wnt6, Wnt7A, Wnt7B, Wnt8A, Wnt9A, Wnt10a, Wnt11, and Wnt16. Certain embodiments of the present methods concern a WNT inhibitor in the differentiation medium. Examples of suitable WNT inhibitors, already known in the art, include N-(2-Aminoethyl)-5-chloroisoquinoline-8-sulphonamide dihydrochloride (CKI-7), N-(6-Methyl-2-benzothiazolyl)-2-[(3,4,6,7-tetrahydro-4-oxo-3-phenylthieno[3,2-d]pyrimidin-2-yl)thio]-acetamide (IWP2), N-(6-Methyl-2-benzothiazolyl)-2-[(3,4,6,7-tetrahydro-3-(2-methoxyphenyl)-4-oxothieno[3,2-d]pyrimidin-2-yl)thio]-acetamide (IWP4), 2-Phenoxybenzoic acid-[(5-methyl-2-furanyl)methylene]hydrazide (PNU 74654) 2,4-diamino-quinazoline, quercetin, 3,5,7,8-Tetrahydro-2-[4-(trifluoromethyl)phenyl]-4H-thiopyrano[4,3-d]pyrimidin-4-one (XAV939), 2,5-Dichloro-N-(2-methyl-4-nitrophenyl)benzenesulfonamide (FH 535), N-[4-[2-Ethyl-4-(3-methylphenyl)-5-thiazolyl]-2-pyridinyl]benzamide (TAK 715), Dickkopf-related protein one (DKK1), and Secreted frizzled-related protein (SFRP1) 1. In addition, inhibitors of WNT can include antibodies to, dominant negative variants of, and siRNA and antisense nucleic acids that suppress expression of WNT. Inhibition of WNT can also be achieved using RNA-mediated interference (RNAi). Exemplary Wnt inhibitors of use are listed in Table 5.

### b. BMP Pathway Inhibitors

Bone morphogenic proteins (BMPs) are multi-functional growth factors that belong to the transforming growth factor beta (TGFβ) superfamily. BMPs are considered to constitute a group of pivotal morphogenetic signals, orchestrating architecture through the body. The important functioning of BMP signals in physiology is emphasized by the multitude of roles for dysregulated BMP signaling in pathological processes.

BMP pathway inhibitors may include inhibitors of BMP signaling in general or inhibitors specific for BMP1, BMP2, BMP3, BMP4, BMP5, BMP6, BMP7, BMP8a, BMP8b, BMP10 or BMP15. Exemplary BMP inhibitors include 4-(6-(4-(piperazin-1-yl)phenyl)pyrazolo[1,5-a]pyrimidin-3-yl)quinoline hydrochloride (LDN193189), 6-[4-[2-(1-Piperidinyl)ethoxy]phenyl]-3-(4-pyridinyl)-pyrazolo[1,5-a]pyrimidine dihydrochloride (Dorsomorphin), 4-[6-[4-(1-Methylethoxy)phenyl]pyrazolo[1,5-a]pyrimidin-3-yl]-quinoline (DMH1), 4-[6-[4-[2-(4-Morpholinyl)ethoxy]phenyl]pyrazolo[1,5-a]pyrimidin-3-yl]quinoline (DMH-2), and 5-[6-(4-Methoxyphenyl)pyrazolo[1,5-a]pyrimidin-3-yl]quinoline (ML 347).

### c. TGFβ Pathway Inhibitors

Transforming growth factor beta (TGFβ) is a secreted protein that controls proliferation, cellular differentiation, and other functions in most cells. It is a type of cytokine which plays a role in immunity, cancer, bronchial asthma, lung fibrosis, heart disease, diabetes, and multiple sclerosis. TGF-β exists in at least three isoforms called TGF-β1, TGF-β2 and TGF-β3. The TGF-β family is part of a superfamily of proteins known as the transforming growth factor beta superfamily, which includes inhibin, activin, anti-müllerian hormone, bone morphogenetic protein, decapentaplegic and Vg-1.

TGFβ pathway inhibitors may include any inhibitors of TGFβ signaling in general. For example, the TGFβ pathway inhibitor is 4-[4-(1,3-benzodioxol-5-yl)-5-(2-pyridinyl)-1H-imidazol-2-yl]benzamide (SB431542), 6-[2-(1,1-Dimethylethyl)-5-(6-methyl-2-pyridinyl)-1H-imidazol-4-yl]quinoxaline (SB525334), 2-(5- Benzo[1,3]dioxol-5-yl-2-ieri-butyl-3H-imidazol-4-yl)-6-methylpyridine hydrochloride hydrate (SB- 505124), 4-(5-Benzol[1,3]dioxol- 5-yl-4-pyridin-2-yl-1H-imidazol-2-yl)-benzamide hydrate, 4-[4-(1,3-Benzodioxol-5-yl)-5-(2- pyridinyl)-1H-imidazol-2-yl]-benzamide hydrate, left-right determination factor (Lefty), 3-(6-Methyl-2-pyridinyl)-N-phenyl-4-(4-quinolinyl)-1H-pyrazole-1-carbothioamide (A 83-01), 4-[4-(2,3-Dihydro-1,4-benzodioxin-6-yl)-5-(2-pyridinyl)-1H-imidazol-2-yl]benzamide (D 4476), 4-[4-[3-(2-Pyridinyl)-1H-pyrazol-4-yl]-2-pyridinyl]-N-(tetrahydro-2H-pyran-4-yl)-benzamide (GW 788388), 4-[3-(2-Pyridinyl)-1H-pyrazol-4-yl]-quinoline (LY 364847), 4-[2-Fluoro-5-[3-(6-methyl-2-pyridinyl)-1H-pyrazol-4-yl]phenyl]-1H-pyrazole-1-ethanol (R 268712) or 2-(3-(6-Methylpyridine-2-yl)-1H-pyrazol-4-yl)-1,5-naphthyridine (RepSox).

### d. MEK Inhibitors

A MEK inhibitor is a chemical or drug that inhibits the mitogen-activated protein kinase enzymes MEK1 or MEK2. MEK inhibitors can be used to affect the MAPK/ERK pathway. For example, MEK inhibitors include N-[(2R)-2,3-Dihydroxypropoxy]-3,4-difluoro-2-[(2-fluoro-4-iodophenyl)amino]- benzamide (PD0325901), N-[3-[3-cyclopropyl-5-(2-fluoro-4-iodoanilino)-6,8-dimethyl-2,4,7-trioxopyrido[4,3-d]pyrimidin-1-yl]phenyl]acetamide (GSK1120212), 6-(4-bromo-2-fluoroanilino)-7-fluoro-N-(2-hydroxyethoxy)-3-methylbenzimidazole-5-carboxamide (MEK162), N-[3,4-difluoro-2-(2-fluoro-4-iodoanilino)-6-methoxyphenyl]-1-(2,3-dihydroxypropyl)cyclopropane-l-sulfonamide (RDEA119), and 6-(4-bromo-2-chloroanilino)-7-fluoro-N-(2-hydroxyethoxy)-3-methylbenzimidazole-5-carboxamide (AZD6244).

### e. bFGF Inhibitors

Basic fibroblast growth factor (also known as bFGF, FGF2 or FGF-β) is a member of the fibroblast growth factor family. bFGF is present in basement membranes and in the subendothelial extracellular matrix of blood vessels. In addition, bFGF is a common component of human ESC culture medium in which it is necessary for the cells to remain in an undifferentiated state.

A bFGF inhibitor refers to bFGF inhibitors in general. For example, bFGF inhibitors include, but are not limited to N-[2-[[4-(Diethylamino)butyl]amino-6-(3,5-dimethoxyphenyl)pyrido[2,3-d]pyrimidin-7-yl]-N'-(1,1-dimethylethyl)urea (PD173074), 2-(2-Amino-3-methoxyphenyl)-4H-1-benzopyran-4-one (PD 98059), 1-tert-Butyl-3-[6-(2,6-dichlorophenyl)-2-[[4-(diethylamino)butyl]amino]pyrido[2,3-d]pyrimidin-7-yl]urea (PD161570), 6-(2,6-Dichlorophenyl)-2-[[4-[2-(diethylamino)ethoxy]phenyl]amino]-8-methyl-pyrido[2,3-d]pyrimidin-7(8H)-one dihydrochloride hydrate (PD166285), N-[2-Amino-6-(3,5-dimethoxyphenyl)pyrido[2,3-d]pyrimidin-7-yl]-N'-(1,1-dimethylethyl)-urea (PD166866), and MK-2206.

### f. RAR Antagonists

RAR antagonists are of use in the disclosed methods. RAR antagonists of use are listed in Table 4. Exemplary RAR antagonists include AGN 193109, CE 2665, ER 5081, LE 135, LY 2955303, MM 11253, and liarozole dihydrochloride. In some non-limiting examples, the RAR antagonist is AGN 193109.

### 6. Kits

In some embodiments, a kit can include, for example, one or more media and components for the production of macular, central, and/or peripheral RPE cells. The reagent system may be packaged either in aqueous media or in lyophilized form, where appropriate. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial. The components of the kit may be provided as dried powder(s). When reagents and/or components are provided as a dry powder, the powder can be reconstituted by the addition of a suitable solvent. It is envisioned that the solvent may also be provided in another container means.

In some examples, a kit includes a retinal induction medium, such as one that includes a WNT pathway inhibitor, a transforming growth factor (TGF)-β pathway inhibitor a bone morphogenic protein (BMP) pathway inhibitor and insulin growth factor 1 (IGF1).

In some examples, a kit includes a retinal differentiation medium, such as one that includes a WNT pathway inhibitor, a TGFβ pathway inhibitor, a BMP pathway inhibitor, IGF1, and an additional inhibitor (e.g., a MEK inhibitor or a fibroblast growth factor (FGF) inhibitor).

In some examples, a kit includes retinal medium, such as one that includes activin A, and can additionally include nicotinamide.

In some examples, a kit includes RPE maturation medium, wherein the RPE maturation medium includes a retinoic acid receptor (RAR) antagonist (such as one or more of AGN 193109, CE 2665, ER 5081, LE 135, LY 2955303, MM 11253, and liarozole dihydrochloride) and/or a canonical Wnt inhibitor (such as one or more of 4-(1,3,3a,4,7,7a-Hexahydro-1,3-dioxo-4,7-methano-2H-isoindol-2-yl)-N-8-quinolinyl-Benzamide (Endo-1-IWR), Calphostin C, Cardionogen 1, CCT 031374 hydrobromide, IWP 12, XAV 939, WIKI4, ICG-001, Wnt-C59 (C59), IWR-1-endo, KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, and TC-E 5001). In one example the RAR antagonist is AGN 193109. In one example the canonical Wnt inhibitor is Endo-1-IWR. In one example, the RPE maturation medium includes a RAR antagonist (such as AGN 193109) but not the canonical Wnt inhibitor. In one example, the RPE maturation medium includes the canonical Wnt inhibitor (such as Endo1-IWR), but not the RAR antagonist. In one example, the RPE maturation medium includes both the canonical Wnt inhibitor (such as Endo1-IWR), and the RAR antagonist such as AGN 193109).

In some examples, a kit includes 1, 2, 3 or 4 of a retinal induction medium, a retinal differentiation medium, a retinal medium and a RPE maturation medium. In some examples, a kit includes a RPE maturation medium and 1, 2, or 3 of a retinal induction medium, a retinal differentiation medium, and a retinal medium. In some examples, such a kit can further include a matrix, such as one that includes at least one recombinant cellular adhesion protein (e.g., laminin, vitronectin or fibronectin). In some examples, such a kit can further include a scaffold, such as one provided herein. The kits also will typically include a container for containing the kit component(s) in close confinement for commercial sale. Such containers may include injection or blow molded plastic containers into which the desired vials are retained. In some examples, the containers are made of glass.

In some examples, a kit includes a scaffold provided herein, which may or may not include RPE cells (such as mature macular, central, and/or peripheral RPE cells), and/or PRP cells. In some examples, the kit includes a scaffold provided herein without attached RPE and PRP cells, and the kit further includes in separate containers one or more of (1) pluripotent stem cells, (2) RPE progenitor cells, (3) committed RPE cells, (4) immature RPE cells, (5) macular, central, and/or peripheral RPE cells, and (6) PRP cells. Such a kit can further include 1, 2, 3 or 4 of a retinal induction medium, a retinal differentiation medium, a retinal medium and a RPE maturation medium. In some examples, such a kit includes a RPE maturation medium and 1, 2, or 3 of a retinal induction medium, a retinal differentiation medium, and a retinal medium. In some examples such a kit further includes at least one recombinant cellular adhesion protein (*e.g.,* laminin, vitronectin or fibronectin). In some examples such a kit further includes one or more of a snap-well culture system, a (polytetrafluoroethylene (PTFE) O-ring, and a non-biodegradable porous polycarbonate membrane

The kit can also include instructions for use, such as in printed or electronic format, such as digital format. The instructions can be for producing the cells, and or introducing the cells and/or an implant including the cells into a subject.

### Methods of Use

Macular, central, and/or peripheral RPE cells are derived from iPSCs using the disclosed methods, and thus can be used to provide "personalized medicine" for patients with eye diseases. In some embodiments, cells obtained from patients, such as somatic cells or CD34+ cells, or umbilical cells, can be used to produce iPSC, which are then used to produce macular, central and/or peripheral RPE cells. In some embodiments, the macular, central and/or peripheral RPE cells (or the starting iPSC) can be genetically engineered to correct the disease-causing mutation, differentiated into macular, central and/or peripheral RPE, and engineered to form a tissue implant. This tissue replacement implant can be used to replace the endogenous degenerated RPE of the same subject. Macular, central and/or peripheral RPE cells can be included on a tissue implant. The type of RPE cell can be selected based on the disease to be treated in the subject. The implant can include a scaffold disclosed herein.

In some embodiments, iPSCs can be generated from a healthy donor or from HLA homozygous "super-donors" or "universal" donor pluripotent (iPS) cells and used to prepare the tissue implant. RPE cells can be treated *in vitro* with certain factors, such as pigment epithelium-derived factor (PEDF), transforming growth factor (TGF)-beta, and/or retinoic acid to generate an anti-inflammatory and immunosuppressive environment *in vivo.* These "superdonor" iPSC are commercially available, see the Cellular Dynamics International (for example, see globenewswire.com/news-release/2015/02/09/704392/10119161/en/Cellular-Dynamics-Manufactures-cGMP-HLA-Superdonor-Stem-Cell-Lines-to-Enable-Cell-Therapy-With-Genetic-Matching, February 15, 2015).

The subject can be a human or veterinary subject. The macular, central and/or peripheral RPE cells can be derived from a single subject, or several populations of macular, central and/or peripheral RPE cells, such as the different types of RPE, each derived from a different subject, can be used to produce an implant for treatment of a subject, such as with retinal degeneration.

As shown in FIGS. 14A-14D, different types of RPEs generated using the methods provided herein (e.g., P1 macular P2 central, and/or P3 peripheral) can be used to customize a treatment for different retinal diseases. For instance, macular RPEs can be used to support function and activity of cone photoreceptors, whereas central and peripheral RPEs can be used to support the activity of rod photoreceptors. In some examples, macular RPEs support a dense iPSC-derived choroidal vasculature when it is developed adjacent to them, whereas central and peripheral RPEs can support a relatively less dense iPSC-derived choroidal vasculature. By combining the correct type of iPSC-derived photoreceptors with the correct type of RPE and the iPSC-derived vasculature, different parts of the back of the eye can be generated (e.g. the entire macular or the entire center or entire periphery or the entire far-periphery of the eye).

Various forms of retinal degenerative disease affect different parts of the eye. For instance, choroideremia (CHM; FIG. 14A) causes peripheral RPE dysfunction in early disease stages that spreads to central and P4 RPE cells. As disease progresses corresponding photoreceptors and choroidal vasculature degenerate as well. Similarly, in disease late-onset retina degeneration (L-ORD, FIG. 14B), disease associated damage starts in central and peripheral RPE cells and eventually in late disease stages, photoreceptors and choroidal vasculature also degenerates. In AMD (FIG. 14C), initially the damage in seen in macular, P4 and P5 RPE that spreads to photoreceptor and choroidal vasculature in advanced stages of AMD.

In one example, early stages of CHM are treated using central RPEs, intermediate stages can be treated using peripheral and P4 iPSC-RPE, and advanced stages with central and peripheral RPE with corresponding rod enriched photoreceptors, for P4 iPSC-RPE with cone enriched photoreceptors, with and without the choroidal vasculature.

In one example, L-ORD is treated using central and peripheral RPE with or without corresponding photoreceptors and choroidal vasculature, depending upon the disease stage.

In one example, macular RPEs (or P4 RPEs), with or without corresponding photoreceptors and choroidal vasculature, are used to treat AMD.

In some embodiments, methods are provided for treating a subject in need thereof, that include producing macular, central or peripheral cells according to the disclosed methods and transplanting the macular, central or peripheral cells into a retina of the subject. In some embodiments, the subject has a retinal degenerative disease, retinal dysfunction, retinal degradation, retinal damage, or loss of retinal pigment epithelium. In some non-limiting examples, the retina degenerative disease is Stargardt's macular dystrophy, retinitis pigmentosa, age related macular degeneration, glaucoma, diabetic retinopathy, Leber's congenital amaurosis, acquired macular degeneration, hereditary macular degeneration, late-onset retinal degeneration, Best disease, retinal detachment, gyrate atrophy, choroideremia, pattern dystrophy. In other non-limiting examples, the retinal damage is caused by laser, inflammatory, infectious, radiation, neovascular or traumatic injury. In further non-limiting examples, the macular central and/or peripheral retinal pigment epithelial cells are introduced in a subretinal space of the eye, a vitreal space, an inner or outer retina, a retinal periphery or within a choroid. Methods for producing single and multi-layer retinal implants are disclosed for example, in PCT Publication No. WO 2018/089515.

Macular and far-peripheral RPE predominantly support function and activity of cone photoreceptors whereas central and peripheral RPE cells predominantly support the activity of rod photoreceptors. Similarly, macular RPE support a dense iPSC-derived choroidal vasculature when itis developed adjacent to them, whereas central and peripheral RPE support a relatively less dense iPSC-derived choroidal vasculature. By combining one or more specific types of iPSC-derived photoreceptors with the selected type(s) of RPE, as disclosed herein, and optionally, iPSC-derived vasculature, implants can be designed for the treatment of retinal degeneration in an area of the eye of interest.

The cells in the retina that are directly sensitive to light are the photoreceptor cells. Photoreceptors are photosensitive neurons in the outer part of the retina and can be either rods or cones. In the process of phototransduction, the photoreceptor cells convert incident light energy focused by the lens to electric signals which are then sent via the optic nerve to the brain. Vertebrates have two types of photoreceptor cells including cones and rods. Cones are adapted to detect fine detail, central and color vision and function well in bright light. Rods are responsible for peripheral and dim light vision. Neural signals from the rods and cones undergo processing by other neurons of the retina.

The retinal pigment epithelium acts as a barrier between the bloodstream and the retina and closely interacts with photoreceptors in the maintenance of visual function. The retinal pigment epithelium is composed of a single layer of hexagonal cells, that includes the macular, central and peripheral RPE cells, that are densely packed with granules of melanin that absorbs light energy that arrives to the retina. Functions of these RPE cells include: transport of nutrients such as glucose, retinol, and fatty acids from the blood to the photoreceptors; transport of water, metabolic end products, and ions from the subretinal space to the blood; absorption of light and protection against photooxidation; reisomerization of all-trans-retinol into 11-cis-retinal; phagocytosis of shed photoreceptor membranes; and secretion of various essential factors for the structural integrity of the retina.

Dysfunction, injury, and loss of RPE cells are factors of many eye diseases and disorders including age-related macular degeneration (AMD) and hereditary macular degenerations such as Best disease, and retinitis pigmentosa. Other diseases are discussed below. Damage to the retina, such as from physical injury, also requires treatment. Exemplary conditions that can be treated are shown in Fig. 14.

Pharmaceutical compositions of the macular, central, and/or peripheral. RPE cells produced by the methods disclosed herein. These compositions can include at least about 1 x 10³ RPE cells, about 1 x 10⁴ RPE cells, about 1 x 10⁵ RPE cells, about 1 x 10⁶ RPE cells, about 1 x 10⁷ RPE cells, about 1 x 10⁸ RPE cells, or about 1 x 10⁹ RPE cells. In certain embodiments, the compositions are substantially purified (with respect to non-RPE cells) preparations comprising differentiated RPE cells produced by the methods disclosed herein. The compositions can be formulated for delivery to the eye of a subject in need thereof, such as to the subretinal space of the eye, a vitreous space, an inner or outer retina, a retinal periphery, within a choroid, or through a supra-choroidal location.

Compositions are also provided that include a scaffold, such as a polymeric carrier and/or an extracellular matrix, and a therapeutically effective amount of the macular, central, and peripheral RPE cells produced by the methods disclosed herein. For example, the cells are provided as a monolayer of cells. The compositions can also include multiple layers, and can include photoreceptors and/or vascular cells. A scaffold can also be included, such as a degradable or non-biodegradable scaffold, which is physiologically acceptable, and suitable for use in in vivo applications. For example, the physiologically acceptable materials include, but are not limited to, solid materials that are absorbable and/or non-absorbable, such as small intestine submucosa (SIS), crosslinked or non-crosslinked alginate, hydrocolloid, foams, collagen gel, collagen sponge, polyglycolic acid (PGA) mesh, fleeces and bioadhesives. The scaffold can be a PLGA scaffold. These scaffolds can be delivered to the eye of a subject in need thereof.

The disclosed macular, central and peripheral RPE, and tissue replacement implants including macular, central and/or peripheral RPE are of use for treating a retinal degenerative disease, retinal or retinal pigment epithelium dysfunction, retinal degradation, retinal or retinal pigment epithelial damage, such as damage caused by light, laser, inflammatory, infectious, radiation, neovascular or traumatic injury. The disclosed macular, central and/or peripheral RPE, and tissue replacement implants including these cells, are also of use for treating loss of retinal pigment epithelium. The methods include locally administering the macular, central and/or peripheral RPE, or the tissue placement implant to the eye of the subject. A pharmaceutical composition including a therapeutically effective amount of RPE cells can be introduced into a subretinal space of the eye, a vitreous space, an inner or outer retina, a retinal periphery or within a choroid. The RPE cells also can be include on an implant.

In some embodiments, the retina degenerative disease is Stargardt's macular dystrophy, retinitis pigmentosa, age related macular degeneration, glaucoma, diabetic retinopathy, Lebers congenital amaurosis, late-onset retinal degeneration, hereditary macular or acquired retinal degeneration, Best disease, Sorsby's fundus dystrophy, retinal detachment, gyrate atrophy, batten disease, bear track dystrophy, traumatic eye injury, or choroideremia, pattern dystrophy. Additional conditions include retinal detachment, pattern dystrophy, and other dystrophies of the RPE. In a specific non-limiting example, the subject has age related macular degeneration. In certain embodiments, methods are provided for treating or preventing a condition characterized by retinal degeneration, comprising administering to a subject in need thereof the disclosed RPE cells or a tissue replacement implant including these RPE cells. The type of RPE included on the implant can be selected based on the disorder to be treated.

These methods can include selecting a subject with one or more of these conditions and administering the tissue replacement implant to treat the condition and/or ameliorate symptoms of the condition. The implant can include macular, central, and/or peripheral RPE, optionally in combination with other retinal cells, such as with photoreceptors and/or vascular cells.

Various forms of retinal degenerative disease affect different parts of the eye. For instance, choroideremia (CHM) causes peripheral RPE dysfunction in early disease stages that spreads to central and far-peripheral RPE cells. As this disease progresses, corresponding photoreceptors and choroidal vasculature degenerate as well. Implants of use in treating CHM include central RPE cells, and optionally far-peripheral RPE cells, and optionally photoreceptors and vascular cells. Similarly, in disease late-onset retina degeneration (L-ORD), disease associated damage starts in central and peripheral RPE cells and eventually in late disease stages, photoreceptors and choroidal vasculature also degenerates. Thus, for early treatment, implants of use include central and or peripheral RPE cells. In late disease stages, these same implants can be used, or an implant can also include photoreceptors and/or vascular cells. In AMD, initially the damage in seen in macular, far-peripheral and ora serrata RPE that spreads to photoreceptor and choroidal vasculature in advanced stages of AMD. Thus, for early treatment, implants of use can include macular RPE, and optionally far-peripheral and ora serrata RPE cells. In later disease stages, these same implants can be used, or an implant can include relevant photoreceptors and/or macula and periphery specific vascular cells.

These methods can include selecting a subject with one or more of these conditions and administering the macular, central and/or peripheral RPE cells, or a tissue replacement implant including the RPE cells, to treat the condition and/or ameliorate symptoms of the condition. The disclosed RPE cells can also be transplanted together (co-transplantation) with other retinal cells, such as with photoreceptors and/or vascular cells.

The disclosed methods allow development of customized cell therapy for different diseases and different stages of those diseases. For instance, at early stages of CHM an implant including central RPE can be used for treatment, whereas at intermediate stages an implant including peripheral and far-peripheral RPE can be used for treatment. In advanced stages the implant can include central and peripheral RPE with corresponding rod enriched photoreceptors. Alternatively an implant can be produced for far-peripheral RPE with cone enriched photoreceptors, with and without the choroidal vasculature. In some embodiments, an implant can be produced with central or peripheral RPE with or without corresponding photoreceptors and choroidal vasculature. The selection of cells can be made based upon the disease stage.

In other embodiments, for the treatment of AMD, implants can be produced including macular or far-peripheral RPE with or without corresponding photoreceptors and choroidal vasculature. These implants can then be used for treatment. In the early stages of AMD, it may sufficient to replace only macular RPE cells and/or other types of RPE cells, see FIG. 14. Without being bound by theory, the implanted macular RPE cells may mediate prevention of further loss of RPE cells and/or degeneration of the Bruch's membrane. At advanced stages of AMD, patients may experience loss of both RPE cells and photoreceptor cells. Thus, in some embodiments, the methods can further comprise implanting compositions comprising photoreceptor cells. Methods for transplanting a scaffold and RPE cells are disclosed, for example, in PCT Publication No. WO 2012177968 and PCT Publication No 2016/007852.

In some embodiments, the macular, central and/or peripheral RPE cells, optionally in the tissue replacement implant, are from the subject to be treated, and thus are autologous. In other embodiments, the macular, central and/or peripheral RPE cells, optionally in the tissue replacement implant. are produced from an MHC-matched donor or a universal donor. In another embodiment, the macular, central and/or peripheral RPE cells, optionally in the tissue replacement implant, are allogeneic.

The RPE cells, such as in the tissue replacement implant, can be introduced to various target sites within a subject's eye. In some embodiments, the tissue replacement implant is introduced, such as by transplantation, to the subretinal space of the eye, which is the anatomical location of the RPE (between the photoreceptor outer segments and the choroids) in mammals. Exemplary methods are disclosed, for example, in PCT Publication No. WO 2018/089521. In some embodiments, the tissue replacement implant is introduced in the outer retina, retinal periphery, macula, or peri-macular regions, or within a choroid. In addition, dependent upon migratory ability and/or positive paracrine effects of the cells, introduction into additional ocular compartments can be considered, such as the vitreous space, the inner or outer retina, the retinal periphery and within the choroids.

The size of the tissue replacement implant to be transplanted may be generally determined by comparing the clinical assessment of the size of the region of retinal pathology present in a particular patient, with the constraints imposed by surgical feasibility of delivering an implant of a particular size. For example, in degenerations involving the central retina (e.g., age-related macular degeneration), a circular implant of from about 1.0-2.5 mm diameter (e.g., of about 1.5 mm diameter) that approximates the anatomic fovea will frequently be appropriate. In some cases, larger implants may be appropriate, maximally corresponding to the area of posterior retina lying between the temporal vascular arcades (histologic macula, clinical posterior pole) which is an ovoid area of approximately 6.0 mm (vertical) x 7.5 mm (horizontal) centered on the fovea or positioned in the extra-foveal region. In some instances, it may likewise be appropriate to fashion a polymer scaffold of smaller dimension, as small as about 0.5 mm, to be placed in an area of circumscribed pathology. In addition, it may be of interest to custom fashion implants of irregular shape to suit the patient, for instance to cover areas of pathology while avoiding areas of residual high vision.

The tissue replacement implant can be introduced by various techniques known in the art. Methods for performing transplants are disclosed in, for example, in U.S. Patent No. 5,962,027, U.S. Patent No. 6,045,791, and U.S. Patent No. 5,941,250; Biochem Biophys Res Commun Feb. 24, 2000; 268(3): 842-6; and Opthalmic Surg February 1991; 22(2): 102-8). Methods for performing corneal transplants are described in, for example, U.S. Patent No. 5,755,785; Curr Opin Opthalmol August 1992; 3 (4): 473-81; Ophthalmic Surg Lasers April 1998; 29 (4): 305-8; and Opthalmology April 2000; 107 (4): 719-24. In some embodiments, transplantation is performed via pars pana vitrectomy surgery followed by delivery of the tissue replacement implant through a small retinal opening into the sub-retinal space. Alternatively, the tissue replacement implant can be delivered into the subretinal space via a trans-scleral, trans-choroidal, or supra-choroidal approach. In addition, direct trans-scleral insertion to the anterior retinal periphery in proximity to the ciliary body can be performed.

In some embodiments, the methods include administering an immunosuppressive agent that reduces an immune response, for example, by downregulating the response of inflammatory cells or by inducing apoptosis of inflammatory cells. In other embodiments, the method includes administering a therapeutically effective amount of a neuroprotective agent that promotes survival and/or reduces degeneration of retinal neurons. In yet other embodiments, the method can include administering a therapeutically effective amount of an agent to inhibit unwanted angiogenesis, for example, to counteract the choroidal new vessel (CNV) growth under the fovea in AMD patients. An exemplary therapeutic agent can reduce activity of vascular endothelial growth factor (VEGF), for example, by binding to the receptor site of active forms of VEGF and preventing interaction of VEGF with its receptors. A therapeutically effective amount of and that suppresses the expression of VEGF by inhibiting pathways leading to VEGF secretion, such as STAT3, NF-kB, HIF-1α. Other drugs can prevent atrophy of RPE cells by targeting complement pathway, autophagy, or NF-kB pathways. Treatments of use for AMD include medications directed to stopping the growth of new blood vessels, such as bevacizumab (AVASTIN^{®}), ranibizumab (LUCENTIS^{®}), and aflibercept (EYLEA^{®}); photodynamic therapy; photocoagulation; and low vision rehabilitation

In further embodiment, the method includes administering to the subject a therapeutically effective amount of Ciliary Neurotrophic Factor (CNTF), Brain-Derived Neurotrophic Factor (BDNF), or Pigment Epithelial Derived Factor (PEDF), which can be used, for example, to promote development or function of neurons such as photoreceptor cells. Other exemplary, non-limiting embodiments include administering to the subject a therapeutically effective amount of thrombospondin 1, an anti-inflammatory cytokine (for example, interleukin (IL)-1ra, IL-6, Fas ligand or tumor growth factor (TGF)-beta, a neurotrophic/neuroprotective growth factor such as, but not limited to, glial cell line-derived growth factor, brain-derived neurotrophic factor, nerve growth factor, neurotrophin-3, - 4/5, -6, and vitamin E. Such agents may be provided singly or in combination.

Personalized medicine applications are disclosed herein. In some embodiments, disclosed are methods of treating a subject with a retinal degenerative disease, retinal or retinal pigment epithelium dysfunction, retinal degradation, retinal damage, or loss of retinal pigment epithelium. The method can include determining, for the subject, which population of RPE cells is affected, such as macular, peripheral, and/or central RPE cells, producing those cells, and then administering these macular, peripheral, and/or central RPE cells to the subject. In some embodiments, the method includes locally administering to the eye of the subject a disclosed tissue replacement implant. The tissue replacement implant can include macular, central, and/or peripheral RPE cells, depending upon the disease process in the subject. The tissue replacement optionally can include photoreceptors, such as rods and/or cones and/or vascular cells. In certain non-limiting examples, the subject has CHM, AMD, L-ORD, or RD.

The disclosure is illustrated by the following non-limiting Examples.

### EXAMPLES

### Example 1

### Material and Methods

*Tissue Donor Information:* Seventeen eye globes from 9 healthy donors were obtained. The eyes were considered for the study if there was no history of retinal degeneration. The globes were enucleated and immediately stored and shipped in ice-cold PBS 1x or RPMI in wet ice. The globes were received, dissected and preserved in 4% PFA within 24h from donor's death.

*Staining and Imaging:* The RPE monolayers were incubated for 1 hour at RT in a PBS buffer containing 1% bovine albumin serum (BSA), 0.5% Tween 20 and 0.5 % Triton X to allow permeabilization and blocking of non-specific sites. Subsequently, the cell borders were stained overnight at RT with anti-ZO1 (cat #339100, Thermo Fisher Scientific) and anti-pan cadherin antibodies (cat #ab6529, Abcam) with a 1:200 dilution. The secondary antibodies anti-mouse-647 (cat #A-21235, Thermo Fisher Scientific) and anti-rabbit-633 (cat #A-21071, Thermo Fisher Scientific) were added for 1 hour at room temperature in the dark at with a 1:500 dilution. Both secondary antibody fluorophores were chosen to have a similar emission spectrum in the far-red wavelength in order to increase the signal to noise ratio. Indeed, because of their physiological activity and the elevated presence of oxygen in the retina, the RPE accumulate a lot of waste products, such as lipofuscin, which are extremely autofluorescent. The wavelength choice also aims at avoiding the broad bandwidth of lipofuscin autofluorescence. To further increase the signal to noise ratio, phalloidin-iFluor 647 (cat #ab176759, Abcam) was added along with the secondary antibodies with a 1:250 dilution. Nonetheless, the signal to noise ratio was not high enough to be able to distinguish a large percentage of RPE borders. After staining, a lipofuscin autofluorescence quencher, TRUEBLACK^{®} (cat #23007, Biotium) was used. TRUEBLACK^{®} was diluted 1:20 in ethanol 70% applied on the samples, with RPE facing up, for maximum 2 minutes. Every petal of the flatmounts was further cut to better flatten the tissue. The samples were finally mounted on a 50 x 75 mm glass slide (cat #5075, Brain Research Laboratories). A weight was put on top of the samples overnight to ensure they are homogeneously flat. The RPE monolayers were imaged using a Zeiss Axio Scan.Z1 widefield scanner (Carl Zeiss). Up to 250,000-275,000 tiles and a 120 µm z-stack were setup to include each sample. The stacks were compressed into a single slice on-line so that the final file would have a maximum dimension of 10 GB with CZI compression.

*iPSC-RPE Differentiation:* The iPSC line used for the screening and the subsequent phenotypic characterization, referred to as TJP1-mEGFP, was derived from the widely used parental line WTC-11 and the Tight Junction Protein 1 gene (*TJP1*; or Zonula occludens 1, *ZO1*) was CRISPR-modified so that the N-terminal exon of one allele has a monomeric enhanced green fluorescent protein (mEGFP) sequence insertion. The resulting TJP1 proteins are conjugated to mEGFP and allows live visualization of cell borders. A number of genomic and cellular validations were performed to exclude obvious adverse effects of tagging. The assays and their results are available at the Allen Institute website (allencell.org/cell-catalog.html). The iPSC line was differentiated into RPE using a protocol developed (Sharma et al., Science Translational Medicine, 11(475), doi.org/10.1126/scitranslmed.aat5580, 2019). RPE differentiation from stem cells can be induced by activation of TGF and WNT pathways at the neuroectodermal stage (Idelson et al., Cell Stem Cell, 5(4), 396-408, 2009; Lamba et al., Proceedings of the National Academy of Sciences of the United States of America, 103(34), 12769-12774, 2006; Leach et al., Investigative Ophthalmology & Visual Science, 56(2), 1002-1013, 2015; Reh et al., Directing Human Embryonic Stem Cells to a Retinal Fate, doi.org/10.1007/978-1-60761-691-7_9, 2010). A triphasic differentiation protocol was developed that further improves the efficiency and reproducibility of differentiation (Sharma et al., *op. cit.,* 2019). First, dual-SMAD inhibition was combined with FGF inhibition to induce the formation of neuroectoderm cells from iPSC, as dual-SMAD inhibition promotes neuronal fate and FGF pathway activation inhibits differentiation of the eye field into RPE (Bharti et al., PLoS Genetics, 8(7). doi.org/10.1371/journal.pgen.1002757, 2012; Chambers et al., Nature Biotechnology, 27(3), 275-280, 2009; Fuhrmann, Current Topics in Developmental Biology, 93, 61-84, 2010; Meyer et al., Proceedings of the National Academy of Sciences of the United States of America, 106(39), 16698-16703, 2009). Second, TGF-β and WNT pathways were activated to promote commitment of neuroectodermal cells to the RPE fate, see (Carr et al., PLoS ONE, 4(12), e81522009; Fuhrmann, Organogenesis, 4(2), 60-67, 2010; Idelson et al., Cell Stem Cell, 5(4), 396-408, 2009). Third, committed RPE were matured by treatment with prostaglandin E2 (PGE2) to actively suppress canonical WNT pathway through stimulation of primary cilium (May-Simera et al., Cell Reports, 22(1), 189-205, 2018).

For differentiation, iPSCs were seeded on vitronectin (cat #A1700, Thermo Fisher Scientific) coated 6-well plates. After 2 days in Essential 8 medium (E8, cat #A1517001, Thermo Fisher Scientific), cells were treated for 2 days with neuroectoderm induction medium (NEIM), which is composed by basal differentiation medium (DMEM/F12 (cat #11330032, Thermo Fisher Scientific), N2 supplement (cat #A1370701, Thermo Fisher Scientific), B27 (cat #17504044, Thermo Fisher Scientific), KSR (cat #12618013, Thermo Fisher Scientific), 200 µM ascorbic acid (cat #A4544, Sigma)) with the addition of 10 nM LDN (cat #04-0074 Stemgent), 0.5 µM CK1-7 Dihydrochloride (cat #C0742, Sigma), 1 µM SB 431542 hydrate (cat #S4317, Sigma), and 1 ng/ml IGF-1 (cat #AFL291, R&D Systems). Neuroectodermal cells were next cultured in RPE induction medium for 10 days (RPEIM, basal differentiation medium with 100 nM LDN (cat #04-0074 Stemgent), 5 µM CK1-7 Dihydrochloride (cat #C0742, Sigma), 10 µM SB 431542 hydrate (cat #S4317, Sigma), and 10 ng/ml IGF-1 (cat #AFL291, R&D Systems), 1 µM PD0325901 (cat #PZ0612Sigma)). Cells were cultured for 10 days in RPE commitment medium (RPECM, basal differentiation medium containing 10mM Nicotinamide (cat #N0636 Sigma), 150 ng/ml ACTIVIN A (cat #338-AC/CF R&D Systems)). Committed RPE were maintained in RPE growth medium for 5 days and then reseeded to remove neuronal formations (RPEGM, MEM + GLUTAMAX^{™} (cat #32561037, Thermo Fisher Scientific), 5%FBS (cat #SH30071.03, Hyclone), Taurine (cat #T-0625, Sigma), Thyronine (cat #T-5516, Sigma), Hydrocortisone (cat #H-0396-10, Sigma)). Subsequently, immature RPE were cultured for 15 days in RPEGM and then enriched by negative selection using anti-CD24 (cat #655154, BD Biosciences) and anti-CD56 (cat #340723, BD Biosciences) antibodies. Finally, immature RPE cells were seeded onto vitronectin-coated transwells (cat #3460, Corning) and cultured in RPE maturation medium (RPEMM, which is RPEGM containing 50 µM PGE2 (cat #2296/10, Tocris)) for 6 weeks to obtain fully mature iPSC-derived RPE. To generate macular (P1) and peripheral (P4) iPSC-RPE cells, AGN 193109 and endo-IWR-1 were respectively added to RPEGM from D21 (committed RPE cells) until mature cells were ready to be used (FIG. 1).

*Screening of Compound Library and Imaging:* 115 drugs targeting various developmental pathways or cytoskeletal stressors were selected to form a new developmental library of compounds. The drugs are either activators or inhibitors of various signaling pathways and target proteins at different levels of the signaling cascades. For each drug, an initial concentration was selected, and adjustments made to the concentration of each drug. The assays were performed on iPSC-derived committed RPE. For every drug, the concentration was lowered if toxicity was detected or increased if there was no evident effect on cell morphology in comparison to control. The list of compounds tested can be found in Table 1. The high-content screening was performed in 384-well plates (cat #6057300, Perkin Elmer) using a range of 3 different concentrations (3x, 1x, 0.3x). All drugs were reconstituted in dimethyl sulfoxide (DMSO, cat#D2650, Sigma) and 40 µl of each drug was aliquoted in triplicate in a 384-well master plate. Serial dilutions with RPEGM were then performed to obtain the final desired working concentrations and the final plates were stored at -80°C. After five days in RPEGM, committed RPE were reseeded in 384-well plates at a concentration of ~ 50 cells/mm² (500 cells/well) and let adhere and adapt for 24 hours with 10 µM rock inhibitor (cat #1254, Tocris). Every day one plate of compounds at the final concentration was thawed and used to add fresh drugs to the cells. This treatment procedure allows a minimal number of freeze/thaw cycles for the compounds, favors precision in comparison to the addition of a very small volume of drugs directly to cultured cells and minimizes the amount of time cultured cells spend outside of the incubator by transferring drugs with a multichannel pipette. Two timepoints, 15 and 30 days from cell seeding in 384-well plates, were chosen for imaging. A Yokogawa Cell Voyager (CV7000) high-throughput spinning disk confocal was used with a 20x air-immersion objective. Each well was included in 9 field of view, a z-stack was set up to take into account the unevenness of the monolayer and the images were projected to a single slice on-the-fly. Live imaging was performed for the first timepoint, while cells fixed with 4% PFA were imaged for the second. No staining was necessary since cells express TJP1-mEGFP protein throughout their lifetime.

**Table 1: List of compounds for high-content drug screening**

| **Drug** | **Target** | **Pathway** | **Effect on Pathway** |
|---|---|---|---|
| Verteporfin | YAP-TEAD disruption | Hippo pathway | Activator |
| Metformin hydrochloride | AMPK activator | Hippo pathway | Activator |
| Dorsomorphin dihydrochloride | AMPK inhibitor | Hippo pathway | Inhibitor |
| HA 1100 hydrochloride | Rho-kinase inhibitor | Hippo pathway | Activator |
| G-Protein antagonist peptide | GPCR inhibitor | Hippo pathway | Inhibitor |
| Dobutamine hydrochloride | YAP phosphorylation | Hippo pathway | Activator |
| Fasudil hydrochloride | Inhibitor of cyclic nucleotide dependent- and Rho-kinases | Hippo pathway | Activator |
| ML 141 | inhibitor of Cdc42 Rho family GTPase | Hippo pathway | Activator |
| Rhosin hydrochloride | Rho GTPase inhibitor | Hippo pathway | Activator |
| CCG 1423 | Rho/SRF pathway inhibitor | Hippo pathway | Activator |
| R18 | Inhibitor of 14.3.3 proteins | Hippo pathway | Inhibitor |
| IQ 1 | Activate canonical wnt pathway | WNT pathway | Activator |
| IWP 12 | Inhibits Wnt/β-catenin and Wnt/planar cell polarity (PCP) signaling pathways and PORCN | WNT pathway | Inhibitor |
| XAV 939 | Inhibits wnt patwhay | WNT pathway | Inhibitor |
| WIKI4 | Inhibits canonical wnt patwhay | WNT pathway | Inhibitor |
| ICG-001 | Canonical/ binds to CBP | WNT pathway | Inhibitor |
| Wnt-C59 (C59) | PORCN inhibitor | WNT pathway | Inhibitor |
| IWR-1-endo | Induce b-catenin degradation | WNT pathway | Inhibitor |
| KY02111 | Inhibits Wnt signaling, may act downstream of APC and GSK3β | WNT pathway | Inhibitor |
| LGK-974 | PORCN inhibitor | WNT pathway | Inhibitor |
| IWP-L6 | PORCN inhibitor | WNT pathway | Inhibitor |
| FH535 | Inhibits canonical wnt patwhay | WNT pathway | Inhibitor |
| CHIR 99021 | GSK-3 inhibitor | WNT pathway | Activator |
| Wnt agonist 1 | Activate canonical wnt pathway | WNTpathway | Activator |
| LDE225 (NVP-LDE225, Erismodegib) | Smoothened (Smo) antagonist | HH pathway | Inhibitor |
| Taladegib (LY2940680) | Binds to the Smoothened (Smo) receptor | HH pathway | Inhibitor |
| PF-5274857 | Smoothened (Smo) antagonist | HH pathway | Inhibitor |
| *Smoothened Agonist (SAG) HCl* | Smoothened (Smo) agonist | HH pathway | Activator |
| Cyclopamine | inhibition of Smoothened | HH pathway | Inhibitor |
| U 18666A | weak inhibitor of hedgehog (Hh) signaling | HH pathway | Inhibitor |
| AY 9944 dihydrochloride | via several mechanisms | HH pathway | Inhibitor |
| SANT-1 | antagonizes smoothened activity | HH pathway | Inhibitor |
| GANT 61 | GLI antagonist | HH pathway | Inhibitor |
| JK 184 | Alcohol dehydrogenase 7 inhibitor | HH pathway | Inhibitor |
| SANT-2 | antagonizes smoothened activity | HH pathway | Inhibitor |
| GANT 58 | GLI antagonist | HH pathway | Inhibitor |
| 20(S)-Hydroxycholesterol | activator/induces Smo accumulation | HH pathway | Activator |
| Ciliobrevin A | (Hh) pathway antagonist, inhibits ciliogenesis; also inhibits dynein | HH pathway | Inhibitor |
| Jervine | binds directly to Smo | HH pathway | Inhibitor |
| AZ 12080282 dihydrochloride | inhibition of Smoothened | HH pathway | Inhibitor |
| RU-SKI 43 hydrochloride | Hedgehog acyltransferase (Hhat) inhibitor | HH pathway | Inhibitor |
| IHR 1 | Smoothened (Smo) antagonist | HH pathway | Inhibitor |
| DAPT | γ-secretase inhibitor | Notch pathway | Inhibitor |
| Compound W | γ-secretase inhibitor | Notch pathway | Inhibitor |
| TW 37 | inhibits the activation of Notch-1 and Jagged-1 | Notch pathway | Inhibitor |
| ISX 9 | Activation of Ca²⁺ influx | Notch pathway | Activator |
| DBZ | γ-secretase inhibitor/ Blocks Notch cleavage | Notch pathway | Inhibitor |
| FLI 06 | Disrupts Notch trafficking and | Notch pathway | Inhibitor |
| RO4929097 | γ-secretase inhibitor/ Blocks Notch cleavage | Notch pathway | Inhibitor |
| Semagacestat (LY450139) | γ-secretase inhibitor | Notch pathway | Inhibitor |
| LY411575 | γ-secretase inhibitor/ Blocks Notch cleavare | Notch pathway | Inhibitor |
| IMR-1 | targets the transcriptional activation | Notch pathway | Inhibitor |
| TC-H 106 | Class I histone deacetylase inhibitor | Epigenetic pathway | Inhibitor |
| Pyroxamide | Histone deacetylase inhibitor | Epigenetic pathway | Inhibitor |
| LMK 235 | Selective HDAC4/HDAC5 inhibitor | Epigenetic pathway | Inhibitor |
| TCS HDAC6 20b | Selective HDAC6 inhibitor | Epigenetic pathway | Inhibitor |
| SAHA | Class I and II HDAC inhibitor | Epigenetic pathway | Inhibitor |
| GSK J4 | Histone demethylase JMJD3/UTX inhibitor; cell permeable | Epigenetic pathway | Inhibitor |
| RN 1 dihydrochloride | LSD1 inhibitor | Epigenetic pathway | Inhibitor |
| Salermide | SIRT1 and SIRT2 inhibitor | Epigenetic pathway | Inhibitor |
| 5-Azacytidine | DNA methyltransferase inhibitor | Epigenetic pathway | Inhibitor |
| KU 60019 | Potent ATM kinase inhibitor | Epigenetic pathway | Inhibitor |
| PF 03814735 | Aurora kinase A and B inhibitor | Epigenetic pathway | Inhibitor |
| U0126 | Potent, selective inhibitor of MEK1 and 2 | Epigenetic pathway | Inhibitor |
| H 89 dihydrochloride | Protein kinase A inhibitor | Epigenetic pathway | Inhibitor |
| I-CBP 112 | Selective CREBBP/EP300 inhibitor | Epigenetic pathway | Inhibitor |
| Bromosporine | bromodomain Broad spectrum bromodomain inhibitor | Epigenetic pathway | Inhibitor |
| UNC 926 hydrochloride | L3MBTL1 domain inhibitor | Epigenetic pathway | Inhibitor |
| 3-Aminobenzamide | PARP inhibitor; demethylates DNA | Epigenetic pathway | Inhibitor |
| PJ 34 hydrochloride | Potent PARP inhibitor; alters epigenetic marks in thyroid cancer cells | Epigenetic pathway | Inhibitor |
| IOX 2 | Potent, selective HIF-1α prolyl hydroxylase-2 (PHD2) inhibitor | Epigenetic pathway | Inhibitor |
| Forskolin | PKA activator; blocks nuclear export of HDAC5 | Epigenetic pathway | Activator |
| Retinoic acid | Endogenous retinoid; alters HDAC-mediated gene repression | Epigenetic pathway | Inhibitor |
| Triptolide | Inhibits RNAPII-mediated transcription | Epigenetic pathway | Inhibitor |
| AICAR | AMPK activator | Stem cell pathway | Activator |
| I-EBIO | Activator of epithelial KCa channels | Stem cell pathway | Activator |
| LY 294002 hydrochloride | Selective PI 3-kinase inhibitor | Stem cell pathway | Inhibitor |
| Prostaglandin E2 | Major endogenous prostanoid | Stem cell pathway | Inhibitor |
| GW 788388 | Selective inhibitor of TGF-βRI | Stem cell pathway | Inhibitor |
| Kenpaullone | Potent cyclin-dependent kinase inhibitor. Also inhibits GSK-3 | Stem cell pathway | Inhibitor |
| RG 108 | Non-nucleoside DNA methyltransferase inhibitor | Stem cell pathway | Inhibitor |
| BIO | Potent, selective GSK-3 inhibitor | Stem cell pathway | Inhibitor |
| Sodium 4-Phenylbutyrate | Histone deacetylase inhibitor | Stem cell pathway | Inhibitor |
| Valproic acid, sodium salt | Histone deacetylase inhibitor | Stem cell pathway | Inhibitor |
| BIX 01294 | G9a-like protein and G9a histone lysine methyltransferase inhibitor | Stem cell pathway | Inhibitor |
| Ceramide | Ser/Thr protein phosphatase activator | Stem cell pathway | Activator |
| RepSox | Selective inhibitor of TGF-βRI | Stem cell pathway | Inhibitor |
| Aphidicolin | Increases ciliogenesis by blocking the G1-to-S transition in cells | Stem cell pathway | Activator |
| FR 180204 | ERK2 and ERK1 inhibitor | FGF Pathway | Inhibitor |
| PD 0325901 | MEK1/2 inhibitor | FGF Pathway | Inhibitor |
| GSK 1059615 | PI3K inhibitor | FGF Pathway | Inhibitor |
| API-2 | inhibitor of Akt/PKB signaling | FGF Pathway | Inhibitor |
| SC 79 | Akt activator | FGF Pathway | Activator |
| Rapamycin | mTOR inhibitor | Cilium-related pathway | Inhibitor |
| PI 103 hydrochloride | Inhibitor of PI 3-kinase, mTOR and DNA-PK | Cilium-related pathway | Inhibitor |
| Compound 401 | DNA-PK and mTOR inhibitor | Cilium-related pathway | Inhibitor |
| KU 0063794 | mTOR inhibitor | Cilium-related pathway | Inhibitor |
| Torin 1 | mTOR inhibitor | Cilium-related pathway | Inhibitor |
| Torin 2 | mTOR inhibitor | Cilium-related pathway | Inhibitor |
| WYE 687 dihydrochloride | mTOR inhibitor | Cilium-related pathway | Inhibitor |
| ETP 45658 | Inhibitor of PI 3-kinase, mTOR and DNA-PK | Cilium-related pathway | Inhibitor |
| PF 04691502 | PI 3-K/mTOR inhibitor | Cilium-related pathway | Inhibitor |
| PF 05212384 | PI 3-K/mTOR inhibitor | Cilium-related pathway | Inhibitor |
| XL 388 | mTOR inhibitor | Cilium-related pathway | Inhibitor |
| Temsirolimus | mTOR inhibitor | Cilium-related pathway | Inhibitor |
| TC-A 2317 hydrochloride | Inhibits Aurora A kinase (Aurora A promote cilium disassembly) | Cilium-related pathway | Inhibitor |
| SBE 13 hydrochloride | Inhibits Polo-like kinase 1 (PLK1 promote cilium disassembly) | Cilium-related pathway | Inhibitor |
| T 5601640 | LIMK2 inhibitor (LIMK2 inhibits ciliogenesis) | Cilium-related pathway | Inhibitor |
| Cytochalasin D | actin filament destabilizer/induces ciliogenesis | Actin | Activator |
| CK 666 | Arp2/3 inhibitor/negative regulator of ciliogenesis | Actin | Inhibitor |
| Wiskostatin | Arp2/3 inhibitor/negative regulator of ciliogenesis | Actin | Inhibitor |
| CK 869 | inhibitor/negative regulator of ciliogenesis | Actin | Inhibitor |
| AGN 193109 | High affinity pan-RAR antagonist | Retinoic acid | Inhibitor |
| Liarozole dihydrochloride | Cytochrome P450 inhibitor | Retinoic acid | Inhibitor |

*Segmentation Using Convolutional Network:* A Convolutional Neural Network (CNN) algorithm was used to recognize RPE cell borders from images of samples labeled by immunofluorescence. The algorithm generates a binary mask of segmented RPE borders; and this output was manually corrected to provide the "right answer" on which the algorithm would learn. Once trained, the CNN algorithm was fed with images of fluorescently labeled RPE cells and the binary mask images that were generated become the input for the REShAPE software for cell shape analysis (FIG. 3).

Morphometric Analysis Using REShAPE: The binary segmentation produced by the CNN algorithm was transferred to REShAPE for cell shape analysis. REShAPE (Retinal Epithelium Shape and Pigment Evaluator) is a Fiji plugin for image analysis. For every cell in a field of view that has been successfully segmented, REShAPE provide quantification of more than 25 different shape metrics. The raw data was stored in a spreadsheet to allow for statistical analysis. In addition, the software created images of segmented cells for every metric analyzed, where every cell is color-coded according to the raw values (FIG. 4). The color-coded images display the location of the cells analyzed. The shape metrics were analyzed with the software to give measurements of cell dimensions, such as area and perimeter, measurements of elongation, such as length of major and minor axis, and measurements of cell regularity, such as hexagonality score and number of neighbors. The analysis was performed in pixel units or µm, as far as the pixel to µm conversion is provided. The analysis of data and the graphs have been done using R software ("R Core Team (2018). R: A language and environment for statistical computing. R Foundation for Statistical Computing, Vienna, Austria. URL https://www.R-project.org/"). The R packages "plyr" and "ggplot2" have been use for data handling and plotting respectively (Wickham, 2009, 2011). The Dunnett's test, a method for post hoc pairwise multiple comparisons, was used to compute statistical analysis by comparing several treatment groups to control groups. A 95% family-wise confidence level has been used. "DescTools" is the R package that has been used to perform Dunnett's test (Signorell, DescTools: Tools for Descriptive Statistics [R package DescTools version 0.99.31], retrieved October 25, 2019, from cran.r-project.org/web/packages/DescTools/index.htm, 2019). The assumption of normality was tested with Shapiro-Wilk's test. Because tests for normality are sensitive to sample size, the test was conducted on an unbiased subset of data of each group. Moreover, quantile-quantile (Q-Q) plots were drawn to assess normality on the whole dataset of each group. The homogeneity of variance was tested by plotting residuals on a "residuals versus fits plot". One-way ANOVA was used to evaluate between-group variance before performing Dunnett's test. Shapiro-Wilk's test and one-way ANOVA were carried out using the R package "dplyr" (Wickham et al., dplyr: A Grammar of Data Manipulation. Retrieved from https://cran.r-project.org/package=dplyr, 2019), while Q-Q plots were drawn using the R package "ggpubr" (Kassambara, ggpubr: "ggplot2" Based Publication Ready Plots. Retrieved from cran.r-project.org/package=ggpubr, 2020). Data are displayed as boxplots, where box limits represent the first and third quartile, the central line shows the median and the whiskers indicate the 5th and 95th percentile, so that the range specifies 90% of the data. For REShAPE morphometric analysis, single-cell measurements were considered as technical replicates, while each eye or each well of the drug screening is considered as biological replicate.

*Quantification of Human Eye Populations:* Some shape metrics parameters revealed the presence of different RPE populations across the human RPE flat-mounts (FIG. 5). A custom-made software was developed to separate these RPE populations. This software takes the x-y coordinates of every cells from the spreadsheet generated by REShAPE and reconstructs the color-coded images. The RPE populations are cropped directly on the color-coded images and the information of the selected cells are retrieved and stored in a new spreadsheet. Since RPE populations are arranged in rings, circular selection tools were used to isolate them. Even though the circular regions were cropped according to gradients of color-coded cells, the variability of the measurements for each ring is under 0.6 mm.

### Example 2

### Complete Morphometric Map of Adult Human RPE

Seventeen cadaver eyes were obtained from nine non-AMD patients to analyze the morphometry of adult human RPE cells. The eyes were shipped from the eye bank on wet ice and processed within 24h of patient's death. The eyes were injected with 1700 mOsm of mannitol solution in the vitreous to induce retinal detachment from RPE and, subsequently, the RPE/choroid complex was dissected and mounted as a flattened tissue. RPE cell borders were immunolabeled with anti-ZO1 and pan-cadherin antibodies, recognizing tight and adherens junctions respectively. Because, in RPE cells, actin filaments form a polygonal ring that connects tight junctions along cell borders, a fluorophore conjugated phalloidin was also used to enhance cell border staining. The whole RPE/choroid flat-mount was imaged and cell shapes were analyzed with our custom-built software, REShAPE. Color-coded images of the whole epithelium visually display the quantification of cell shapes for every metric analyzed. As reported previously at a smaller scale, not for the entire eye (Bhatia et al., *op. cit.,* 2016), a clear heterogeneity was observed across the whole RPE for certain shape metrics. For instance, cells were observed with smaller area, corresponding to the macular RPE, compared to cells located at the periphery of the epithelium (FIGS. 5-6). RPE cell area increases gradually with eccentricity. Interestingly a ring of small RPE cells was observed in a region included between 14 to 17 mm from the center of the eye. The change in cell area between peripheral RPE on the inner side of the ring and the peripheral ring of small RPE is abrupt, as well as the transition between the ring of small RPE and peripheral RPE on the outer side of the ring. Outside of the ring of small RPE, cell areas increase dramatically. However, all RPE cells appear to be similarly regular in shape throughout the epithelium, except for the cells at the very periphery which look very elongated and irregular. Fig. 2 illustrates the differences in human macular and peripheral RPE cells.

### Example 3

### Comparison Between Macular and RPE Cells

Five different RPE populations were distinguished according to their cell area (FIG. 6). The images of flat-mounts were divided into concentric rings centered around the middle of the eye, which is roughly halfway between the optic nerve and the macula, plus a ring around the macula. From the center to the periphery, the RPE populations were named from P1, macular RPE, to P5, the RPE at the very edge of the epithelium. P1 is macular, P2 is central, and P3 is peripheral; P4 is far-peripheral; P5 is ora-serrata RPE. Note that even though the circular regions were manually cropped according to gradients of color-coded cells, the spread around the average is under 0.6 mm. Therefore, the RPE populations in adult eyes have a very precise location. In addition, there is a slight asymmetry of RPE central and peripheral populations (P2 and P3): The central (P2) population always extends further on the temporal side, while peripheral (P3) population extends further on the nasal side. As a result, in the color-coded images of cell area, the color gradient on the temporal side is less pronounced than on the nasal side, suggesting that on the temporal side RPE cell areas tends to remain smaller.

The average RPE cell size increases with eccentricity until population P3 (peripheral), between 11.2 ± 0.6 mm and 14.3 ± 0.5 mm from the center of the eye. This RPE population has an average cell area of 238.6 ± 37.8 µm², that is almost 67% larger than macular RPE cell area in population P1 (macular, 149.6 ± 33.4 µm²). Peripherally to P3 (peripheral), from 14.6 ± 0.6 mm to 17.2 ± 0.4 mm, RPE cell size of population P4 reduces considerably (181.8 ± 39.5 µm²). Because of the variability of the dissection, this kind of quantification always includes in the P4 population, a fraction of P3 (peripheral) and P5 populations that biases measurements towards bigger area dimensions. Therefore, as can be appreciated from the color-coded image (FIG. 6), the P4 population may have a very similar average area as the macular cells (P1). Overall, this indicates the presence of macular type of RPE in the periphery.

At the very periphery of the flat-mount, from 17.6 ± 0.4 mm up to 21.1 ± 0.5 mm, the average area of RPE population P5 becomes considerably larger and variable as compared to the rest of the eye (336.8 ± 49.6 µm²). By visual inspection, in the first couple of millimeters P5 area contains cells with an area comparable to peripheral population (P3), while more eccentrically cell areas become much larger. It is likely that the most peripheral RPE cells belong to the region of the *ora serrata,* where the photosensitive retina transitions to the non-photosensitive area of the ciliary body.

### Example 4

### Comparison of Morphometry Between iPSC-RPE and Adult Human RPE

Next, the morphological RPE heterogeneity of P1 (macular) and P3 (peripheral) was recreated *in vitro.* Among the non-macular RPE populations (P2-P5), peripheral (P3) was selected because it is the most likely to support a pure population of rod photoreceptors. There is no report in the literature which links stem cell-derived RPE to any specific human RPE population, despite the importance of location in regional retinal degenerations. Generating *in vitro* RPE populations that reproduce the different susceptibility to degeneration would provide a better system to study diseases, such as AMD. Far-peripheral (P4) cells can be damaged in late-stage AMD and in traumatic injuries. Ora serratta (P5) RPE cells can be damaged in traumatic injuries

The morphometry of fully mature iPSC-derived RPE (iPSC-RPE) generated in *ex vivo* culture was compared human RPE populations that were identified in the 17 flat-mounts from adult healthy donors. Curiously, when cell size was compared, the iPSC-RPE cells generated *in-vitro* appear to have a smaller area than any human RPE population. The population with the most similar dimensions is the macular population, labeled P1 (iPSC-RPE: 107.0 ±61.7 µm² vs P1: 149.6 ± 33.4 µm²) (FIG. 8).

### Example 5

### Comparison of Treated iPSC-RPE With Adult Human RPE Morphometry

Since REShAPE was shown to be a very sensitive tool to quantify cell morphology and since morphometric analysis is a very simple and scalable technique, morphometry was used as a screening assay to identify macular (P1) and peripheral (P3) RPE populations derived from iPSCs. A high content screening of compounds on iPSC-RPE was performed during differentiation to enrich and separate macular and peripheral RPE. The 115 selected compounds include activators and inhibitors of different developmental pathways and stressors of cytoskeleton. Committed RPE cells were seeded in 384-well plates and treated for 30 days before performing cell shape analysis. Cells were treated while they are committed to the RPE fate in order to intervene early during RPE differentiation, when developmental pathways still play major roles, but without changing the RPE fate. An iPSC-RPE cell line whose TJP1 (ZO1) protein was conjugated to mEGFP was used. High-content imaging was performed, and fluorescent images were analyzed with REShAPE. Three concentrations, separated three-fold from one another, were assessed. The data for each shape metric were analyzed and plotted. Cell area was the metric chosen to compare treated iPSC-RPE with human RPE because of the clear distinction between RPE populations (FIG. 7).

All of the drugs that induced a shift of cell size distribution in any concentration range were selected and the raw images were checked for the non-specific effects caused by toxicity. The remaining drugs are listed in Table 2.

**Table 2: Subset of drugs from screening**

| **Drug** | **Target** | **Pathway** | **Effect on Pathway** |
|---|---|---|---|
| DAPT | γ-secretase inhibitor | Notch pathway | Inhibitor |
| ISX 9 | Induces neuronal differentiation | Notch pathway | Activator |
| DBZ | γ-secretase inhibitor/ | Notch pathway | Inhibitor |
| | Blocks Notch cleavage | | |
| RO4929097 | γ-secretase inhibitor/ Blocks Notch cleavage | Notch pathway | Inhibitor |
| Semagacestat (LY450139) | γ-secretase inhibitor | Notch pathway | Inhibitor |
| LY411575 | γ-secretase inhibitor/ Blocks Notch cleavage | Notch pathway | Inhibitor |
| *Smoothened Agonist (SAG) HCl* | Smoothened (Smo) agonist | HH pathway | Activator |
| SANT-1 | antagon antagonizes smoothened activity | HH pathway | Inhibitor |
| SANT-2 | antagonizes smoothened activity | HH pathway | Inhibitor |
| GANT 58 | GLI antagonist | HH pathway | Inhibitor |
| GW 788388 | Selective inhibitor of TGF-βRI | Stem cell pathway | Inhibitor |
| Kenpaullone | Potent cyclindependent kinase inhibitor. Also inhibits GSK-3 | Stem cell pathway | Inhibitor |
| BIO | Potent, select selective GSK-3 inhibitor | Stem cell pathway | Inhibitor |
| IWP 12 | Inhibits Wnt/β-catenin and Wnt/planar cell polarity (PCP) signaling pathways and PORCN | WNT pathway | Inhibitor |
| XAV 939 | Inhibits wnt patwhay | WNT pathway | Inhibitor |
| WIKI4 | Inhibits canonical wnt patwhay | WNT pathway | Inhibitor |
| ICG-001 | Canonical/ binds to CBP | WNT pathway | Inhibitor |
| IWR-1-endo | Induce b-catenin degradation | WNT pathway | Inhibitor |
| KY02111 | Inhibits Wnt signaling, may act downstream of APC and GSK3β | WNT pathway | Inhibitor |
| LGK-974 | PORCN inhibitor | WNT pathway | Inhibitor |
| FH535 | Inhibits canonical Inhibits canonical wnt patwhay | WNT pathway | Inhibitor |
| CHIR 99021 | GSK-3 inhibitor | WNT pathway | Activator |
| PI 103 hydrochloride | Inhibitor of PI 3-kinase, mTOR and DNA-PK | Cilium-related pathway | Inhibitor |
| ETP 45658 | Inhibitor of PI 3-kinase, mTOR and DNA-PK | Cilium-related pathway | Inhibitor |
| PF 04691502 | PI 3-K/mTOR inhibitor | Cilium-related pathway | Inhibitor |
| XL 388 | mTOR inhibitor | Cilium-related pathway | Inhibitor |
| T 5601640 | LIMK2 inhibitor LIMK2 inhibitor (LIMK2 inhibits ciliogenesis) | Cilium-related pathway | Inhibitor |
| HA 1100 hydrochloride | Rho-kinase inhibitor | Hippo pathway | Activator |
| Fasudil hydrochloride | Inhibitor of cyclic nucleotide dependent- and Rhokinases | Hippo pathway | Activator |
| CCG 1423 | Rho/SRF pathway inhibitor | Hippo pathway | Activator |
| FR 180204 | ERK2 and ERK1 inhibitor | FGF Pathway | Inhibitor |
| PD 0325901 | MEK1/2 inhibitor | FGF Pathway | Inhibitor |
| GSK 1059615 | PI3K inhibitor | FGF Pathway | Inhibitor |
| API-2 | inhibitor of Akt/PKB signaling | FGF Pathway | Inhibitor |
| SC 79 | Akt activator | FGF Pathway | Activator |
| TC-H 106 | Class I histone deacetylase inhibitor | Epigenetic pathway | Inhibitor |
| LMK 235 | Selective HDAC4/HDAC5 inhibitor | Epigenetic pathway | Inhibitor |
| TCS HDAC6 20b | Selective HDAC6 inhibitor | Epigenetic pathway | Inhibitor |
| KU 60019 | Potent ATM kinase inhibitor | Epigenetic pathway | Inhibitor |
| U0126 | Potent, selective inhibitor of MEK1 and 2 | Epigenetic pathway | Inhibitor |
| H 89 dihydrochloride | Protein kinase A inhibitor | Epigenetic pathway | Inhibitor |
| Bromosporine | Broad spectrum bromodomain inhibitor | Epigenetic pathway | Inhibitor |
| PJ 34 hydrochloride | Potent PARP inhibitor; alters epigenetic marks in thyroid cancer cells | Epigenetic pathway | Inhibitor |
| IOX 2 | Potent, selective HIF-1α prolyl hydroxylase-2 (PHD2) inhibitor | Epigenetic pathway | Inhibitor |
| Retinoic acid | Endogenous retinoid; alters HDAC-mediated gene repression | Epigenetic pathway | Inhibitor |
| Wiskostatin | Arp2/3 inhibitor/negative regulator of ciliogenesis | Actin | Inhibitor |
| AGN 193109 | High affinity pan-RAR antagonist | Retinoic acid | Inhibitor |

The selected drugs were grouped according to the pathway they act on and their role (activator or inhibitor). For each group, if there was more than one drug, the one that induced the strongest shift in cell size was carried forward for characterization of their effect on iPSC-RPE phenotype. Eleven drugs were carried forward for iPSC-RPE phenotypic analysis (Table 3).

**Table 3: Subset of drugs chosen for phenotypic characterization.**

| **Drug** | **Target** | **Pathway** | **Effect on Pathway** | **Concentration (µM)** |
|---|---|---|---|---|
| DBZ | γ-secretase inhibitor/ Blocks Notch cleavage | Notch pathway | Inhibitor | 24 |
| *Smoothened Agonist (SAG) HCl* | Smoothened (Smo) agonist | HH pathway | Activator | 1.2 |
| SANT-2 | antagon antagonizes smoothened activity | HH pathway | Inhibitor | 12 |
| GW 788388 | Select ive inhibitor of TGF-βRI | Stem cell pathway | Inhibitor | 4 |
| IWR-1-endo | Induce b-catenin degradation | WNT pathway | Inhibitor | 4 |
| CCG 1423 | Rho/SRF pathway inhibitor | Hippo pathway | Activator | 1 |
| FR 180204 | ERK2 and ERK1 inhibitor | FGF Pathway | Inhibitor | 55.8 |
| TCS HDAC6 20b | Selective HDAC6 inhibitor | Epigenetic pathway | Inhibitor | 60 |
| Wiskostatin | Arp2/3 inhibitor/negative regulator of ciliogenesis | Actin | Inhibitor | 4.1 |
| AGN 193109 | High affinity pan-RAR antagonist | Retinoic acid | Inhibitor | 0.2 |

### Example 6

### Phenotypic Characterization of Selected Drug Treatments

Eleven compounds were selected for further phenotypic characterization based on their initial effect in RPE cell size. The goal of this characterization was two-fold: 1) to analyze iPSC-RPE phenotype after drug treatment to confirm that the drugs did not adversely affect the health of cells; and 2) to confirm the presence of macular or peripheral type apical processes on cells. The structure of RPE apical processes has been described as cone or rod specific: petal-like apical processes have been observed to wrap around cone outer segments, while finger-like structures are juxtaposed to rod outer segments (Fisher & Steinberg, J. Comparative Neurology, 206(2), 131-145, 1982; Steinberg & Wood, Proceedings of the Royal Society of London - Biological Sciences, 187(1089), 461-478, 1974). Because the macula is dominated by cones while the periphery is dominated by rods, RPE cells in these two different regions might be enriched in one of the two apical process structures. It was hypothesized that the phenotype of RPE apical processes will serve as an additional discriminatory factor, other than RPE morphometry.

Different techniques were used to study RPE morphologic features. Trans-epithelial electrical resistance was used to examine tight junction integrity, brightfield imaging was useful to determine RPE pigmentation defects, hematoxylin and eosin staining and transmission electron microscopy were valuable to inspect gross and fine morphologic alterations, respectively, while scanning electron microscopy revealed modifications in apical processes structures. Two compounds, ENDO-1-IWR and AGN 193109, were not disruptive of iPSC-RPE health and revealed different types of apical processes phenotypes.

Starting at D21, iPSC-RPE were treated with the different compounds. At D40 immature RPE were enriched by negative selection, as per differentiation protocol, and seeded on transwells for maturation. iPSC-RPE were treated with the compounds for other 6 weeks until cells were fully mature (FIG. 1 and FIG. 11).

At this point the trans-epithelial electrical resistance (TER) was assayed to confirm the quality of differentiation and examine the effect of drugs on tight junction integrity. The minimum threshold of 400 Ωcm² for epithelial resistance per unit area is used to determine good batches of differentiation. DMSO-treated iPSC-RPE showed an average TER measurement around 1100 Ωcm², confirming the quality of this batch of differentiation. While other compounds (see Table 3) disrupted the epithelial barrier integrity ENDO-1-IWR and AGN 193109 not only maintained tight junction integrity, but also induced an increase in TER resistance (ENDO-1-IWR: ~1200 Ωcm², AGN 193109: ~1600 Ωcm²).

Subsequently, shape metrics were analyzed to confirm the effect of the drugs on cell areas. Compared to DMSO-treated iPSC-RPE, AGN 193109 induced an increase in iPSC-RPE cell areas which is comparable to the dimension of macular RPE population (P1) (iPSC-RPE area = 161.8 ± 149.7 µm², P1 = 149.6 ± 33.4 µm²), while ENDO-1-IWR induced a larger increase in cell sizes, which is comparable to cells of peripheral RPE population P3 (iPSC-RPE area = 251.8 ± 218.4 µm², P3 = 238.6 ± 37.8µm²). The graph and the color-coded images in FIGS. 8-9 show the comparison between the different groups.

Brightfield imaging was used to determine RPE pigmentation defects, while hematoxylin and eosin (H&E) staining was valuable to inspect gross morphologic alterations of the monolayer. A section of transwell membrane was cut and mounted on a glass slide for brightfield imaging. Pigmentation levels were preserved after ENDO-1-IWR and AGN 193109 treatments as compared to DMSO-treated iPSC-RPE. Another area of the transwell was sectioned and stained with H&E. After drug treatment, cells were still arranged as a monolayer, no gross morphological alterations were detected, and pigment granules were still present on the apical side of RPE cells.

Finally, transmission electron microscopy (TEM) was used to detect fine morphological alterations, whereas scanning electron microscopy (SEM) was essential to study modifications in apical processes structures. While no fine intracellular structure defects were identified after ENDO-1-IWR and AGN 193109 treatments, the two drugs induced changes of apical processes structures. In DMSO-treated iPSC-RPE we found both types of apical processes, petal and finger-like. Remarkably, upon ENDO-1-IWR treatment, all RPE cells were enriched uniquely by finger-like apical processes, which have been associated with RPE interaction with rod outer segments. On the other hand, AGN 193109 treatment enriched RPE cells with petal-like apical processes (with undulation) which support cone outer segments. The fact that ENDO-1-IWR increases cell sizes up to the dimensions of the peripheral RPE population P3 and enriches cells with finger-like apical processes evidence that this compound induces a peripheral RPE phenotype. Conversely, AGN 193109 shifts iPSC-RPE cell sizes toward macular RPE population (P1) and induces formation of petal-like apical processes (with undulation) evidence that this compound induces a macular RPE phenotype (FIGS. 10 and 11).

Overall, these results indicate that using cell shape as a screening tool does not only separate populations of big cells from small ones, but selects morphological characteristics. Without being bound by theory, the data indicates that developmental pathways were targeted during RPE differentiation.

In summary, 17 RPE/choroid flat-mounts were dissected from the eyes of 9 non-AMD donors, stained for RPE cell borders, imaged, and an average of 3-4 million cells were segmented per flat-mount. Complete morphometric maps of the entire human RPE were generated for the first time for each human eye used in this study. Cell area was chosen among other shape metrics to highlight the heterogeneity of RPE cells across the entire eye. A gradient of RPE cells was identified with progressively larger areas, starting from small RPE cells in the macula and moving eccentrically toward the periphery. A peripheral ring of small RPE cells was detected around 14-17 mm of radius from the center of the eye. Five unique zones of RPE cells were identified.

The finding of a peripheral ring of smaller RPE cells indicates the presence of macular type RPE cells in the far periphery. The RPE population P4 may correspond to the same area where a peripheral rim of cones has been described. For example, there is a sudden transition from big RPE cells in populations P3 (peripheral) to small cells in population P4 (far-peripheral) and big again in population P5 (ora serrata). In addition, the RPE population P4 is about 1-2 mm wide contained in a region that is about 14 to 17 mm away from the center of the eye and before the transition zone of the *ora serrata* - the same area in which a loose band of cones was described. It appears that small RPE cells are present in cone dominated areas, such as the macula and the peripheral rim, while big RPE cells develop in rod-dominated areas.

The disclosed methods achieve the reproduction of regional RPE heterogeneity *in vitro.* To achieve this, cell size of iPSC-RPE generated in culture was compared with the average cell area of each human RPE population. The comparison showed that fully mature RPE grown in culture have a cell area that is smaller than any other human RPE population identified in cadaver eye samples. The closest human RPE population in terms of size was the macular RPE (P1). It's unlikely that seeding concentration induces a smaller cell size, because cells were seeded in different plate formats and concentration was adjusted accordingly, but the average cell area did not substantially change.

To identify such a signal that dictates RPE cell size, iPSC-RPE cells were manipulated during a differentiation stage where they have just been committed to the RPE fate. This manipulation was performed using 115 activators and inhibitors of different developmental pathways and modulators of the actin cytoskeleton. RPE morphometry was analyzed for every drug and compared as a reference to human macular RPE populations (P1) and peripheral RPE population P3.

Peripheral population P3 was selected as reference for periphery, because population P5 contains RPE cells of the ora serrata that may not have a "true" RPE feature, and population P4 (far-peripheral) resembles macular RPE in cell size.

Compounds were grouped by pathway and by their role in activating or inhibiting it, and the compounds that showed the greatest shift in cell size for each group was tested for phenotypic characterization. It was found that two compounds, AGN 193109 and ENDO-1-IWR, were able to reproduce the cellular size of macular population (P1) and peripheral population (P3) respectively, and enrich RPE cells with petal-like and finger-like apical processes, respectively. Both compounds did not affect the healthy RPE phenotype as verified by TER, brightfield, H&E and TEM.

AGN 193109 is a high affinity pan-retinoic acid receptor (RAR) antagonist. Retinoic acid is a form of vitamin A not available in the diet but is synthesized from retinol. Its levels are therefore regulated by its synthesis and degradation (Duester, Molecular and Cellular Biology, 11(3), 1638-164; Napoli, J. Biol. Chem., 261(29), 13592-13597j 1986). Retinoic acid can regulate many important biological processes, such as embryogenesis, immunity, promotion or inhibition of cell proliferation and differentiation (Lotan, Biochimica et Biophysica Acta (BBA) - Reviews on Cancer, 605(1), 33-91, 1980). The RPE plays a central role in retinoid metabolism in the eye: it is a reservoir of retinyl esters, the storage form of vitamin A, and the isomerization and oxidation of all-trans retinyl esters to form 11-cis-retinaldehyde occurs in RPE cells (Rando, Biochemistry, 30(3), 595-60, 1991). Moreover, the RPE is one of the tissues that contains the highest concentration of retinoids in the body, highlighting the importance of RPE cells in vitamin A metabolism (Berman et al., Investigative Ophthalmology, 13(9), 675-687, 1974). It has been shown that cultured bovine RPE cells metabolize retinoic acid through the activity of a cytochrome P-450 monooxygenase. The RPE converts retinoic acid to a more polar metabolite, the 4-oxo-retinoic acid, that can be rapidly released from the cells (Doyle et al., Investigative Ophthalmology & Visual Science, 36(3), 708-717, 1995). This result indicates that the RPE may be important in the deactivation of this biologically potent retinoid in the retina.

A localized degradation of retinoic acid from the RPE and retina during early eye development might designate the location of the future macular RPE and fovea. Without being bound by theory, AGN 193109 may mimic retinoic acid depletion by antagonizing RARs of RPE cells. To verify this hypothesis, basal levels of retinoic acid signal can be verified and the expression and activity of cytochromes in control and AGN 193109-treated RPE cells can be checked.

Other commercially available retinoic acid inhibitors can be used to generate macular iPSC-RPE (P1). These are shown in Table 4.

**Table 4: Exemplary retinoic acid inhibitors which can be used to generate peripheral iPSC-RPE**

| **Drug** | **Target** | **Pathway** | **Effect on Pathway** |
|---|---|---|---|
| BMS 195614 | Neutral retinoic acid receptor (RAR) α-selective antagonist | Retinoic acid | Inhibitor |
| CD 2665 | RARβγ antagonist | Retinoic acid | Inhibitor |
| ER 50891 | Antagonist of RARα receptors | Retinoic acid | Inhibitor |
| LE 135 | RARβ antagonist | Retinoic acid | Inhibitor |
| LY 2955303 | RARγ antagonist | Retinoic acid | Inhibitor |
| MM 11253 | RARy-selective antagonist | Retinoic acid | Inhibitor |
| Liarozole dihydrochloride | Cytochrome P450 inhibitor | Retinoic acid | Inhibitor |

ENDO-1-IWR is a canonical Wnt pathway inhibitor. Wnt is an evolutionarily conserved pathway that regulates crucial aspects of cell fate determination, cell migration, cell polarity, neural patterning and organogenesis during embryonic development. The Wnt pathway can be subdivided downstream of Frizzled receptors into two main branches: canonical Wnt pathway, which is dependent on β-catenin, and the non-canonical Wnt pathway. The latter can be further subdivided into Planar Cell Polarity (PCP) and Wnt/Ca²⁺ pathways. Wnt molecules are secreted glycoproteins that bind to the Frizzled receptor family. Wnt signaling is initiated by binding of Frizzled receptor to a co-receptor, such as low-density-lipoprotein-related protein 5/6 (LRP5/6) in the case of canonical Wnt signaling. The signal is then transduced to the cytoplasmic protein Dishevelled (Dsh) and at this level the pathway branches (Komiya & Habas, Organogenesis, 4(2), 68-75, 2008). The hallmark of the canonical Wnt pathway is the accumulation and translocation of the adherens junction associated-protein β-catenin into the nucleus, which activates transcription of target genes through the binding with DNA-binding transcription factors, such as LEF/TCF (Clevers, Cell 127(3), 469-480.2006; Reya & Clevers, Nature, 434(7035), 843-850, 2005). In the absence of Wnt signaling, β-catenin is phosphorylated by the β-catenin destruction complex and targeted for degradation by the proteasome. Planar Cell Polarity has been shown to regulate the organization and orientation of epithelial cells (Mlodzik, Trends in Genetics : TIG, 18(11), 564-571, 2002). This pathway appears to be independent of transcription, but functions through direct regulation of the actin cytoskeleton to achieve organization of structures. The second branch of the non-canonical pathway is named Wnt/Ca²⁺. It is independent from β-catenin-induced transcription and it is characterized by G-protein-mediated intracellular Ca²⁺ release from ER (Kohn & Moon, Cell Calcium, 38(3-4), 439-446, 2005; Slusarski & Pelegri, Developmental Biology, 307(1), 1-13, 2007).

ENDO-1-IWR, the compound used for RPE phenotype characterization, acts by stabilizing the Axin protein of β-catenin destruction complex. The stabilization of the destruction complex promotes β-catenin degradation, hence canonical Wnt pathway inhibition.

The Wnt pathway plays a role in RPE development. First, Wnt pathway inhibition along with BMP inhibition allows for the generation of the eye field from the anterior neuroepithelium. *Dkk-1* and *Noggin* endogenous expression, Wnt/β-catenin and BMP inhibitors respectively, was upregulated in human embryonic stem cells during eye field specification (Meyer et al., Proceedings of the National Academy of Sciences of the United States of America, 106(39), 16698-16703, 2009). Second, during RPE specification, canonical Wnt is activated and β-catenin translocates into the nucleus and directly binds to enhancer sites for the RPE specific genes, *Mitf* and *Otx2,* inducing their expression. When β-catenin is deleted, the RPE transforms into a multilayered tissue in which *Mitf* and *Otx2* are downregulated and results in transdifferentiation of RPE into retina (Westenskow et al., Development, 136(15), 2505-2510, 2009).

Based on the data disclosed herein, the canonical Wnt pathway further plays a role in RPE at later stages of development, from the RPE commitment stage all the way up the mature RPE stage. During this phase, canonical Wnt inhibition by ENDO-1-IWR increases RPE cell size to match human peripheral RPE population P3 and enriches these cells with finger-like apical processes to support a rod-dominated region of the retina. The gradient of cell sizes can be achieved through a combination of retinoic acid and Wnt inhibition.

Other Wnt inhibitors used in the screening show a similar trend in increase of iPSC-RPE cell area, further supporting the role of Wnt inhibition in the generation of peripheral RPE cells (FIG. 12). This evidence indicates that other commercially available canonical Wnt inhibitors can be used to generate peripheral iPSC-RPE (P3). Table 5 provides an exemplary list of these compounds.

**Table 5: Exemplary canonical Wnt inhibitors which can be used to generate peripheral iPSC-RPE**

| **Compound** | **Target** | **Pathway** | **Effect on Pathway** |
|---|---|---|---|
| Calphostin C | PKC inhibitor | Wnt pathway | Inhibitor |
| Cardionogen 1 | Inhibitor of Wnt/β-catenin signaling | Wnt pathway | Inhibitor |
| CCT 031374 hydrobromide | Inhibits TCF-dependent transcription; lowers β-catenin levels | Wnt pathway | Inhibitor |
| IWP 12 | Inhibits Wnt/β-catenin and Wnt/planar cell polarity (PCP) and PORCN | Wnt pathway | Inhibitor |
| XAV 939 | Inhibits wnt patwhay | Wnt pathway | Inhibitor |
| WIKI4 | Inhibits canonical wnt patwhay | Wnt pathway | Inhibitor |
| ICG-001 | Canonical/ binds to CBP | Wnt pathway | Inhibitor |
| Wnt-C59 (C59) | PORCN inhibitor | Wnt pathway | Inhibitor |
| IWR-1-endo | Induce b-catenin degradation | Wnt pathway | Inhibitor |
| KY02111 | Inhibits Wnt signaling, may act downstream of APC and GSK3β | Wnt pathway | Inhibitor |
| LGK-974 | PORCN inhibitor | Wnt pathway | Inhibitor |
| IWP-L6 | PORCN inhibitor | Wnt pathway | Inhibitor |
| FH535 | Inhibits canonical wnt patwhay | Wnt pathway | Inhibitor |
| iCRT 14 | Inhibits β-catenin-responsive transcription (CRT) | Wnt pathway | Inhibitor |
| IWP 4 | Potent inhibitor of Wnt/β-catenin signaling | Wnt pathway | Inhibitor |
| JW 67 | induces degradation of active β-catenin | Wnt pathway | Inhibitor |
| JW 74 | Targets the β-catenin destruction complex | Wnt pathway | Inhibitor |
| KYA 1797K | Wnt/β-catenin signaling inhibitor | Wnt pathway | Inhibitor |
| NLS-StAx-h | inhibits β-catenin-transcription factor interactions | Wnt pathway | Inhibitor |
| PNU 74654 | β-catenin binder | Wnt pathway | Inhibitor |
| TAK 715 | inhibits Wnt-3a-stimulated β-catenin signaling | Wnt pathway | Inhibitor |
| IWP 2 | PORCN inhibitor | Wnt pathway | Inhibitor |
| CKI 7 dihydrochloride | CK1 inhibitor | Wnt pathway | Inhibitor |
| (R)-CR8 | CK1 inhibitor | Wnt pathway | Inhibitor |
| D 4476 | CK1 inhibitor | Wnt pathway | Inhibitor |
| (R)-DRF053 dihydrochloride | CK1 inhibitor | Wnt pathway | Inhibitor |
| Epiblastin A | CK1 inhibitor | Wnt pathway | Inhibitor |
| IC 261 | CK1 inhibitor | Wnt pathway | Inhibitor |
| LH 846 | CK1 inhibitor | Wnt pathway | Inhibitor |
| PF 4800567 hydrochloride | CK1 inhibitor | Wnt pathway | Inhibitor |
| PF 5006739 | CK1 inhibitor | Wnt pathway | Inhibitor |
| PF 670462 | CK1 inhibitor | Wnt pathway | Inhibitor |
| SR 3029 | CK1 inhibitor | Wnt pathway | Inhibitor |
| AZ 6102 | TNKS1/2 inhibitor | Wnt pathway | Inhibitor |
| JW 55 | Tankyrase inhibitor | Wnt pathway | Inhibitor |
| MN 64 | tankyrase inhibitor | Wnt pathway | Inhibitor |
| TC-E 5001 | tankyrase inhibitor | Wnt pathway | Inhibitor |

Canonical Wnt inhibition during the RPE maturation phase (D40-D75, which are the total number of days in culture) can promote RPE maturation (May-Simera et al., Cell Reports 22(1), 189-205, 2018). The treatment can start 15 days after the start of culture in the maturation medium. In contrast to the data presented here, Wnt inhibition starts during cell commitment to the RPE fate and is maintained until the end of RPE maturation (D25-D75). During RPE commitment, developmental pathways still play major roles and can influence the final RPE phenotype, but without changing the RPE fate.

Thus, human RPE populations of macular (P1) and peripheral (P3) RPE cells were reproduced *in vitro.* Exemplary non-limiting concentrations that were used are listed in Table 6.

**Table 6: Exemplary gradient to reproduce other RPE populations**

| **Population** | **AGN 193109** | **ENDO-1-IWR** |
|---|---|---|
| P1 (macular) | 0.1-0.2 µM | - |
| P2 (central) | 25-50 nM | 0.1-0.2 µM |
| P3 (peripheral) | - | 1-4 µM |

**Table 7. Additional Concentrations of Use**

| **Population** | **AGN 193109** | **ENDO-1-IWR** |
|---|---|---|
| P1(macular) | 0.05-0.4 µM | - |
| P2 (central) | 10-50 nM | 0.025-0.5 µM |
| P3 (peripheral) | - | 0.05-8 µM |

The work provides insights on macular and RPE development, and can be used to improve current iPSC-based disease models of retinal degenerations and to develop cell therapy products or one or more regions of the retina (FIG. 14).

### Example 7

### Generation of Bi-Layer Scaffold

This example describes methods used to prepare a bi-layer scaffold, referred to herein as a "fuzzy" or "PLGA/PCL" scaffold. Such a scaffold can be used to seed RPE and PRP cells. In some examples, the scaffold generated is biodegradable, for example 20 days to 6 months following its implantation into an eye.

The bi-layer scaffold was prepared using a modification of the methods described in US Patent No. 10,480,031. Briefly, heat fused electrospun poly(lactic-co-glycolic acid) (PLGA) scaffold was used, which was generated by heat treatment at >56C allowing for gentle fusion of fibers and permits cells to grow on top.. The PLGA scaffold was about 20-30 microns in height, having average pore size of less than 1 micron, a DL-lactide/glycolide ratio of 1:1, and a fiber diameter of 150 to 650 nm.

The PLGA scaffolds were used as a base to add polycaprolactone (PCL) loops on a surface of the PLGA scaffold. During the electrospinning, PCL becomes permanently attached to the surface of the PLGA scaffold. To deposit PCL loops, the PCL was melted at 100°C (a range of about 70°C to about 140°C can be used) for 30 minutes prior to ejection during the electrospun process, which used an electric field voltage of 25 kV (a range of about 5 to about 50 kV can be used), a gas ejection pressure of 300 kPa, a working distance between nozzle and PLGA scaffolds of 27mm (a range of about 10mm -about 40mm can be used), with 5 minutes (a range of about 2 minutes to about 10 minutes can be used) of electrospinning time. These parameters generated randomly deposited loops that form a spongy mesh structure on the PLGA scaffold surface. The PCL diameter ranged from about 5 um to 300um. Loops were distributed in random pattern, having a random thickness of loop forming fibers. Loop density and number of loops can be experimentally determined to provide sufficient gaps for multiple RPE cells (10-1000 cells) form monolayer areas. PCL loops deposited on PLGA scaffold include a mixture completely closed or open loops.

The PLGA/PCL scaffold was sterilized as follows. The Plasma Etch PE50XL device was used to create oxygen plasma emission for the treatment of scaffold fibers and to sterilize the surfaces of scaffolds. The working pressure was ~0.187 hPa (~140 mtorr), with oxygen flow to ~7 cc/min. The cycle is the maximum voltage (120V) for 30 minutes.

The sterilized PLGA/PCL scaffold (10 mm in diameter, can be about 10 mm to about 30 mm in diameter) was mounted on the surface of a Corning^{®} Costar^{®} Snapwell^{™} system. This system provides the structure and platform for the PLGA/PCL scaffolds. The microporous membrane of the system creates an apical and basal side, which provides support to the scaffold as well as isolating the distinct sides of the polarized layer of cells. The ability of the Corning^{®} Costar^{®} Snapwell^{™} system insert to detach the membrane allows the support ring of the insert to be used an anchor for the scaffold. However, after combining the support ring and membrane, there is a small void between the bottom lip of the support ring and the porous membrane itself. Therefore, a bioinert O-ring (cured silicone polymer) was used to fill the void. The support ring can apply pressure uniformly around the outer edge of the membrane to hold the scaffold against the membrane. This silicone ring held the PLGA/PCL scaffold in place over the non-biodegradable porous polycarbonate membrane of the Snapwell^{™} to hold scaffold during cell culturing and media changes securely.

Briefly, the well top was separated from the well bottom, and the PLGA/PCL scaffold precut to match the interior dimensions of the Corning^{®} Costar^{®} Snapwell^{™} system membrane) was placed into the center of the Corning^{®} Costar^{®} Snapwell^{™} system bottom so that it layed flat. An O-ring (polytetrafluoroethylene (PTFE), 12 mm x 10.25 mm x.1.2 mm, Superior Washer and Gasket Corp, US) was placed on top of the PLGA/PCL to holds the scaffold in place (but other O-rings can be used). The O-ring was previously sterilized by 20-minute treatment in 70% ethanol. The Corning^{®} Costar^{®} Snapwell^{™} system top was placed on top of the O-ring and gently pressed into place.

As shown in FIG. 15, the resulting "fuzzy" bi-layer scaffold includes a mesh-like structure of PCL loops. These loops serve as an anchors and scaffolding for PRP cells to stabilize their attachment to RPE cells (such as the peripheral, macular, and/or central RPE cells generated using the methods provided herein, for example as described in Examples 1-6). In native biological systems, photoreceptor attachment to the RPE cell surface is weak. The PCL loops create scaffolding to reinforce and maintain this attachment during mechanical handling stages *(e.g.,* during implantation procedure).

### Example 8

### Attachment of RPE and PRP Cells to Bi-Layer Scaffold

This example describes methods used to attach RPE and PRP cells to the PLGA/PCL scaffold generated in Example 7.

Briefly, RPE and PRP cells were attached/seeded onto the scaffold prepared in Example 7 as follows. Central RPE cells (*e.g.,* generated using the disclosed methods) were seeded on vitronectin (VTN)-coated PLGA/PCL scaffold (VTN concentration: 45.5ug/ml, 12hrs, about 20 to about 95 ug/ml can be used) assembled in a snap-well culture system (*e.g*., Corning^{®} Costar^{®} Snapwell^{™} system). Other RPE cells can be used, such as peripheral RPE cells and macular RPE cells generated using the methods provided herein. Pure, committed, and immature RPE cells were seeded at 350,000 cells/cm² and permitted to grow for 3 weeks, with RPE-MM media (standard RPE growth media) change every 2-3 days. Subsequently, PRP cell seeding was performed using iPRPs from FUJIFILM Cellular Dynamics, Inc. that arrived in frozen form, ready for culturing. After thawing and washing cells, iPRPs were seeded on top of iRPE at 3.5 million cells/cm² (0.5 mL media volume) in RPE-MM with no media modifications. Cells were grown for an additional 2 weeks prior use at 37°C, environmental oxygen, 5% CO₂.

As shown in FIGS. 16A-16B, the disclosed PLGA/PCL scaffold can be used to grow cells. The smaller hexagonal cells are the RPE monolayer that covers all surfaces of the scaffold. Larger appearing cells that grow in smaller patches are the PRP cells. Most PRP "patches" are located in "sheltered" compartments formed by PCL loops.

### Example 9

### Implantation of Bi-Layer Scaffold

This example describes methods used to implant a PLGA/PCL scaffold containing RPE and PRP cells generated in Example 8, into a pig eye. Briefly, the seeded PLGA/PCL scaffold was surgically implanted into a pig eye as follows. Sterilization of the surgical area with povidone iodine, a temporal canthotomy, superior rectus traction and nictitating membrane retraction is performed to increase the surgical exposure area. A nasal peritomy is done to exposed sclera and 4 surgical ports (infusion, chandelier illumination, and 2 working ports) are created 3.5 mm from limbus using 25G valve trocar cannulas (Alcon surgical). After vitrectomy and posterior vitreous detachment, a localized retinal detachment (RD) is done in the visual streak (laser area) using a 25G/38G cannula (MedOne Surgical Inc.) and scissors retinotomy is done at the base of the RD. A sclerotomy (2.3-2.5 mm) is done in the area of the nasal port to accommodate the transplantation tool. Tip of the tool loaded with RPE/PLGA scaffold is introduced through the retinotomy into the subretinal space were the iRPE scaffold is released with the help of the viscous fluid injector device of the vitrectomy system (Alcon surgical). An ocular wound clamp (custom made) is used to temporary close sclerotomy wound until second PRP/PGS scaffold is loaded into transplantation tool. Then, tip of the tool loaded with PRP/PGS scaffold is introduced through the retinotomy into the subretinal space were the PRP scaffold is released aiming to deposit it on top of RPE scaffold. An ocular wound clamp is used to temporary seal sclerotomy wound Soft tip 25G cannula or brush is used to manipulate both scaffolds to superposition in subretinal space. Fluid air exchange is used to flatten the detached area while monitored with intraoperative OCT to ensure that scaffold location is still unchanged. If needed, small corrections can be done using soft tip or brush cannula during fluid-air exchange. The sclerotomy is closed with nylon 8-0.

FIG. 17A shows a digital SLO image of the implant 1 month following surgery. As shown in FIG. 17B, one month after implantation, OCT B-scan shows complete integration of scaffold where residual scaffold matrix is observed by a slight elevation of retinal structures. No inflammatory reaction is observed. The retina layers are well pronounced on top of the scaffold area.

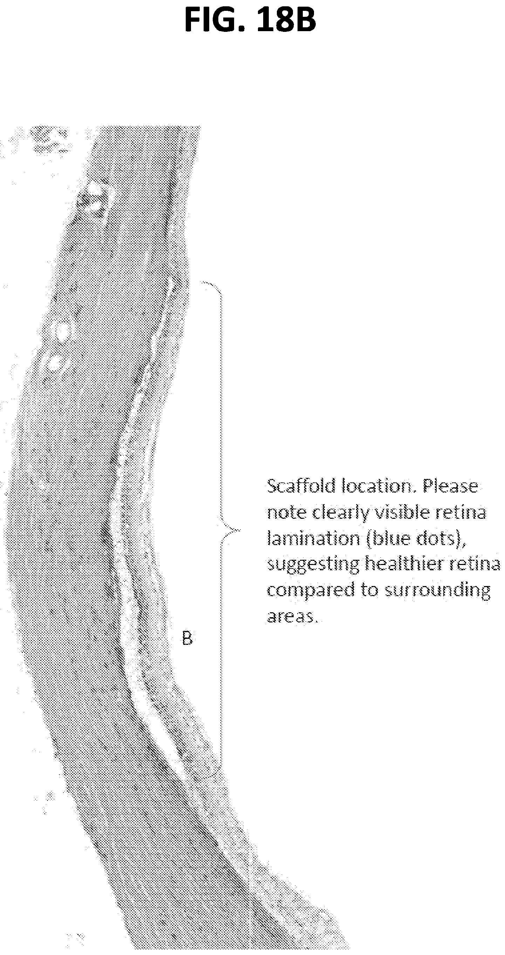

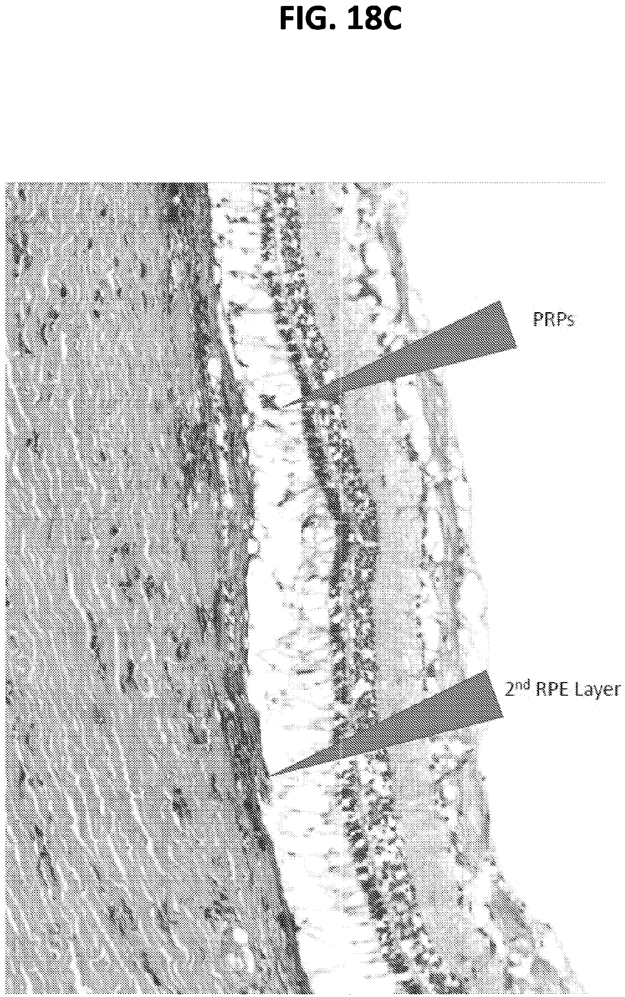

Two-months after surgery, the retina has a normal appearance and the scaffold is not visible. FIG. 18A shows a dissected and fixed fragment of the eye tissue showing area where the implant was placed. The retina is entirely healthy without any visible inflammatory or scarring processes. As shown in FIG. 18B, in the area where the scaffold was implanted, retinal lamination is evident (blue dots), indicating healthier retina as compared to surrounding areas. Thus, retina thickness in the scaffold area is more regular and has clearly visible retinal layers. These layers only present in the area where the scaffold was placed. It contrasts with clearly visible retinal degeneration that on both sides of scaffold borders. Some retinal separation in the scaffold area may be caused by sample processing. FIG. 18C provides a higher magnification image, showing the PRPs and 2^{nd} RPE layer. This finding demonstrates that the disclosed PLGA/PCL scaffolds can successfully integrate into retinal tissues and sustain normal retinal function.

### Example 10

### Additional Characterization of Cells Treated with AGN 193109 or endo-IWR-1

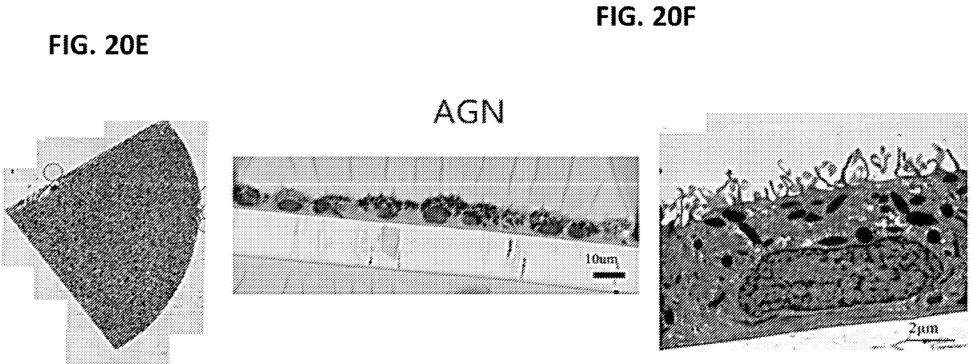

To demonstrate that DMSO (control, carrier), endo-IWR-1, and AGN 193109 are not detrimental, iPSC-RPE pigmentation levels were examined after treatment with DMSO (FIG. 20A), endo-IWR-1 (FIG. 20C) or AGN 193109 (FIG. 20E). AGN 193109 was used at 0.2 .µM and endo-IWR-1 at 4 uM. Both drugs were dissolved in DMSO, so that the final concentration of DMSO was not more than 0.1%. The final concentration of DMSO control was 0.1%. The compounds were added in RPE maturation medium until cells were mature. Gross cell morphology was also analyzed (FIGS. 20B, 20D, and 20F, left image) to detect possible abnormalities. Fine intracellular structures were examined for alterations with transmission electron microscopy (TEM, FIGS. 20B, 20D, and 20F, right image). These agents did not alter pigmentation levels, nor did they affect gross or fine cell morphology.

Trans-epithelial electrical resistance (TER) is a measure of the tightness of the monolayer. The tighter the monolayer, the healthier are the cells in the monolayer. A cutoff of 400 ohm*cm² was used to exclude cells with low TER. As shown in FIG. 21, both AGN 193109- and endo-IWR-1-treated cells were well above this threshold. The res data is displayed as boxplots, where box limits represent the first and third quartile, the central line shows the median, and the whiskers indicate the 5th and 95th percentile. The range specifies 90% of the data.

Single-cell RNA sequencing was performed to confirm that the compounds changed cell transcriptome. As shown in FIG. 22, the macular and mid-peripheral iPSC-RPE are functionally different from DMSO and from each other.

Bulk RNA sequencing was performed, and the expression levels of specific genes were compared to data available from the literature (FIG. 23). The data demonstrate a non-perfect, but consistent match of genes more expressed in macular RPE; a set of genes with opposite expression; and an almost perfect match of genes more expressed in peripheral RPE cells.

iPSC-RPE cells were provided purified bovine outer segment (mostly rod photoreceptors) to phagocytose. The outer segments were labelled with a dye that fluoresce when the outer segments are phagocytosed. RPE cells were incubated for 4 hours with outer segments and then analyzed with flow cytometry to measure outer segment ingestion or put back in the incubator for 20 hours before performing flow cytometry to measure outer segment digestion. As shown in FIG. 24, endo-iwr-1-treated cells ingest (peripheral cells) more outer segments, showing a possible affinity for rod outer segments, that are normally dominant in the periphery of the retina. One-way ANOVA showed no difference.

Acid phosphatase has higher expression in the macula (Boulton et al., Br. J. Opthalm. 78:125-9, doi: 10.1136/bjo.78.2.125, 1994). This enzyme has an important role in photoreceptor outer segment digestion. The acid phosphatase activity was determined in cells treated with DMSO, AGN 193109 or endo-IWR-1. RPE cells were lysed in distilled water to extract the enzyme. The lysates were then incubated with p-nitrophenyl phosphate, a phosphatase substrate that turn yellow when dephosphorylated by acid phosphatase. The color of the solution was measured with a spectrophotometer to estimate the enzyme activity. As shown in FIG. 25, AGN 193109-treated cells (macular iPSC-RPE) had higher acid phosphatase activity than DMSO- or endo-IWR-1-treated cells.

Peripheral (P)-RPE have higher mitochondrial oxidative phosphorylation (OXPHOS) as compared to macular (M)-RPE. Metabolic processes in the two types of RPE cells (M-RPE and P-RPE) were measured using Seahorse technology. The Seahorse system measures oxygen consumption rate and extracellular acidification rate of live cells, indicators of mitochondrial respiration and glycolysis respectively. As shown in FIG. 26, under two different carbon sources (proline and succinate), P-RPE had a higher ability to undergo OXPHOS as compared to M-RPE, which showed higher rate of glycolysis.

In view of the many possible embodiments to which the principles of our invention may be applied, it should be recognized that illustrated embodiments are only examples of the invention and should not be considered a limitation on the scope of the invention. Rather, the scope of the invention is defined by the following claims. We therefore claim as our invention all that comes within the scope of these claims.

## Claims

1. A scaffold, comprising
a first layer about at least 5 microns in height and having an average pore size of at least about 0.2 microns in diameter, comprising poly(lactic-co-glycolic acid) (PLGA) having a DL-lactide/glycolide ratio of about 0.25:3 to 3:0.25, and a fiber diameter of at least about 150 nm; and
a second layer comprising polycaprolactone (PCL) loops with a diameter of at least about 5 microns, which is attached to the first layer.

2. The scaffold of claim 1, wherein the first layer has a height of about 5 to about 40 microns, and/or the PLGA has a DL-lactide/glycolide ratio of about 1:1, and/or the PLGA has an average pore size of about 0.2 to 2 microns, and/or the PLGA has a fiber diameter of about 150 to about 650 nm, and/or the PCL loops have a diameter of about 5 to about 300 microns.

3. The scaffold according to claims 1 or 2, wherein the second layer is attached to the first layer using electrospinning or chemical etching and optionally, wherein the electrospinning comprises:
an electric field voltage of at least 5 kV,
a gas ejection pressure of at least 10 kPa (such as about 100 kPa to about 400kPa,
a working distance between nozzle and PLGA scaffolds of at least 10 mm, and
at least 2 minutes of electrospinning time.

4. The scaffold of any one of claims 1 to 3, further comprising a coating of a cellular adhesion protein, and optionally, wherein the cellular adhesion protein is vitronectin, laminin, fibronectin, or combinations thereof.

5. The scaffold of any one of claims 1-4, further comprising retinal pigment epithelial (RPE) cells, photoreceptor progenitor (PRP) cells, or both RPE cells and PRP cells, and/or
wherein the scaffold further comprises:
at least 100,000 cells/cm² RPE cells,
at least 1 million cells/cm² PRP cells, or
at least 100,000 cells/cm² RPE cells and at least 1 million cells/cm² PRP cells.

6. The scaffold of claim 5, wherein the RPE cells are macular, central, and/or peripheral RPE cells.

7. The scaffold of any one of claims 5-6, wherein the RPE cells are macular, central and/or peripheral human RPE cells, generated by an in vitro method comprising:
a) culturing pluripotent stem cells in a retinal induction medium to initiate differentiation of the cells into RPE progenitor cells;
b) culturing the RPE progenitor cells in a retinal differentiation medium to further differentiate the RPE progenitor cells into committed RPE cells;
c) culturing the committed RPE cells in a retinal medium to form immature RPE cells; and
d) culturing the immature RPE cells in a RPE maturation medium comprising a retinoic acid receptor (RAR) antagonist and/or a canonical Wnt inhibitor, thereby producing human RPE cells;
wherein the human RPE cells are macular, central and/or peripheral human RPE cells,
wherein the culturing steps can be performed on the scaffold and the method includes seeding the pluripotent stem cells onto the scaffold and the macular, central and/or peripheral human RPE cells are mature on the scaffold, or the culturing steps are not performed on the scaffold and the macular, central and/or peripheral human RPE cells are seeded onto the scaffold.

8. The scaffold of claim 7, wherein the stem cells are induced pluripotent stem cells (iPSC), and/or
wherein the RAR antagonist is AGN 193109, CE 2665, ER 5081, LE 135, LY 2955303, MM 11253, or liarozole dihydrochloride, and/or
wherein the canonical Wnt inhibitor is 4-(1,3,3a,4,7,7a-Hexahydro-1,3-dioxo-4,7-methano-2H-isoindol-2-yl)-N-8-quinolinyl-Benzamide ,Calphostin C, Cardionogen 1, CCT 031374 hydrobromide, IWP 12, XAV 939, WIKI4, ICG-001, Wnt-C59 (C59), KY02111, LGK-974, IWP-L6, FH535, iCRT 14, IWP 4, JW 67, JW 74, KYA 1797K, NLS-StAx-h, PNU 74654, TAK 715, IWP 2, CKI 7 dihydrochloride, (R)-CR8, D 4476, (R)-DRF053 dihydrochloride, Epiblastin A, IC 261, LH 846, PF 4800567 hydrochloride, PF 5006739, PF 670462, SR 3029, AZ 6102, JW 55, MN 64, or TC-E 5001 and/or wherein the RPE maturation medium comprises at least one primary cilium inducer and optionally,
wherein the at least one primary cilium inducer is prostaglandin E2 (PGE2) or aphidicolin.

9. A non-biodegradable porous polycarbonate membrane comprising the scaffold of any one of claims 1-8.

10. A kit comprising:
the scaffold of any one of claims 1-8 or the non-biodegradable porous polycarbonate membrane of claim 9; and
one or more of vitronectin, laminin, fibronectin, a snap-well culture system, a (polytetrafluoroethylene (PTFE) O-ring, retinal induction media, retinal differentiation media, retinal maturation media, retinal media, a non-biodegradable porous polycarbonate membrane, pluripotent stem cells, RPE progenitor cells, committed RPE cells, immature RPE cells, mature RPE cells and PRP cells.

11. The scaffold of any one of claims 1 to 8 or the non-biodegradable porous polycarbonate membrane of claim 9 for use in the treatment of a retinal degenerative disease, retinal dysfunction, retinal degradation, retinal damage, or loss of retinal pigment epithelium in a subject and optionally wherein the subject is a mammal.

12. The scaffold or the non-biodegradable porous polycarbonate membrane for use according to claim 11 wherein the retina degenerative disease is Stargardt's macular dystrophy, retinitis pigmentosa, age related macular degeneration, glaucoma, diabetic retinopathy, Lebers congenital amaurosis, acquired macular degeneration, hereditary macular degeneration, Best disease, late onset retinal degeneration, bear track dystrophy, retinal detachment, gyrate atrophy, choroideremia, pattern dystrophy and optionally, wherein the retinal damage is caused by laser, inflammatory, infectious, radiation, neovascular or traumatic injury.

13. A pharmaceutical composition comprising the scaffold of any one of claims 1-9, for use in the treatment of a retinal degenerative disease, retinal dysfunction, retinal degradation, retinal damage, or loss of retinal pigment epithelium in a subject.

14. An in vitro method, comprising:
culturing macular, central and/or peripheral RPE cells onto the scaffold of any one of claims 1-8 or the non-biodegradable porous polycarbonate membrane of claim 9, wherein the macular, central and/or peripheral RPE cells are generated by an in vitro method comprising:
a) culturing pluripotent stem cells in a retinal induction medium to initiate differentiation of the cells into RPE progenitor cells;
b) culturing the RPE progenitor cells in a retinal differentiation medium to further differentiate the RPE progenitor cells into committed RPE cells;
c) culturing the committed RPE cells in a retinal medium to form immature RPE cells; and
d) culturing the immature RPE cells in a RPE maturation medium comprising a retinoic acid receptor (RAR) antagonist and/or a canonical Wnt inhibitor, thereby producing macular, central or peripheral RPE cells; and
subsequently culturing PRP cells on top of the isolated macular, central and/or peripheral RPE cells, and optionally, wherein the culturing steps are performed in the presence of retinal maturation media, and/or
wherein culturing macular, central and/or peripheral RPE cells onto the scaffold forms a monolayer of the macular, central and/or peripheral RPE cells on the scaffold and/or wherein the culturing steps used to generate the macular, central and/or peripheral RPE cells are performed in the presence of the scaffold.

15. The scaffold or the non-biodegradable porous polycarbonate membrane for use according to claims 11 or 12 or the composition for use according to claim 13 wherein
the scaffold degrades within about a year following the implantation into the retina;
the first layer of the scaffold degrades within about 3 months following the implantation into the retina; and/or
the second layer of the scaffold degrades within about 1 year following the implantation into the retina.

## Patentansprüche

1. Gerüst, umfassend
eine erste Schicht mit einer Höhe von etwa 5 Mikrometern und mit einer durchschnittlichen Porengröße von zumindest etwa 0,2 Mikrometern im Durchmesser, umfassend Poly(milch-co-glycolsäure) (PLGA) mit einem DL-Lactid/Glycolid-Verhältnis von etwa 0,25:3 bis 3:0,25 und einem Faserdurchmesser von zumindest etwa 150 nm; und
eine zweite Schicht, die Polycaprolacton(PCL-)Schleifen mit einem Durchmesser von zumindest etwa 5 Mikrometern umfasst, die an der ersten Schicht angebracht ist.

2. Gerüst nach Anspruch 1, wobei die erste Schicht eine Höhe von etwa 5 bis etwa 40 Mikrometern aufweist und/oder die PLGA ein DL-Lactid/Glycolid-Verhältnis von etwa 1:1 aufweist und/oder die PLGA eine durchschnittliche Porengröße von etwa 0,2 bis 2 Mikrometern aufweist und/oder die PLGA einen Faserdurchmesser von etwa 150 bis etwa 650 nm aufweist und/oder die PCL-Schleifen einen Durchmesser von etwa 5 bis etwa 300 Mikrometern aufweisen.

3. Gerüst nach Anspruch 1 oder 2, wobei die zweite Schicht an der ersten Schicht unter Verwendung von Elektrospinnen oder chemischem Ätzen angebracht ist und wobei optional das Elektrospinnen Folgendes umfasst:
eine elektrische Feldspannung von zumindest 5 kV,
einen Gasausstoßdruck von zumindest 10 kPa (wie etwa 100 kPa bis etwa 400 kPa,
einen Arbeitsabstand zwischen Düse und PLGA-Gerüsten von zumindest 10 mm, und
zumindest 2 Minuten Elektrospinnzeit.

4. Gerüst nach einem der Ansprüche 1 bis 3, ferner umfassend eine Beschichtung aus einem zellulären Adhäsionsprotein und wobei optional das zelluläre Adhäsionsprotein Vitronectin, Laminin, Fibronectin oder Kombinationen davon ist.

5. Gerüst nach einem der Ansprüche 1-4, ferner umfassend retinale Pigmentepithel(RPE-)Zellen, Photorezeptorvorläufer(PRP-)Zellen oder sowohl RPE-Zellen als auch PRP-Zellen, und/oder
wobei das Gerüst ferner Folgendes umfasst:
zumindest 100.000 Zellen/cm² RPE-Zellen,
zumindest 1 Million Zellen/cm² PRP-Zellen, oder
zumindest 100.000 Zellen/cm² RPE-Zellen und zumindest 1 Million Zellen/cm² PRP-Zellen.

6. Gerüst nach Anspruch 5, wobei die RPE-Zellen makuläre, zentrale und/oder periphere RPE-Zellen sind.

7. Gerüst nach einem der Ansprüche 5-6, wobei die RPE-Zellen makuläre, zentrale und/oder periphere menschliche RPE-Zellen sind, die durch ein In-vitro-Verfahren erzeugt werden, umfassend:
a) Kultivieren von pluripotenten Stammzellen in einem retinalen Induktionsmedium, um Differenzierung der Zellen in RPE-Vorläuferzellen zu initiieren;
b) Kultivieren der RPE-Vorläuferzellen in einem retinalen Differenzierungsmedium, um die RPE-Vorläuferzellen weiter in engagierte RPE-Zellen zu differenzieren;
c) Kultivieren der engagierten RPE-Zellen in einem retinalen Medium, um unreife RPE-Zellen zu bilden; und
d) Kultivieren der unreifen RPE-Zellen in einem RPE-Reifungsmedium, das einen Retinsäurerezeptor(RAR-)Antagonisten und/oder einen kanonischen Wnt-Inhibitor umfasst, wodurch menschliche RPE-Zellen produziert werden;
wobei die menschlichen RPE-Zellen makuläre, zentrale und/oder periphere menschliche RPE-Zellen sind,
wobei die Kultivierungsschritte auf dem Gerüst durchgeführt werden können und das Verfahren Aussäen der pluripotenten Stammzellen auf das Gerüst beinhaltet und die makulären, zentralen und/oder peripheren menschlichen RPE-Zellen auf dem Gerüst reif sind oder die Kultivierungsschritte nicht auf dem Gerüst durchgeführt werden und die makulären, zentralen und/oder peripheren menschlichen RPE-Zellen auf das Gerüst ausgesät werden.

8. Gerüst nach Anspruch 7, wobei die Stammzellen induzierte pluripotente Stammzellen (iPSC) sind, und/oder
wobei der RAR-Antagonist AGN 193109, CE 2665, ER 5081, LE 135, LY 2955303, MM 11253 oder Liarozoldihydrochlorid ist, und/oder
wobei der kanonische Wnt-Inhibitor 4-(1,3,3a ,4,7,7a-Hexahydro-1,3-dioxo-4,7-methano-2H-isoindol-2-yl)-N-8-chinolinyl-benzamid, Calphostin C, Cardionogen 1, CCT 031374 Hydrobromid, IWP 12, XAV 939, WIKI4, ICG-001, Wnt-C59 (C59), KY02111, LGK-974, IWP-L6, FH535, iCRT 14, IWP 4, JW 67, JW 74, KYA 1797K, NLS-StAx-h, PNU 74654, TAK 715, IWP 2, CKI 7 Dihydrochlorid, (R)-CR8, D 4476, (R)-DRF053 Dihydrochlorid, Epiblastin A, IC 261, LH 846, PF 4800567 Hydrochlorid, PF 5006739, PF 670462, SR 3029, AZ 6102, JW 55, MN 64 oder TC-E 5001 ist und/oder wobei das RPE-Reifungsmedium zumindest einen primären Ziliuminduktor umfasst, und
wobei optional der zumindest eine primäre Ziliuminduktor Prostaglandin E2 (PGE2) oder Aphidicolin ist.

9. Nicht biologisch abbaubare poröse Polycarbonatmembran, umfassend das Gerüst nach einem der Ansprüche 1-8.

10. Kit, umfassend:
das Gerüst nach einem der Ansprüche 1-8 oder die nicht biologisch abbaubare poröse Polycarbonatmembran nach Anspruch 9; und
eines oder mehrere von Vitronectin, Laminin, Fibronectin, einem Snap-Well-Kultursystem, einem (Polytetrafluorethylen(PTFE-)O-Ring, retinalen Induktionsmedien, retinalen Differenzierungsmedien, retinalen Reifungsmedien, retinalen Medien, einer nicht biologisch abbaubaren porösen Polycarbonatmembran, pluripotenten Stammzellen, RPE-Vorläuferzellen, engagierten RPE-Zellen, unreifen RPE-Zellen, reifen RPE-Zellen und PRP-Zellen.

11. Gerüst nach einem der Ansprüche 1 bis 8 oder nicht biologisch abbaubare poröse Polycarbonatmembran nach Anspruch 9 zur Verwendung bei der Behandlung einer retinalen degenerativen Erkrankung, einer retinalen Dysfunktion, eines retinalen Abbaus, einer retinalen Schädigung oder eines Verlusts von retinalem Pigmentepithel bei einem Subjekt und wobei optional das Subjekt ein Säugetier ist.

12. Gerüst oder nicht biologisch abbaubare poröse Polycarbonatmembran zur Verwendung nach Anspruch 11, wobei die degenerative Retinaerkrankung Stargardts Makuladystrophie, Retinitis pigmentosa, altersbedingte Makuladegeneration, Glaukom, diabetische Retinopathie, kongenitale Leberssche Amaurose, erworbene Makuladegeneration, erbliche Makuladegeneration, Best-Erkrankung, spät einsetzende Retinadegeneration, Bear-Track-Dystrophie, retinale Ablösung, Gyratatrophie, Choroiderämie, Muster-Dystrophie ist und wobei optional die retinale Schädigung durch Laser-, entzündliche, infektiöse, Strahlen-, neovaskuläre oder traumatische Verletzung bewirkt wird.

13. Pharmazeutische Zusammensetzung, die das Gerüst nach einem der Ansprüche 1-9 umfasst, zur Verwendung bei der Behandlung einer retinalen degenerativen Erkrankung, einer retinalen Dysfunktion, eines retinalen Abbaus, einer retinalen Schädigung oder eines Verlusts von retinalem Pigmentepithel bei einem Subjekt.

14. In-vitro-Verfahren, umfassend:
Kultivieren von makulären, zentralen und/oder peripheren RPE-Zellen auf das Gerüst nach einem der Ansprüche 1-8 oder die nicht biologisch abbaubare poröse Polycarbonatmembran nach Anspruch 9, wobei die makulären, zentralen und/oder peripheren RPE-Zellen durch ein In-vitro-Verfahren erzeugt werden, umfassend:
a) Kultivieren von pluripotenten Stammzellen in einem retinalen Induktionsmedium, um Differenzierung der Zellen in RPE-Vorläuferzellen zu initiieren;
b) Kultivieren der RPE-Vorläuferzellen in einem retinalen Differenzierungsmedium, um die RPE-Vorläuferzellen weiter in engagierte RPE-Zellen zu differenzieren;
c) Kultivieren der engagierten RPE-Zellen in einem retinalen Medium, um unreife RPE-Zellen zu bilden; und
d) Kultivieren der unreifen RPE-Zellen in einem RPE-Reifungsmedium, das einen Retinsäurerezeptor(RAR-)Antagonisten und/oder einen kanonischen Wnt-Inhibitor umfasst, wodurch makuläre, zentrale oder periphere RPE-Zellen produziert werden; und
anschließendes Kultivieren von PRP-Zellen auf den isolierten makulären, zentralen und/oder peripheren RPE-Zellen, und wobei optional die Kultivierungsschritte in der Gegenwart von retinalem Reifungsmedien durchgeführt werden, und/oder
wobei das Kultivieren von makulären, zentralen und/oder peripheren RPE-Zellen auf dem Gerüst eine Monoschicht der makulären, zentralen und/oder peripheren RPE-Zellen auf dem Gerüst bildet und/oder wobei die Kultivierungsschritte, die verwendet werden, um die makulären, zentralen und/oder peripheren RPE-Zellen zu erzeugen, in der Gegenwart des Gerüsts durchgeführt werden.

15. Gerüst oder nicht biologisch abbaubare poröse Polycarbonatmembran zur Verwendung nach Anspruch 11 oder 12 oder Zusammensetzung zur Verwendung nach Anspruch 13, wobei
sich das Gerüst innerhalb von etwa einem Jahr nach der Implantation in die Retina abbaut;
sich die erste Schicht des Gerüsts innerhalb von etwa 3 Monaten nach der Implantation in die Retina abbaut; und/oder
sich die zweite Schicht des Gerüsts innerhalb von etwa 1 Jahr nach der Implantation in die Retina abbaut.

## Revendications

1. Échafaudage comprenant
une première couche d'environ au moins 5 microns de hauteur et présentant une taille de pore moyenne d'au moins environ 0,2 micron de diamètre, comprenant de l'acide poly(lactique-co-glycolique) (PLGA) présentant un rapport DL-lactide/glycolide d'environ 0,25:3 à 3:0,25 et un diamètre de fibre d'au moins environ 150 nm ; et
une seconde couche comprenant des boucles de polycaprolactone (PCL) d'un diamètre d'au moins environ 5 microns, qui est fixée à la première couche.

2. Échafaudage de la revendication 1, dans lequel la première couche présente une hauteur d'environ 5 à environ 40 microns, et/ou le PLGA présente un rapport DL-lactide/glycolide d'environ 1:1, et/ou le PLGA présente une taille de pore moyenne d'environ 0,2 à 2 microns, et/ou le PLGA présente un diamètre de fibre d'environ 150 à environ 650 nm, et/ou les boucles de PCL présentent un diamètre d'environ 5 à environ 300 microns.

3. Échafaudage selon l'une des revendications 1 ou 2, dans lequel la seconde couche est fixée à la première couche par électrofilage ou gravure chimique et éventuellement, dans lequel l'électrofilage comprend :
une tension de champ électrique d'au moins 5 kV,
une pression d'éjection de gaz d'au moins 10 kPa (par exemple d'environ 100 kPa à environ 400 kPa,
une distance de travail entre une buse et des échafaudages PLGA d'au moins 10 mm, et
au moins 2 minutes de temps d'électrofilage.

4. Échafaudage de l'une quelconque des revendications 1 à 3, comprenant en outre un revêtement d'une protéine d'adhésion cellulaire, et éventuellement, ladite protéine d'adhésion cellulaire étant la vitronectine, la laminine, la fibronectine ou des combinaisons de celles-ci.

5. Échafaudage de l'une quelconque des revendications 1 à 4, comprenant en outre des cellules épithéliales pigmentaires rétiniennes (RPE), des cellules progénitrices photoréceptrices (PRP), ou à la fois des cellules RPE et des cellules PRP, et/ou
ledit échafaudage comprenant en outre :
au moins 100 000 cellules/cm² de cellules RPE,
au moins 1 million cellules/cm² de cellules PRP, ou
au moins 100 000 cellules/cm² de cellules RPE et au moins 1 million cellules/cm² de cellules PRP.

6. Échafaudage de la revendication 5, dans lequel les cellules RPE sont des cellules RPE maculaires, centrales et/ou périphériques.

7. Échafaudage de l'une quelconque des revendications 5 à 6, dans lequel les cellules RPE sont des cellules RPE humaines maculaires, centrales et/ou périphériques, générées par un procédé in vitro comprenant :
a) la culture de cellules souches pluripotentes dans un milieu d'induction rétinienne pour initier la différenciation des cellules en cellules progénitrices RPE ;
b) la culture des cellules progénitrices RPE dans un milieu de différenciation rétinien pour différencier davantage les cellules progénitrices RPE en cellules RPE engagées ;
c) la culture des cellules RPE engagées dans un milieu rétinien pour former des cellules RPE immatures ; et
d) la culture des cellules RPE immatures dans un milieu de maturation RPE comprenant un antagoniste du récepteur de l'acide rétinoïque (RAR) et/ou un inhibiteur de Wnt canonique, produisant ainsi des cellules RPE humaines ;
dans lequel les cellules RPE humaines sont des cellules RPE humaines maculaires, centrales et/ou périphériques,
dans lequel les étapes de culture peuvent être réalisées sur l'échafaudage et le procédé comprend l'ensemencement des cellules souches pluripotentes sur l'échafaudage et les cellules RPE humaines maculaires, centrales et/ou périphériques sont matures sur l'échafaudage, ou les étapes de culture ne sont pas réalisées sur l'échafaudage et les cellules RPE humaines maculaires, centrales et/ou périphériques sont ensemencées sur l'échafaudage.

8. Échafaudage de la revendication 7, dans lequel les cellules souches sont des cellules souches pluripotentes induites (iPSC), et/ou
dans lequel l'antagoniste RAR est AGN 193109, CE 2665, ER 5081, LE 135, LY 2955303, MM 11253, ou le dichlorhydrate de liarozole, et/ou
dans lequel l'inhibiteur de Wnt canonique est le 4-(1,3,3a,4,7,7a-hexahydro-1,3-dioxo-4,7-méthano-2H-isoindol-2-yl)-N-8-quinolinyl-benzamide, la calphostine C, le cardionogen 1, le bromhydrate de CCT 031374, IWP 12, XAV 939, WIKI4, ICG-001, Wnt-C59 (C59), KY02111, LGK-974, IWP-L6, FH535, iCRT 14, IWP 4, JW 67, JW 74, KYA 1797K, NLS-StAx-h, PNU 74654, TAK 715, IWP 2, le dichlorhydrate de CKI 7, (R)-CR8, D 4476, le dichlorhydrate de (R)-DRF053, l'épiblastine A, IC 261, LH 846, le chlorhydrate de PF 4800567, PF 5006739, PF 670462, SR 3029, AZ 6102, JW 55, MN 64 ou TC-E 5001 et/ou dans lequel le milieu de maturation RPE comprend au moins un inducteur de cil primaire, et éventuellement
dans lequel l'au moins un inducteur de cil primaire est la prostaglandine E2 (PGE2) ou l'aphidicoline.

9. Membrane en polycarbonate poreux non biodégradable comprenant l'échafaudage de l'une quelconque des revendications 1 à 8.

10. Kit comprenant :
l'échafaudage de l'une quelconque des revendications 1 à 8 ou la membrane en polycarbonate poreux non biodégradable de la revendication 9 ; et
un ou plusieurs parmi la vitronectine, la laminine, la fibronectine, un système de culture snap-well, un joint torique de polytétrafluoroéthylène (PTFE), des milieux d'induction rétinienne, des milieux de différenciation rétinienne, des milieux de maturation rétinienne, des milieux rétiniens, une membrane en polycarbonate poreux non biodégradable, des cellules souches pluripotentes, des cellules progénitrices RPE, des cellules RPE engagées, des cellules RPE immatures, des cellules RPE matures et des cellules PRP.

11. Échafaudage de l'une quelconque des revendications 1 à 8 ou membrane en polycarbonate poreux non biodégradable de la revendication 9 destiné(e) à être utilisé(e) dans le traitement d'une maladie dégénérative rétinienne, d'un dysfonctionnement rétinien, d'une dégradation rétinienne, d'un dommage rétinien ou d'une perte d'épithélium pigmentaire rétinien chez un sujet et éventuellement ledit sujet étant un mammifère.

12. Échafaudage ou membrane en polycarbonate poreux non biodégradable destiné(e) à être utilisé(e) selon la revendication 11, ladite maladie dégénérative rétinienne est la dystrophie maculaire de Stargardt, la rétinite pigmentaire, la dégénérescence maculaire liée à l'âge, le glaucome, la rétinopathie diabétique, l'amaurose congénitale de Lebers, la dégénérescence maculaire acquise, la dégénérescence maculaire héréditaire, la maladie de Best, la dégénérescence rétinienne à apparition tardive, la dystrophie en pattes d'ours, le décollement rétinien, l'atrophie gyrée, la choroïdérémie, une pattern dystrophie et éventuellement, dans lequel les dommages rétiniens sont causés par une lésion au laser, inflammatoire, infectieuse, radiologique, néovasculaire ou traumatique.

13. Composition pharmaceutique comprenant l'échafaudage de l'une quelconque des revendications 1 à 9, destinée à être utilisée dans le traitement d'une maladie dégénérative rétinienne, d'un dysfonctionnement rétinien, d'une dégradation rétinienne, d'un dommage rétinien ou d'une perte d'épithélium pigmentaire rétinien chez un sujet.

14. Procédé in vitro, comprenant :
la culture de cellules RPE maculaires, centrales et/ou périphériques sur l'échafaudage de l'une quelconque des revendications 1 à 8 ou sur la membrane en polycarbonate poreux non biodégradable de la revendication 9, lesdites cellules RPE maculaires, centrales et/ou périphériques étant générées par un procédé in vitro comprenant :
a) la culture de cellules souches pluripotentes dans un milieu d'induction rétinienne pour initier la différenciation des cellules en cellules progénitrices RPE ;
b) la culture des cellules progénitrices RPE dans un milieu de différenciation rétinien pour différencier davantage les cellules progénitrices RPE en cellules RPE engagées ;
c) la culture des cellules RPE engagées dans un milieu rétinien pour former des cellules RPE immatures ; et
d) la culture des cellules RPE immatures dans un milieu de maturation RPE comprenant un antagoniste du récepteur de l'acide rétinoïque (RAR) et/ou un inhibiteur de Wnt canonique, produisant ainsi des cellules RPE maculaires, centrales ou périphériques ; et
la culture ultérieure de cellules PRP sur les cellules RPE maculaires, centrales et/ou périphériques isolées, et éventuellement, dans lequel les étapes de culture sont réalisées en présence de milieux de maturation rétiniens, et/ou
dans lequel la culture de cellules RPE maculaires, centrales et/ou périphériques sur l'échafaudage forme une monocouche des cellules RPE maculaires, centrales et/ou périphériques sur l'échafaudage et/ou dans lequel les étapes de culture utilisées pour générer les cellules RPE maculaires, centrales et/ou périphériques sont réalisées en présence de l'échafaudage.

15. Échafaudage ou membrane en polycarbonate poreux non biodégradable destiné(e) à être utilisé(e) selon l'une des revendications 11 ou 12 ou composition destinée à être utilisée selon la revendication 13,
ledit échafaudage se dégradant dans un délai d'environ un an suivant l'implantation dans la rétine ;
ladite première couche de l'échafaudage se dégradant dans un délai d'environ 3 mois suivant l'implantation dans la rétine ; et/ou
ladite seconde couche de l'échafaudage se dégradant dans un délai d'environ un an suivant l'implantation dans la rétine.
